# EUROPEAN PATENT SPECIFICATION

(11) **EP 3 592 858 B1**
(45) Date of publication and mention of the grant of the patent: **12.06.2024**
(21) Application number: 18710031.8
(22) Date of filing: 08.03.2018
(51) Int. Cl.: C12P 7/44, C10B 53/02, C10C 5/00, C07C 39/08, C07C 4/04, C07C 63/26, C07D 201/08, C08L 77/06, C12N 1/21, C12N 15/01

(54) **MEANS AND METHODS FOR LIGNIN PYROLYSIS**
MITTEL UND VERFAHREN ZUR PYROLYSE VON LIGNIN
MOYENS ET PROCÉDÉS POUR LA PYROLYSE DE LIGNINE

(30) Priority: 09.03.2017 LU 100139; 09.03.2017 EP 17160205; 20.10.2017 EP 17197421
(43) Date of publication of application: 15.01.2020
(73) Proprietor: Universität des Saarlandes, 66123 Saarbrücken (DE)
(72) Inventor: WITTMANN, Christoph, 66123 Saarbruecken (DE); VAN DUUREN, Joost, 68199 Mannheim (DE); KOHLSTEDT, Michael, 66123 Saarbruecken (DE); STOLZENBERGER, Jessica, 33615 Bielefeld (DE); STARCK, Sören, 67435 Neustadt (Weinstraße) (DE); BARTON, Nadja, 66123 Saarbruecken (DE); SELZER, Mirjam, 66123 Saarbruecken (DE); FRITZ, Michael, 66123 Saarbruecken (DE); DE WILD, Paul, 1755 ZG Petten (NL); KUHL, Martin, 66123 Saarbruecken (DE); BECKER, Judith, 66123 Saarbruecken (DE)
(74) Representative: Schiweck Weinzierl Koch Patentanwälte Partnerschaft mbB
(86) International application number: PCT/EP2018/055697
(87) International publication number: WO 2018/162612

(56) References cited:
- EP-A1- 2 562 249
- WO-A1-2017/037013
- US-A1- 2016 017 381
- SHEN ET AL: "A thermochemical- biochemical hybrid processing of lignocellulosic biomass for producing fuels and chemicals", BIOTECHNOLOGY ADVANCES, vol. 33, 2015, pages 1799-1813, XP029328720,
- VARDON ET AL: "Adipic acid production from lignin", ENERGY & ENVIRONMENTAL SCIENCE, vol. 8, 2015, pages 617-628, XP002771403, cited in the application
- BECKHAM ET AL: "Opportunities and challenges in biological lignin valorization", CURRENT OPINION IN BIOTECHNOLOGY, vol. 42, 2016, pages 40-53, XP029831132,
- DE WILD ET AL: "Biobased alkylphenols from lignins via a two-step pyrolysis - hydrodeoxygenation approach", BIORESOURCE TECHNOLOGY, vol. 229, 10 January 2017 (2017-01-10), pages 160-168, XP029907522,
- BECKHAM: "Biological conversion of thermochemical aqueous streams", NREL (National Renewable Energy Laboratory) Project review, 8 March 2017 (2017-03-08), pages 1-31, XP002771404, Retrieved from the Internet: URL:https://energy.gov/sites/prod/files/20 17/05/f34/Biological%20Conversion%20of%20T hermochemical%20Aqueous%20Streams.pdf [retrieved on 2017-06-26]
- FUNKE ET AL: "Fast pyrolysis char - assessment of alternative uses within the bioliq(R) concept", BIORESOURCE TECHNOLOGY, vol. 200, 2016, pages 905-913, XP029312873,
- DIAS ET AL: "Exploring the microbial production of aromatic fine chemicals to overcome the barriers of traditional methods", ADVANCES IN APPLIED SCIENCE RESEARCH, vol. 8, 2017, pages 94-110, XP002771405,
- JUNG ET AL: "Metabolic engineering of Klebsiella pneumoniae for the production of cis,cis-muconic acid", APPLIED MICROBIOLOGY AND BIOTECHNOLOGY, vol. 99, 2015, pages 5217-5225, XP002780985,
- BARTON ET AL: "Enabling the valorization of guaiacol-based lignin: integrated chemical and biochemical production of cis,cis-muconic acid using metabolically engineered Amycolatopsis sp ATCC 39116", METABOLIC ENGINEERING, vol. 45, 12 December 2017 (2017-12-12), pages 200-210, XP002780986,

## Description

The present invention refers to a novel and inventive process comprising fast pyrolysis of lignin, or optionally lignocellulose, and biocatalyzed conversion of catechol to cis-cis- muconic acid.

### Background

Biomass is a renewable feedstock containing carbon and therefore an alternative to crude oil. The conversion of biomass into biofuel and bio-based chemicals can be part of the solution to a carbon neutral society. The main problems for the application of biomass are related to transport and handling, the limited scale of conversion process and the competition with the food industry (Butler *et al.,* 2011, Yildiz *et al.,* 2016). To overcome this problem, the integral fast pyrolysis process in combination with an *ex-situ* catalysis step using the oilphase of the fast pyrolysis process seems to be promising (Vispute *et al.,* 2010).

Pyrolysis processes are robust, continuous, and able to process different biomass streams. For fast pyrolysis, various reactor designs have been adopted. For example, a fluid bed reactor (Dynamotive and RTI, USA), a circulating fluidized and transported bed reactor (Ensyn, USA; Fortum, FI), an ablative reactor (only at demo scale), a rotating cone reactor (BTG, NL), a vacuum reactor (Pyrovac, USA), and an auger reactor (KIT, DE and ROI, USA). With these reactor technologies biomass can be depolymerized by thermal decomposition in the absence of oxygen. According to BTG, pyrolysis oil can be generated at a yield of 60-75%, which can substitute heavy and light oil in industrial boiler applications. Such a product values 111 €/ ton, based on the price for heating oil (257 €/ton; Trading Economics) and the caloric values of 20 and 46.2 GJ/ton for bio-oil and heating oil, respectively (European biomass Industry association; Natural Gas Australia). To expand the application, techniques have been developed to produce substitutes for fuel from bio-oil, which would value 115 €/ ton based on the price for gasoline (267 €/ton) and the caloric values of 20 and 46.5 GJ/ton of bio-oil and petrol, respectively. In addition, it is expected that bio-oil could function as platform intermediate for high-value chemicals and materials. The profitability of the process could improve with higher oil prices and increasing prices for CO₂ credits will not harm the generated added value, since it is a carbon neutral process. As a result, it is expected that lignocellulosic biomass as e.g. wheat straw (19-36 €/ton; USDA 2016) can be commercialized via these processes at large scale. Lignocellulose is in this context a preferred resource, because it is not easily accessible for the food chain due to its low digestibility.

Various configurations of fast pyrolysis processes undergo intense development currently. Besides bio-oil, gas and char are being produced. Among others, the bio-oil contains oligomeric phenolic substances, monomeric phenols, and -depending on the specifics of the pyrolysis process-, separately an acquatic phase. For the latter stream, which may contain up to 32% of the carbon no industrial application has been defined yet. The crude-like-oil can subsequently be used for industrial processes by hydrotreating. As a result, a high yield of hydrocarbon fuels and potentially aromatic chemicals can be produced, as well as half fabricates as drop-in products for existing refinery processes (Bridgwater et al., 2012, Butler *et al.,* 2011, Yildiz *et al.,* 2016). To optimize the fast pyrolysis process catalyst materials are applied to improve the selectivity to the desired product. Catalysts can be built in the process by impregnation in the biomass feed, by mixing with the biomass in the fast pyrolysis reactor (*in-situ*), by adding to primary pyrolysis vapours (*ex-situ*), or by modifying the condensed liquids (hydrodeoxygenation). These options could be applied separately or in various combinations. Catalysts make sure that the mixture is less heterogenic and the oxygen content is reduced, whereby the viscosity is increased as well as the aromatic concentration. A well described process is the integrated catalytic approach that combines hydroprocessing with zeolite catalysis (Vispute *et al.,* 2010). First polyols and alcohols are being produced by hydroprocessing, which products are subsequently converted to olefins and aromatic hydrocarbons with the zeolite catalyst. The biochar is tested as growth enhancer and the gas is used for the production of hydrogen, syngas, and as a resource for the Fischer Tropsch process. When lignocellulose is processed with the pyrolysis process, the acquatic phase contains aromatics, carboxylic acids, aldehydes, furan and levoglucosan (Vispute *et al.,* 2009, Vispute *et al.,* 2010, Valle *et al.,* 2013, Remón *et al.,* 2014). To valorize this stream, the different fractions have to be separated based on the unique chemical and physical properties (Pollard *et al.,* 2012). Because of the heterogeneity this stream is still slated for wastewater treatment. Therefore, it can be considered as a double economic burden (Black *et al.,* 2016; Mohan *et al.,* 2006; Sipilä *et al.,* 1998; Vispute *et al.,* 2010).

Already several industrial initiatives are being developed for lignocellulose such as Bioliq from the KIT (Germany), Envergent Technologies a joint venture between Honeywell (USA) and Ensyn (Canada), and BTG BioLiquids (The Netherlands). Other examples of developed processes are Inearis Technologies, Anellotech and GTI with the IH2 technology (USA). These companies provide stand-alone processes or provide facilities that function as an addition to existing processes, e.g. embodied in the pulp and paper industry or the production of cellulosic fuel. In the latter situations, the process uses concentrated lignin instead of lignocellulose as resource. The pulp and paper industry produces over 50 Mt/yr lignin that results from fractionation of lignocellulosic biomass.

In the pulp and paper industry cellulose from lignocellulose is used at large scale. As a result, lignin and hemicellulose can be considered "waste products". The hydrophobic nature of lignin devastates the formation of hydrogen connections between cellulose and eventually hemi-cellulose, which is needed for a robust paper. During the kraft process, which represents 85% of the total lignin production, 90-95% of the lignin present in soft- and hardwood is dissolved together with hemicellulose, extractives and cooking chemicals in an aqueous phase, which is called black liquor. A mixture of sodium hydroxide and sodium sulfite is applied (Vishtal and Kraslawski. 2011). After several hours of delignification at about 170°C, lignin and hemicellulose are degraded. Because of the first compound alkali soluble fragments are solved, while the latter compound causes the dissolution of lignin into small aromatics. Black liquor contains approximately 15% lignin on a dry weight basis and generates about 15 GJ/ ton dry weight when it is incinerated (Azadi *et al.,* 2013). Based on the price for heating oil and its intrinsic energy value, it can be calculated that the generated value with the lignin containing black liquor is 83 €/ ton dry weight when it is burned (Trading Economics; Natural Gas Australia). However, in case lignin is converted into bulk chemicals such as adipic acid about 1.000 €/ ton can be obtained (ICIS, 2016). From the black liquor 63*10⁴ tons of kraft lignin (178 €/ ton; Alibaba) is produced with a heating value of about 26.7 MJ/ kg (Tomani, 2010). The cost for this process step is estimated 23 to 53 €/ ton dependent on the sulfur sensitivity of the lignin application, which can vary from 1% to 3% (Vishtal and Kraslawski. 2011).

Other sulfur free lignins as e.g. organosolv lignin are expected to be produced as a consequence of the expanding second generation biobased economy (Laurichesse and Avérous, 2014). At commercial scale processes based on steam explosion (Beta Renewables, IT; ABNT, USA) and enzymatic hydrolysis (Poet-DSM, USA) are applied. At demo scale hydrothermal conversion (Inbicon, DK) and organolsolv pulping (Lignol, CA; Chempolis, Fl; CIMV, FR) processes are developed. Especially from the organosolv process lignin can be fractionated. Due to its isolation process, organosolv lignin has a high purity because of a limited amount of residual carbohydrates and minerals. Since lignocellulose is refined for the production of bio-ethanol from C6 and C5 sugars, lignin is a left over product. In the organosolv process, lignocellulose is cooked in water in the presence of a mixture of (organic) solvents (40-80%) (e.g. ethanol, butanol, methanol, ketons such as acetone, etc.) and/or acids (e.g. acetic acid, formic acid) at 140°C to 220°C.

Lignin is the second most abundant biopolymer of the world after cellulose. Various biomass sources have a different percentage of lignin content. Softwood exists for 25-35% out of lignin, hardwood for 18-25%, while grasses have a content of less than 20% (Azadi *et al*., 2013). The chemical structure is based on an amorphous, polyphenolic material composed out of three phenylpropane monomers. Because of the heterogeneity and recalcitrance of lignin, this chemical structure could not yet be valorized in large scale industrial processes. The different compositions of the three monomers and various kinds of ether and carbon-carbon bonds make lignin a complex substance to be processed under standardized conditions. As a result, still lignin is primarily (98%) incinerated in industry to generate process heat and steam (Gosselink *et al.,* 2004; Tuck *et al.,* 2012). To obtain a higher added value, new biorefinery technologies are developed to pretreat lignin as such that it can be used for large scale for commercial processes (Rahimi *et al.,* 2014).

Lignin can be used for polymer additives (e.g. polyolefins like PP and PE), bitumen, cement, carbon fibers, agrochemical applications (e.g. soil improver), and for phenolic resins and adhesives (e.g. coating applications) (de Wild *et al.,* 2014). Given its structure, it is a potential source of renewable aromatic chemicals. The Energy research Center of the Netherlands (ECN) has developed a pyrolysis process to convert lignin thermochemically to expand the usage of its chemical structure in e.g. phenol-formaldehyde resin (2.225 €/ton; Alibaba) (Kim, 2015). The process uses a bubbling fluidized bed reactor to convert lignin from various biomass streams (de Wild et al., 2014). The developed process is named LIBRA, which is short for lignin biorefinery approach. The LIBRA process has been verified for various lignin types. In general, lignin can be processed by this method in approximately 11% gas, 35% char, and 43% oil. To provide the best economics, co-production related to electricity, heat, fuels, and chemicals is needed. Pyrolysis gas consists out of CO, H₂, CH₄, and CO₂, with an energy value (HHV) of 14.2 GJ/ ton (Larson 2004). Based on the price for natural gas of 1.87 €/ GJ, a value can be calculated of 27 €/ ton (Trading Economics). The LIBRA lignin pyrolysis oil is composed out of approximately 50-67% of an organic and 33-50% of an hydrophilic (acquatic) phase. Approximately 66% oligomeric phenolic substances and 34% monomeric phenols can further define the organic phase from which bio-fuels as well as bio-chemicals can be produced. No higher value chain has yet been established for the lignin pyrolysis aqueous products.

Different to the fast pyrolysis process with lignin, compared to lignocellulose is that the acquatic phase is much less heterogenic due to the absence of water-soluble thermal degradation products from carbohydrates. As a matter of fact, e.g. by starting with kraft and organosolv lignin from the pulp and paper or cellulose industry, respectively, it can be expected to obtain degradatives from mainly lignin. As a consequence of the lower heterogeneity, in this research new possibilities come available for an integrated biochemical value chain. It was researched if the present small hydrophilic aromatics could be valorized by an integrated biochemical value chain. Vispute and Huber (2009) describe the separation of commercially available bio oil from lignocellulosic biomass into a hydrophilic and hydrophobic phase by addition of water to the bio oil to separate a complex mixture of sugars, anhydrosugars, acetic acid, hydroxyacetone, furfural and small amounts of guaiacols to produce hydrogen, alkanes and polyols. However, the process required extensive purification and transformation steps due to the complexity of organic compounds in the samples.

In Wild et al (2009), a process of lignin valorisation for fuel and chemicals is described wherein the gas resulting from the pyrolysis of lignin is condensed in an ice-water cooled condenser at approximately 0°C, a room temperature electrostatic precipitation filter (ESP) and a freeze condenser (< 0°C). The process results in two fractions: a high boiling organic fraction and an aqueous fraction. Shen et al. (2015, Biochemical Advances 33(8):1799-1813) provided a review paper on thermochemical-biochemical hybrid processing of lignocellulosic biomass for producing fuels and chemicals, and Dias et al. (2017, Advances in Applied Science Research 8(1):94-109) on the exploration of microbial production of aromatic fine chemicals to overcome the barriers of traditional methods. Funkte et al. (2016, Biorescue Technology 200:905-913) reported on the production of char, indicating that significant value can be added by demineralizing and activating the char and the potential to increase the economic feasibility of fast pyrolysis. Jung et al. (2015, Applied Microbiology and Biotechnology 99:5217-5225) reported on the metabolic engineering of Klebsiella pneumoniae for the production of cis,cis-muconic acid. European patent application EP 2562249 A1 disclosed engineered host cell for the production of *cis,* cis-muconic acid from catechol, specific pseudomonas strains wherein at least parts of the cat operon are silenced, and a method for production of *cis,* cis-muconic acid using the engineered host cells or the pseudomonas strains. WO 2017/037013 A1 disclosed decomposition and conversion of organic educts into organic compounds with the help of biocatalysts, wherein a method of producing an organic product comprises i) fluid-assisted decomposition of an organic educt under sub- or supercritical conditions ii) obtaining an intermediate product from step i) iii) subjecting the intermediate product to biocatalytic conversion, by contacting the intermediate product obtained in step ii) with a biocatalyst, wherein said biocatalyst is a host cell selected from the group consisting of bacteria, yeast, filamentous fungi, cyanobacteria, algae, and plant cells; and further disclosed a host cell that can be employed in the disclosed methods.

Subject of the present invention is the provision of new processes to access nylon 6,6, adipic acid, terephthalic acid, optionally hexamethylenediamine, caprolactam, optionally caprolactone, or optionally 1,6-hexanediol and intermediates of these compounds - such as cis-cis muconic acid or adipic acid - starting from lignin.

It is known that the aerobic grown, gram-negative *Pseudomonads* strains are promising for the production of bio-based chemicals (Nikel *et al.,* 2014). *Pseudomonas putida* KT2440 is a non-pathogenic bacterium isolated from the soil that has a robust and versatile metabolism, based on which it is able to convert aromatic compounds (Nelson et al., 2002; Jiménez et al., 2002). Because of its strong redox balance, a high carbon yield can be generated in biotechnological processes and the strain is able to cope with oxidative stress (van Duuren *et al.,* 2013; Chavarría *et al.,* 2013). In this work, the BN6 strain was used to accumulate *cis,* cis-muconic acid in the medium from catechol. The described intermediate product can easily be hydrogenated to bio-adipic acid (1.000 €/ ton) (Niu et al., 2002; ICIS, 2016). To put the findings of the present invention into a more general context, the present inventors found that host cells, in particular bacterial host cells which express a functional catA polypeptide having catechol-1,2-dioxygenase activity and which do preferably not express a functional catB polypeptide having muconate cycloisomerase activity are useful for the accumulation of *cis,* cis-muconic acid in the medium, said *cis,* cis-muconic acid is generated from phenolic compounds, such as catechol or guaiacol. These phenolic compounds are obtainable by the processes of the present invention by processing lignin. Thus, in addition to new processes as described herein, disclosed herein are also host cells which can be applied in these new processes.

### Definitions

Lignin as used herein is a well-understood term by a skilled person and refers to polymers class of complex organic polymers that form important structural materials in the support tissues of vascular plants and some algae. The chemical structure of the polymer is based on at least two out of three phenylpropane monomers, namely coumaryl alcohol, coniferyl alcohol and sinapyl alcohol. An exemplary lignin structure is shown in formula (1). However, the exact structure may vary depending on the source and pre-treatment of the compound.

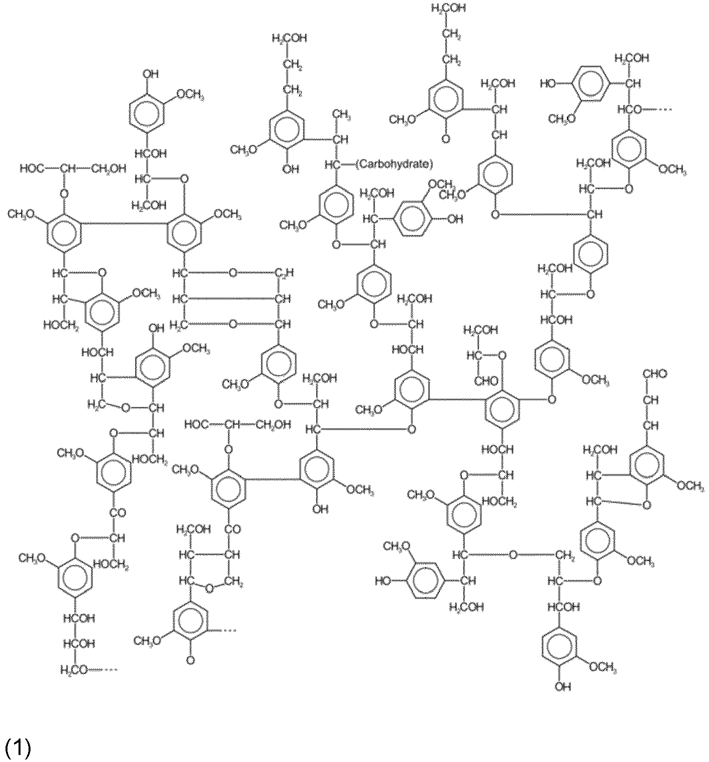

Generally, softwood (coniferous) lignin comprises between 80% w/w and 95% w/w coniferyl alcohol and between 5% w/w and 10% w/w synapyl alcohol in view of the sum of the monomers building the lignin. Generally, hardwood (deciduous) lignin contains between 40% w/w and 60% w/w coniferyl alcohol and between 40% w/w and 60% w/w synapyl alcohol in view of the sum of the monomers building the lignin. Generally, herbaceous lignin from grasses also includes besides between 75 % w/w and 85 % w/w coniferyl alcohol and between 15 % w/w and 35 % w/w synapyl alcohol up to 5% w/w coumaryl alcohol in view of the sum of the monomers building the lignin. Preferably, the term "lignin" refers to polymers wherein the amount of the sum of the three (or two) lignin building monomers are at least 90% w/w of the lignin, more preferably 95%, even more preferably more than 98%, such as 99%, 99,9% or even 100% w/w of the weight of the lignin. Further compounds which may be integrated into the lignin structure are, e.g. cinnamic acid or cinnamic aldehydes (which are, e.g., educts of the phenylpropane monomers), acidic residues, alkali or earth alkali ions (which may from a salt with the lignin polymer).

The term "lignin" includes naturally occurring lignin (a water-insoluble macromolecule comprised of at least two of the three monomers: p-coumaryl alcohol, coniferyl alcohol, and sinapyl alcohol) and also processed lignin derivatives, for example the following compounds obtainable from Sigma-Aldrich: alkali lignin (CAS Number 8068-05-1), organosolv lignin (CAS Number: 8068-03-9), hydrolytic lignin (CAS Number: 8072-93-3) lignosulfonic acid sodium salt (CAS Number: 8061-51-6) and guaiacol (CAS Number: 90-05-1). In one embodiment, the term "lignin" may also refer to "lignin sulfonate". In another embodiment, ammonium lignosulfonate is used as lignin compound. Lignin compounds are found in cell walls as a cement layer between cellulose strands. Lignins can be obtained from wood, like e.g. softwood (conifers) or hardwood. The molecular weight varies between about 5,000 and 10,000.

The term "lignocellulose" as used herein is a well-known term to any skilled person. Lignocellulose is composed of carbohydrate polymers (cellulose, hemicellulose), and the aromatic polymer lignin. The carbohydrate polymers comprise to at least 90% w/w or even contain different sugar monomers (six and five carbon sugars) and they are tightly bound to lignin.

The term "fast pyrolysis process" as used herein refers to a well-known process in which organic materials are rapidly heated to 350 °C - 600 °C. Under these conditions, organic vapors, pyrolysis gases and charcoal are produced. The vapors are condensed to bio-oil. Typically, 60-75 wt.% of the feedstock is converted into oil. An example of a fast pyrolysis is, e.g., described in WO 2011/159154.

The term "pyrolysis vapors" as used herein refers to pyrolysis products of a sample subjected to a fast pyrolysis process. When lignin is subjected to a fast pyrolysis process, the pyrolysis vapors will comprise aromatic compounds such as but not limited to phenolic compounds, such as catechol, phenol, o-, p-, n-cresol and guaiacol. Generally, the amount of catechol in the pyrolysis vapors is around 2 g to 6 g per 100 g lignin subjected to a fast pyrolysis reaction, depending on the lignin composition.

The term "aqueous phase" as used in relation to the fast pyrolysis process according to the invention, refers to a phase comprising at least 90% w/w, more preferably at least 90% w/w such as at least 95 % w/w water, in which the pyrolysis vapors are introduced. Minor components of the aqueous phase can be, e.g., buffer compounds or water miscible solvents such as low chain alcohol, e.g., methanol or ethanol. In one embodiment, the aqueous phase consists of water and a buffer. In another embodiment, the aqueous phase consists to 99,9 % w/w or more of water.

The term "directly introducing the resulting pyrolysis vapors into at least one aqueous phase" refers to the fact that vapors resulting from a fast pyrolysis reaction of lignin are introduced into water (or an aqueous phase mainly consisting of water) before the vapor condenses, i.e. in a way that the temperature of the vapors do not significantly decrease before entering the aqueous phase or the first aqueous phase (if more than one aqueous phases are part of the process). This can also mean, the time between formation of the vapor and introducing of the vapor into an aqueous phase is that short so that the loss in temperature of the vapor is only minimal. Alternatively, the line through which the vapor is directed into an aqueous phase is heated so that the loss in temperature of the vapors is minimized. Generally, a line transporting vapors from a reactor to a aqueous phase ends below the surface of the aqueous phase so that the pyrolysis vapors directly bubble or stream (depending from the transportation speed of the vapor in the line and/or the crosssection dimension of a transporting line compared to the amount of vapor to be transported) into the aqueous phase.

The term "acquatic" phase as used herein refers to the condensed, water soluble pyrolysis vapors (in contrast to the organic phase) or optionally to the water soluble pyrolysis vapors which were introduced into an aqueous phase, i.e. the aqueous phase in which water soluble components of the vapors are dissolved, e.g., catechol, phenol, o-, p-, n-cresol and/or guaiacol.

The term "at least one acquatic phase" as used herein refers to the fact that after fast pyrolysis according to the invention the resulting vapors may be condensed in more than one vessel, such as a cooling trap, e.g., an impinger, or introduced and solved in more than one aqueous phase (e.g., two, three, or four) to ensure to dissolve most of the catechol comprised in the vapors into the water. In the case more than one acquatic phase is produced, one can combine two or more acquatic phases to have one acquatic phase or analyse/use at least one separated acquatic phase.

The term "organic" phase as used herein is known to the skilled person in the field of pyrolysis, especially in the field of LIBRA. It refers to the condensed vapours which are essentially not soluble in water, preferably of course not soluble in water at all.

The term "purifying catechol" as used herein refers to any method which is suitable to remove other compounds from the acquatic, catechol comprising phase and/or suitable to concentrate catechol by, e.g., reducing the volume of the solvent. In one preferred embodiment, the purification is distillation, more preferably steam distillation.

The term "cis-cis-muconic acid" as used herein refers to (2Z,4Z)-2,4-Hexadienedioate, also referred to as muconate or cis-cis-muconate according to formula (2) which can advantageously be used, e.g., as raw material for new functional resins, pharmaceuticals, and agrochemicals.

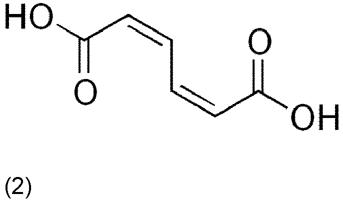

For example, cis-cis-muconic acid can be easily converted to adipic acid, caprolactam, and terephthalic acid which are used as a commodity chemical for production of value-added or valuable products including nylon-6 (fibers and resins), nylon-6,6, polyurethane, PVC, polyethylene terephthalate (PET), polyesters and/or polyamides. Furthermore, highly stereoregular polymers, useful functional resins, can be produced through topochemical polymerization of muconic acid esters. Verrucarin is an antibiotic that can be synthesized from cis-cis-muconic acid by organic synthesis.

It is in particular envisaged that cis-cis-muconic acid as obtained from the lignin processing method of the invention is white in colour, which is envisaged to greatly increase its economic value. Without wishing to be bound by theory, this advantageous property of the end product is thought to be due to its substantially complete chemical conversion from catechol.

The term "biocatalyst" refers to a host cell which (preferably constitutively) expresses an enzyme showing catechol-1,2-dioxygenase activity (EC 1.13.11.1) (catA).

The term "purifying cis-cis muconic acid" as used herein refers to any known method to separate and concentrate cis-cis muconic acid from its biocatalyst, e.g. by adjusting the pH to precipitate cis-cis muconic acid from water.

If not explicitly defined differently, % values refer to weight percentages % w/w.

The term "substantially" herein will be understood by the person skilled in the art. The term "substantially" may also include embodiments with "entirely", "completely", "all", etc. Hence, in embodiments the adjective substantially may also be removed. Where applicable, the term "substantially" may also relate to 90% of the related value or higher, such as 95% of the related value or higher, especially 99% of the related value or higher, even more especially 99.5% of the related value or higher, including 100%.

The term "comprise" includes also embodiments wherein the term "comprises" means "consists of".

All embodiments disclosed herein can of course be combined. Combinations or parts of combinations which would contradict any law of nature are excluded.

### Description of the Figures

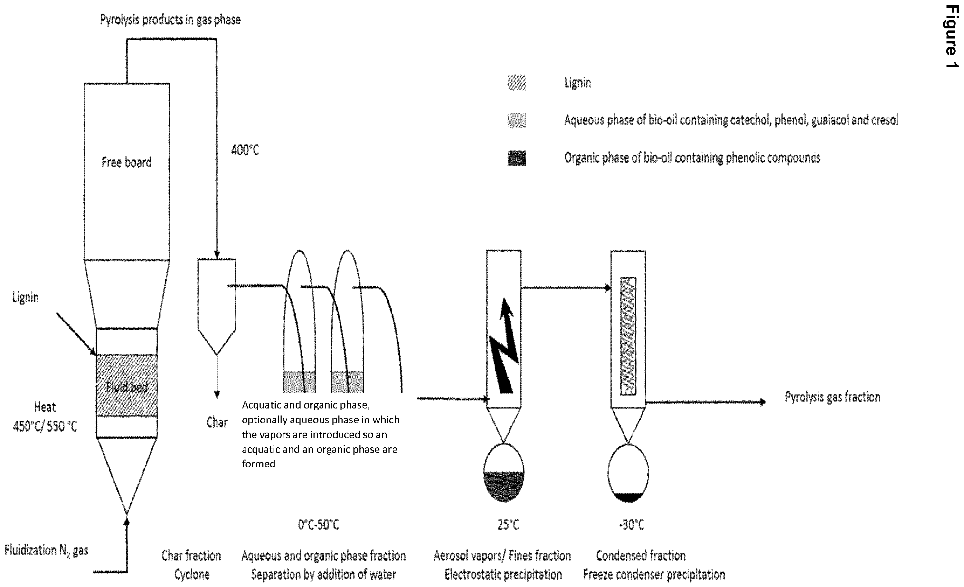

**Figure** 1 shows a scheme of a fast pyrolysis process, based on bubbling fluid bed pyrolysis, developed by ECN, which is named LIBRA (lignin biorefinery approach). Based on the solubility capacity of water, mainly catechol, phenol, guaiacol and cresol were recovered in the acquatic phase from the pyrolysis bio-oil. Until now for these small hydrophilic compounds no specific value chain was developed.

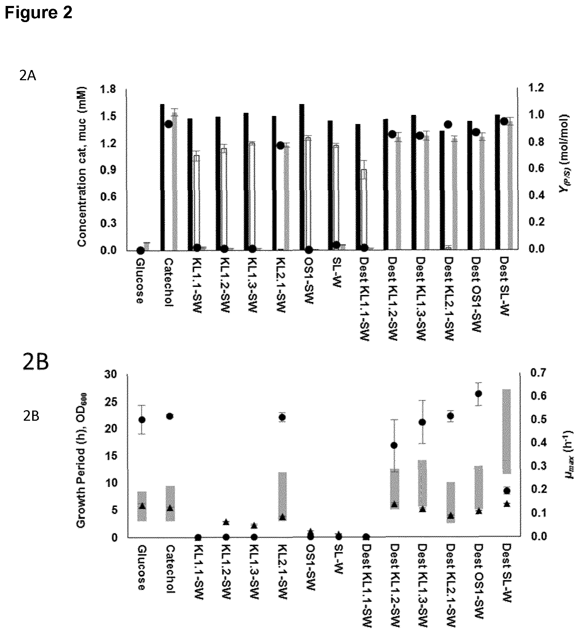

**Figure 2** The ability of *P*. *putida* BN6 to convert the extracted catechol of the aqueous phase of the bio-oil obtained after pyrolysis and before and after steam bath distillation. In figure 2A the start (black) and end (white) concentration of catechol as well as the end concentration of *cis, cis*-muconic acid (grey) are shown. The yield (*Y*_{*(P*/*S)*}) is represented by a circle. In figure 2B the growth period (grey), the maximum measured OD600 value (tringle) and the *µₘₐₓ* (circles) are shown.

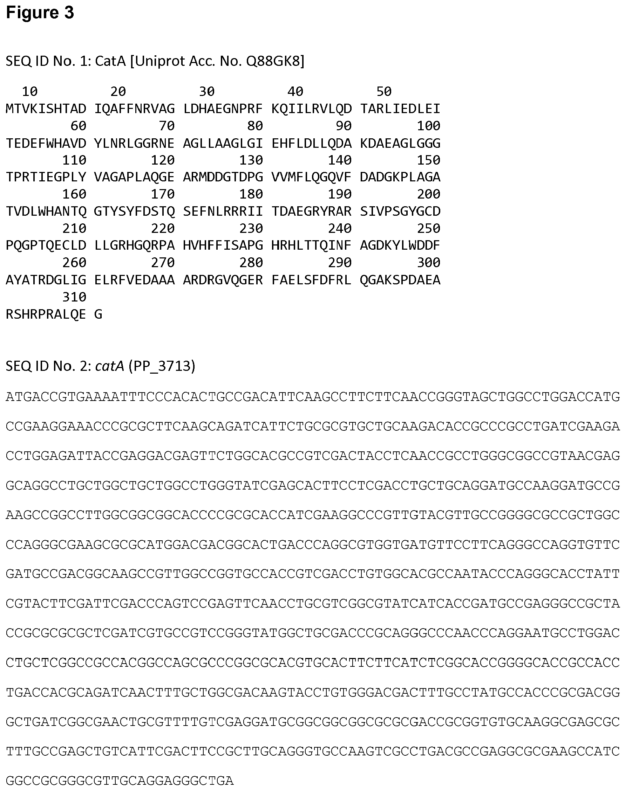

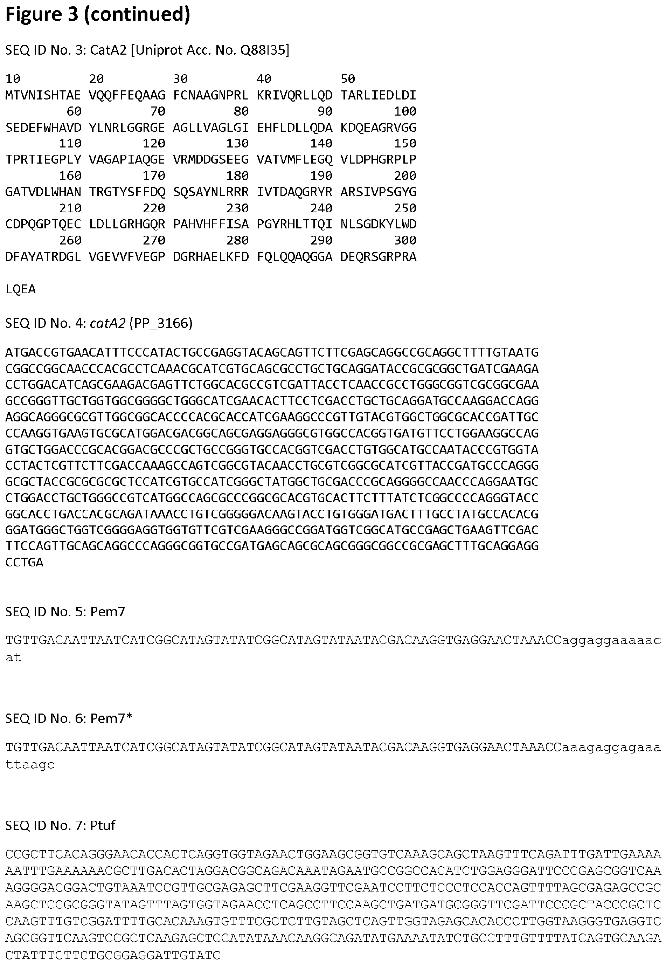

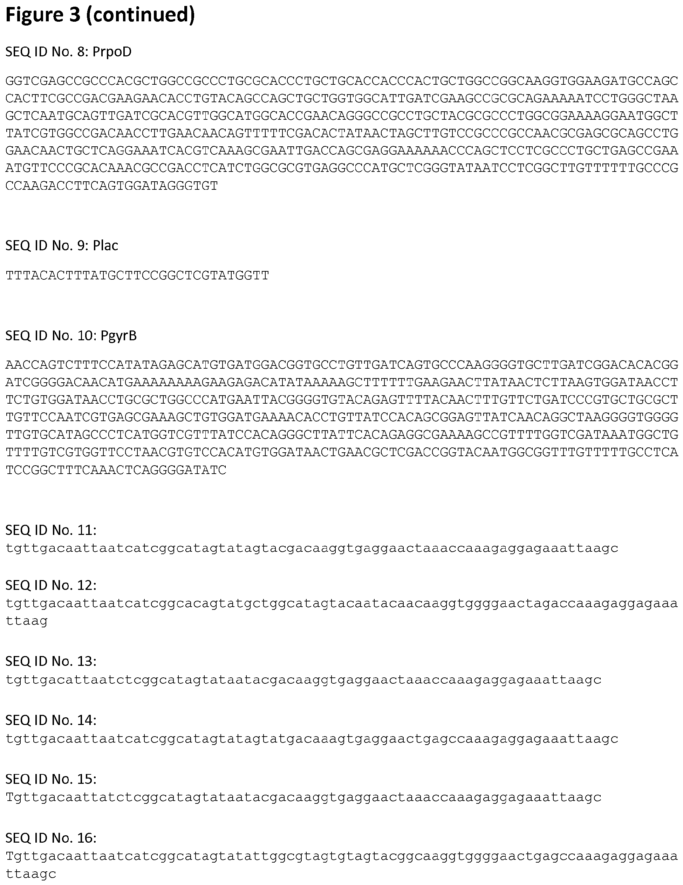

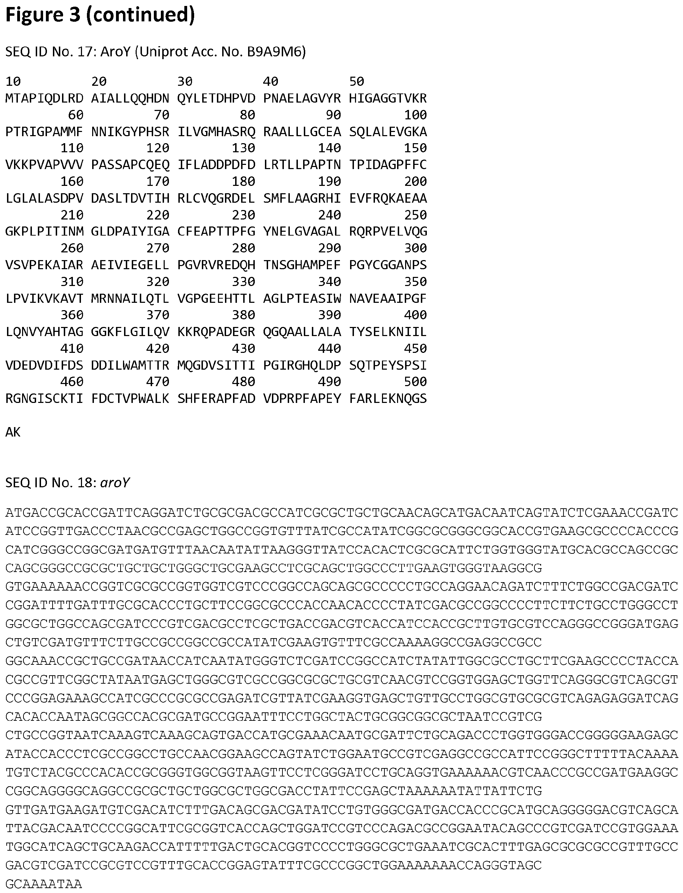

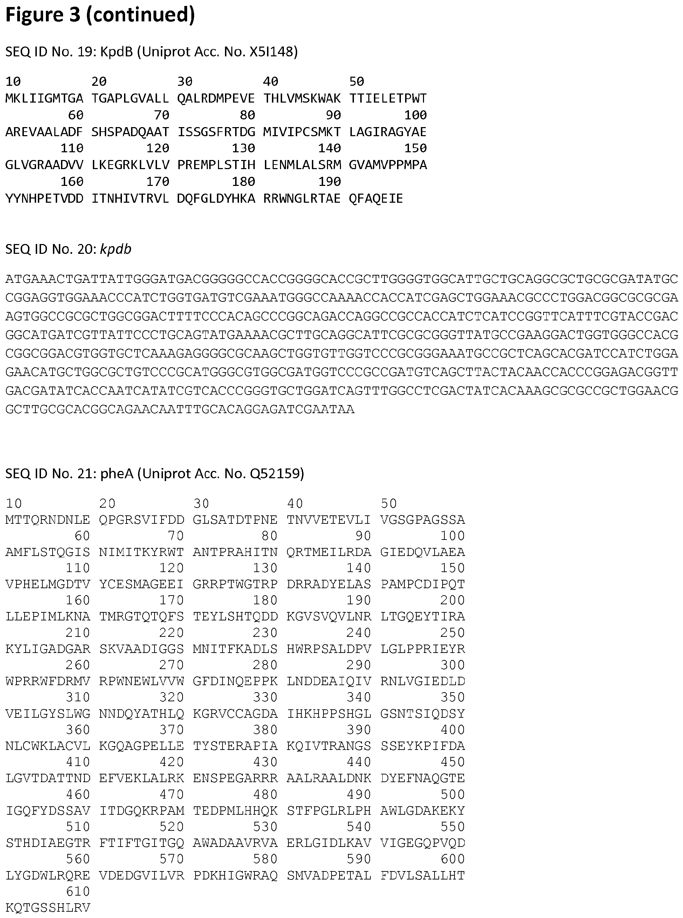

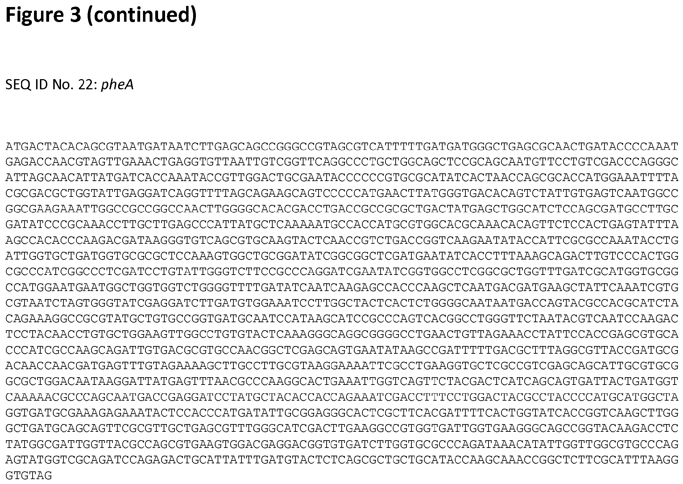

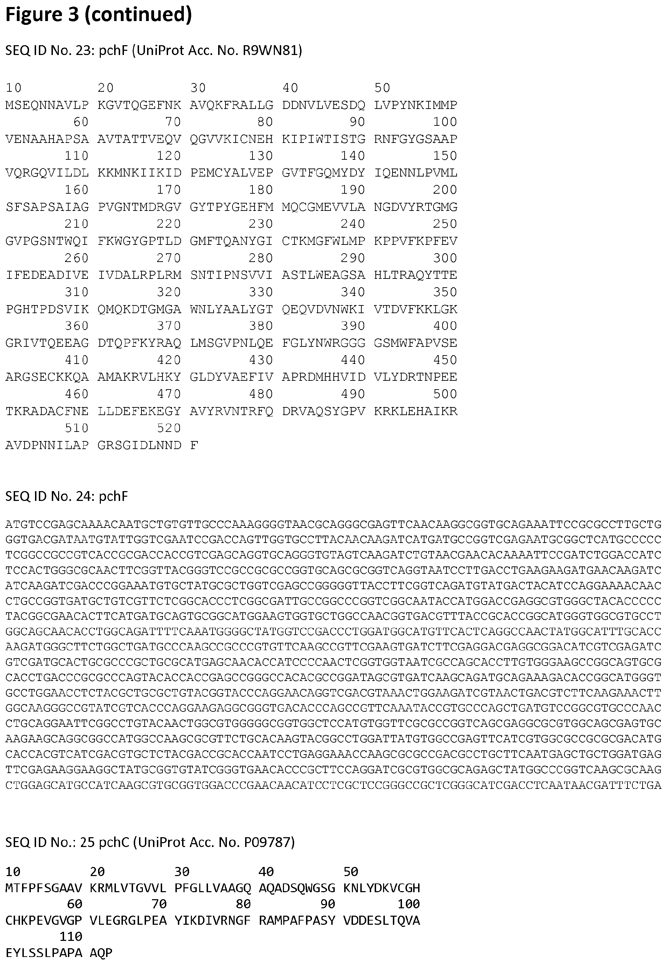

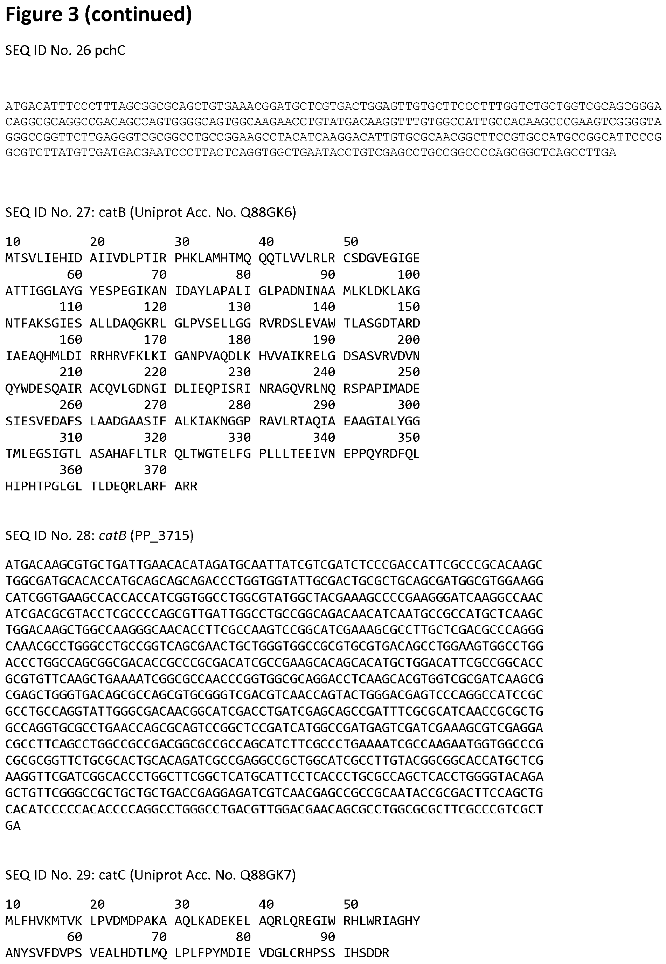

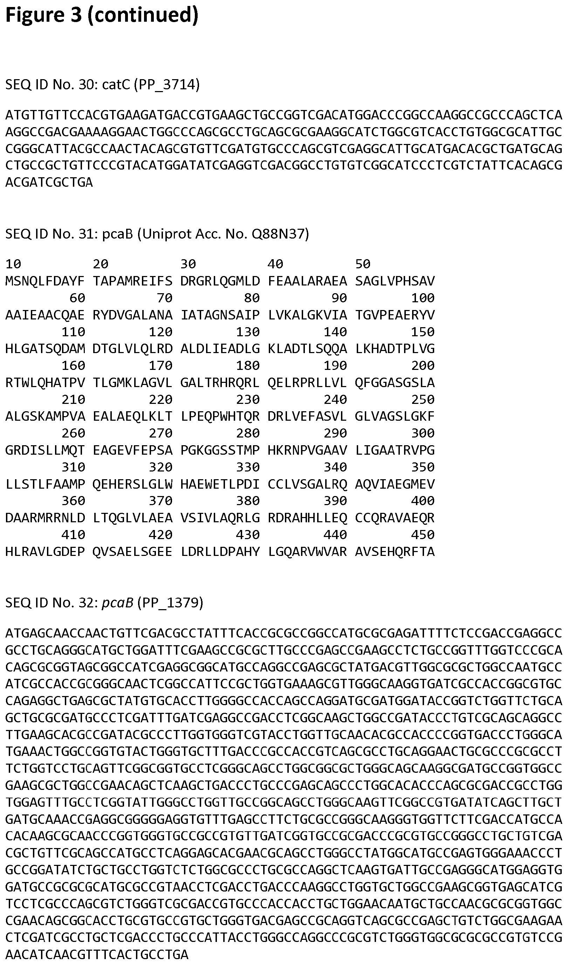

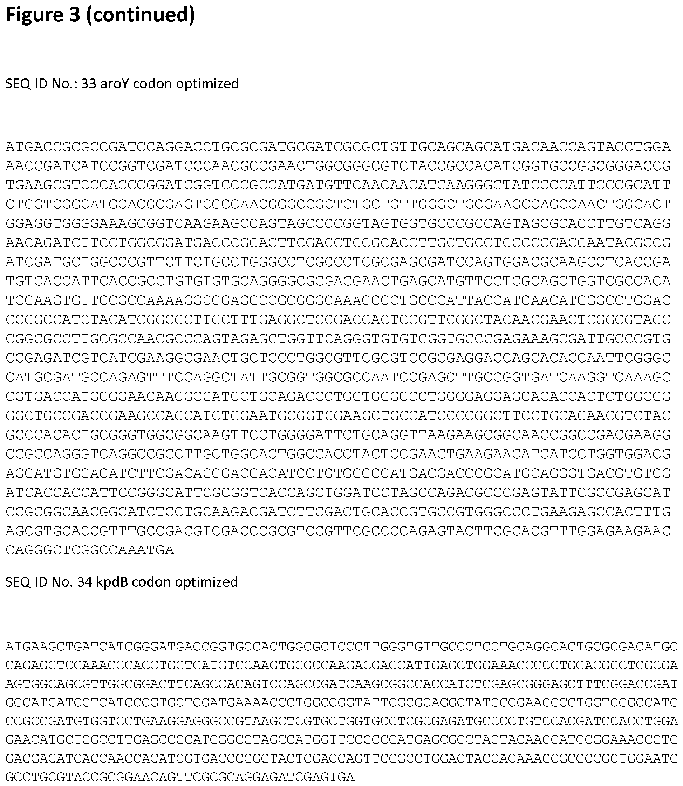

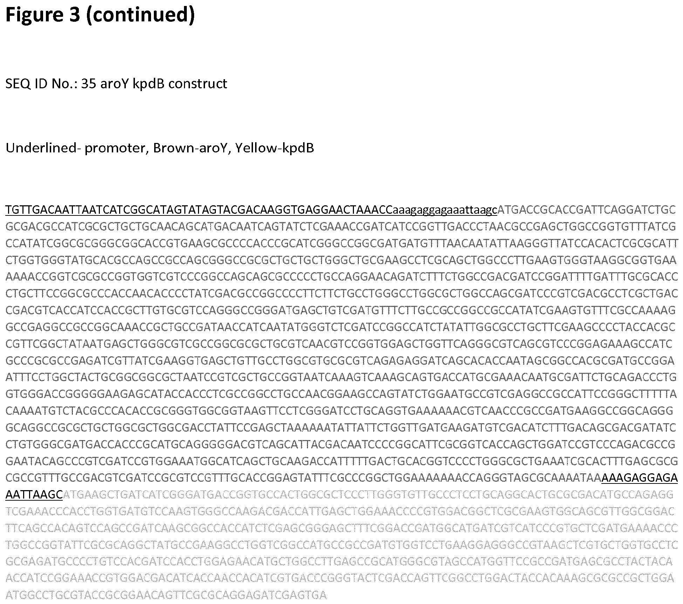

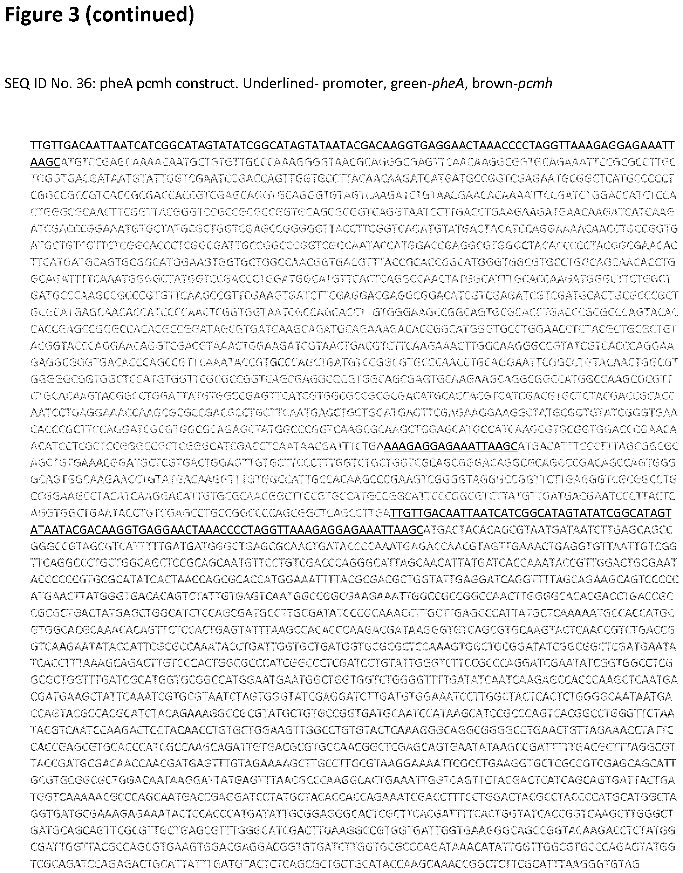

**Figure 3** is a depiction of SEQ ID No.1 to SEQ ID No. 16 referenced herein.

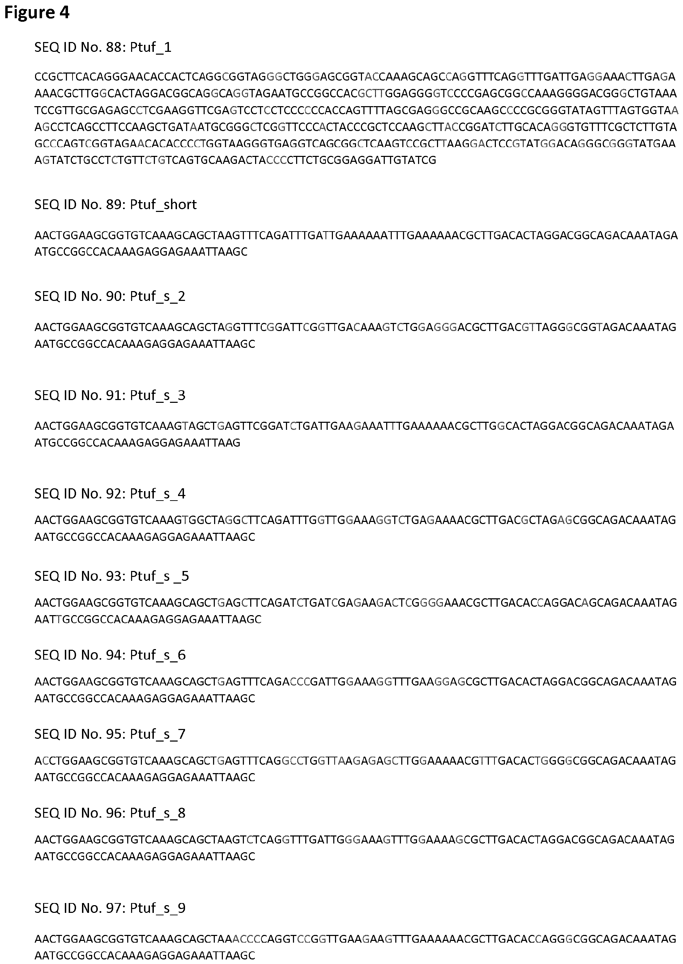

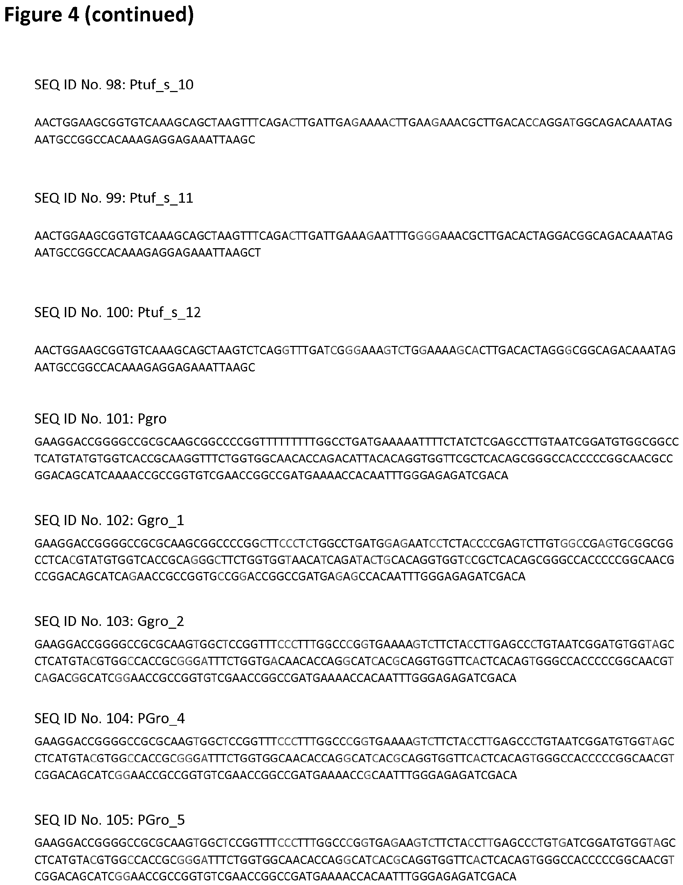

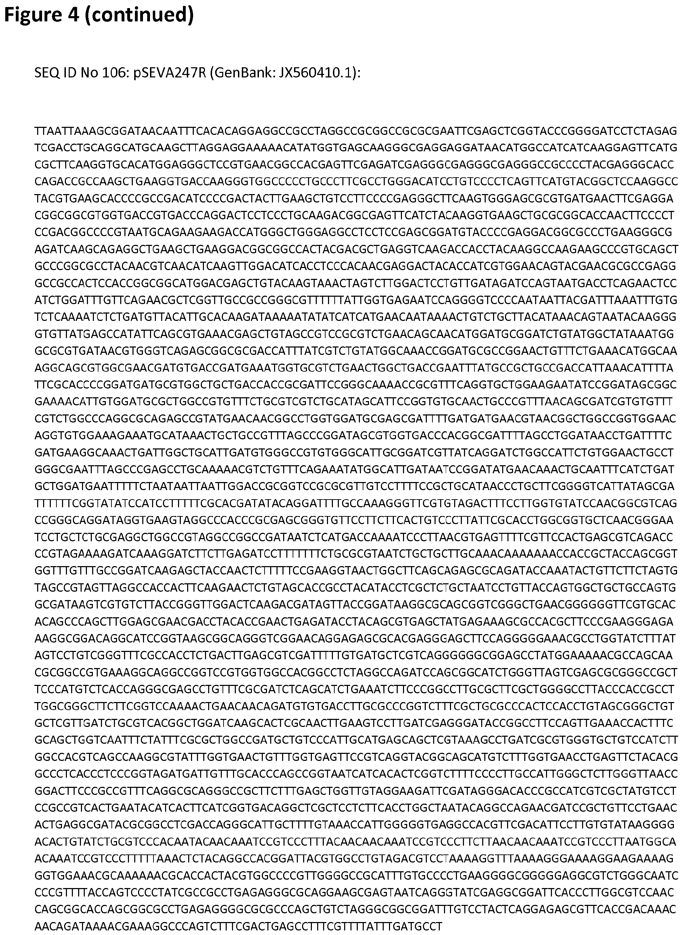

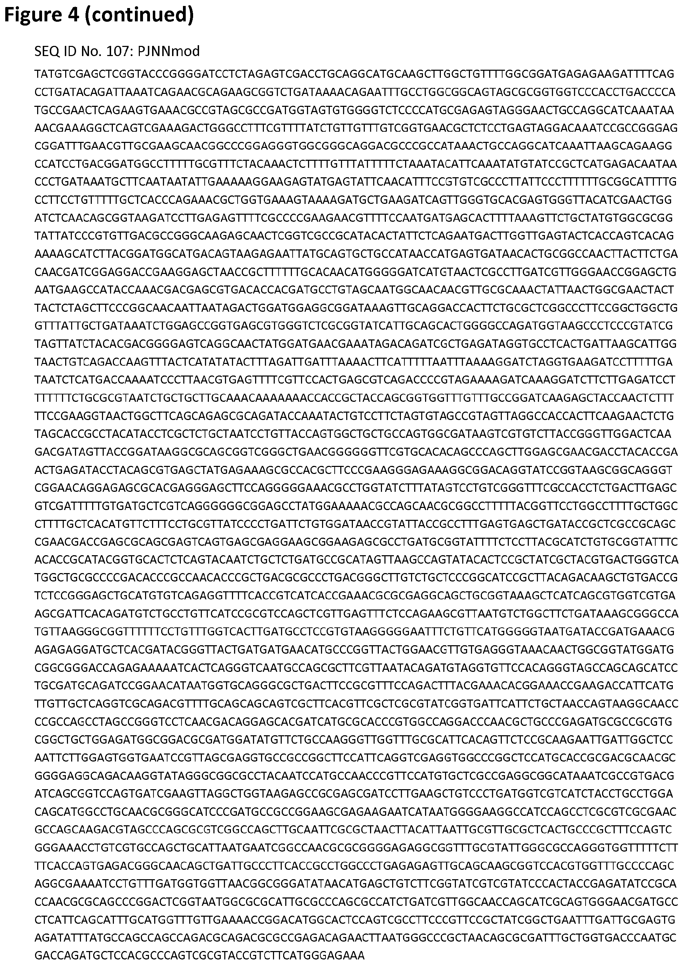

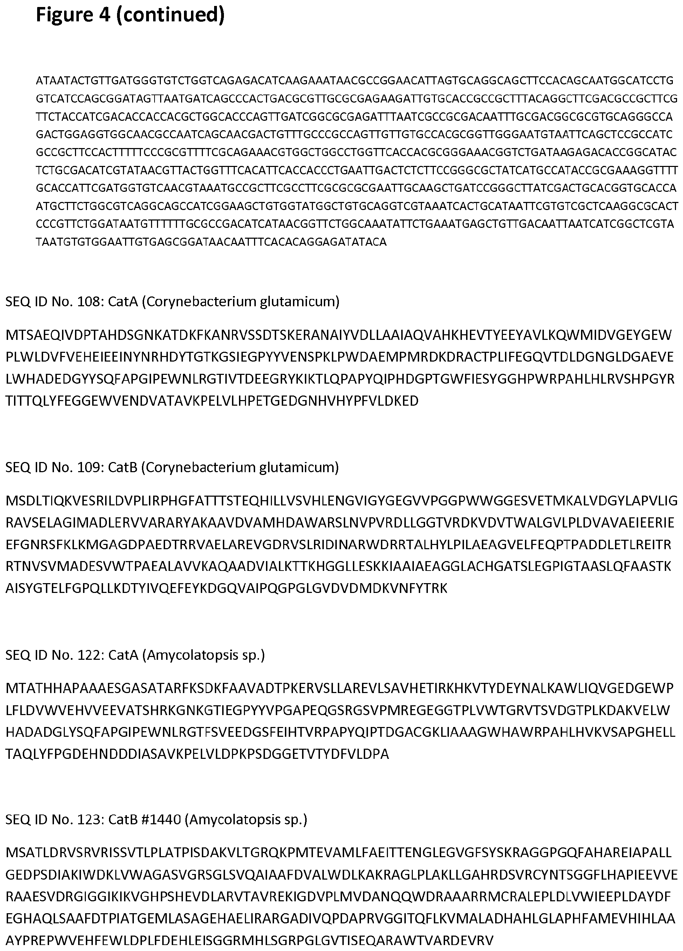

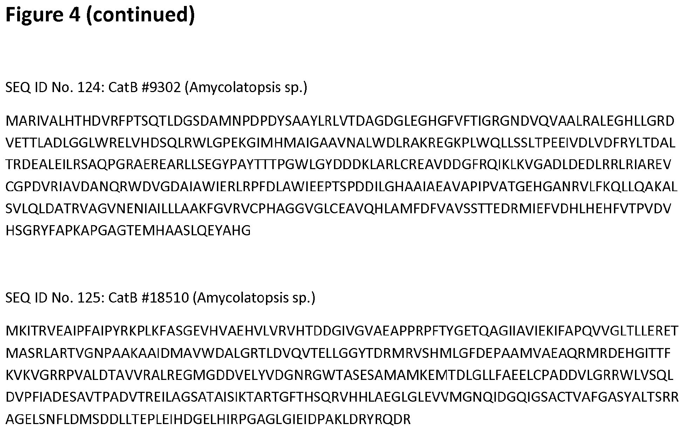

**Figure 4****:** is a depiction of sequences referenced herein.

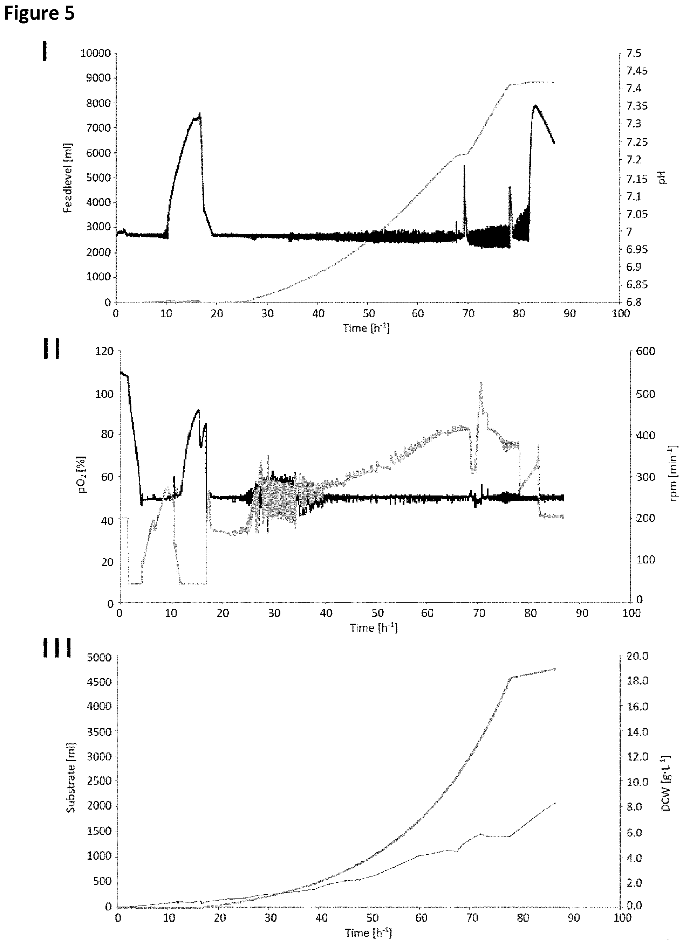

**Figure 5****:** The feedstock demands of benzoate (1.2 M) and sodium hydroxide (2.4 M) in E-2 mineral medium (grey) in relation to the measured pH (black) during the pH-stat fed-batch process (I). The stirring speed (grey) related to the PO₂ in the medium (black) (II). The addition of glucose (1.4 M) and ammonium sulfite (72 g L⁻¹) in E-2 mineral medium (grey) and the determined dry cell weight (black) (III).

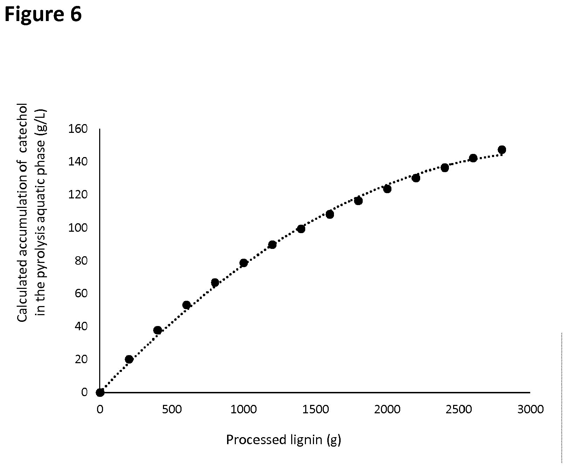

**Figure 6****:** Calculated accumulation of Catechol in the pyrolysis acquatic phase.

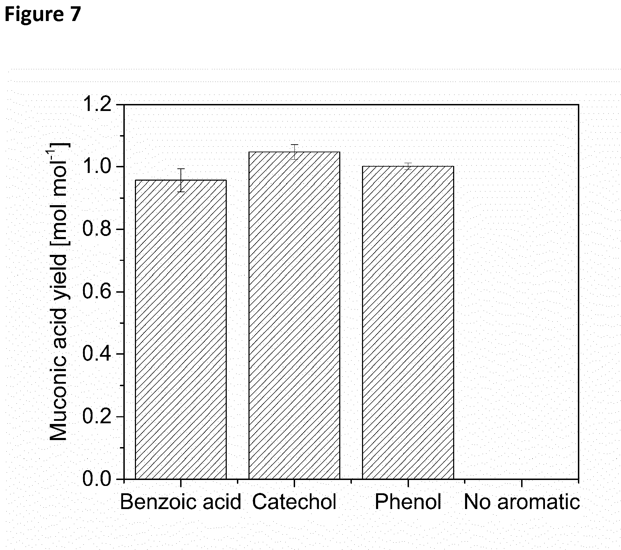

**Figure 7****:** Production of *cis-cis*-muconic acid (MA) from small aromatics, using *Corynebacterium glutamicum* LIMA-1. The yield for MA on benzoic acid (20 mM), catechol (10 mM), and phenol (5mM) was obtained from the differential measurement of substrates and the product at the beginning and after 24 hours of incubation. The data represent mean values and standard deviations from three biological replicates.

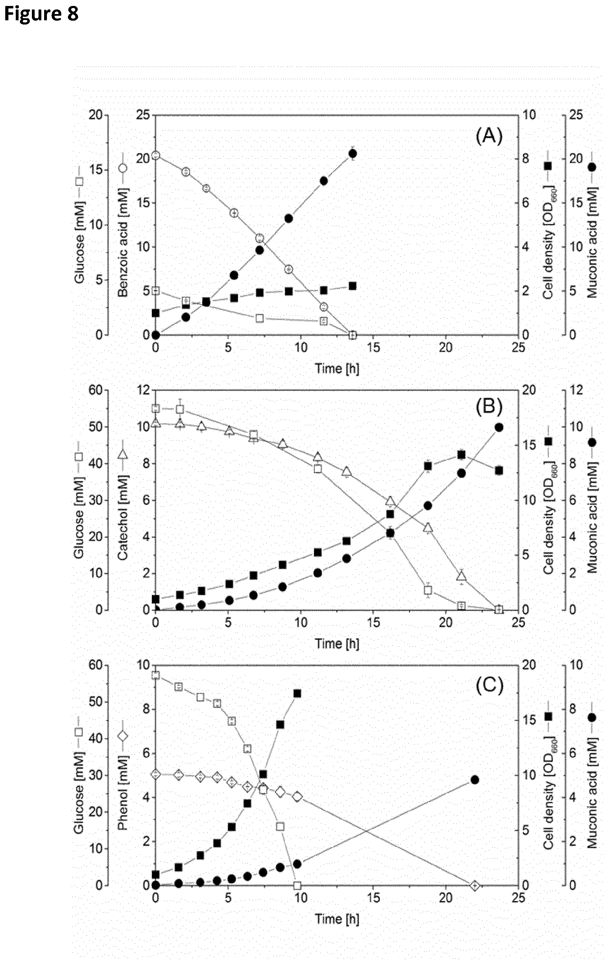

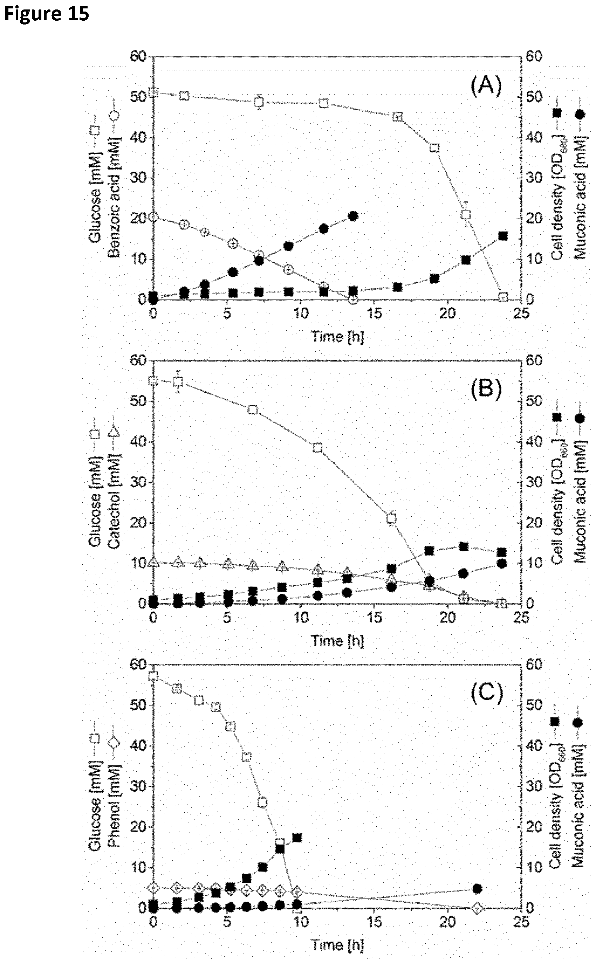

**Figure 8****:** Kinetics and stoichiometry of *cis-cis*-muconic acid (MA) production, using the first generation producer *Corynebacterium glutamicum* LIMA-1. The aromatics benzoic acid (A), catechol (B) and phenol (C) were used for production. In addition, glucose was added as growth substrate. The data represent the culture profiles until the depletion of the aromatic. The full culture profiles are given in the supplement (**Fig. 15**). The concentration of glucose was corrected to represent the amount consumed during this phase. The data comprise mean values and deviations from three biological replicates.

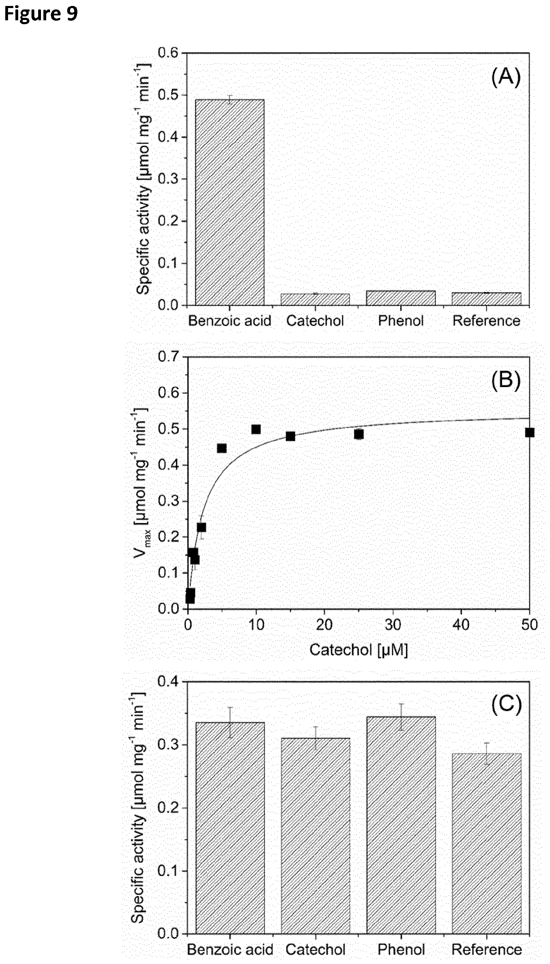

**Figure 9****:** Characteristics of catechol-1,2-dioxygenase (CatA) in *Corynebacterium glutamicum,* grown on different aromatics. The data comprise the specific enzyme activity of the first generation producer *C*. *glutamicum* LIMA-1 (A), the kinetics of the enzyme with a fit of the experimental data from benzoate-grown cells to a Michaelis-Menten type kinetics (B), and the specific enzyme activity of the second generation producer *C*. *glutamicum* LIMA-2 (C). The data represent mean values and standard deviations from three biological replicates.

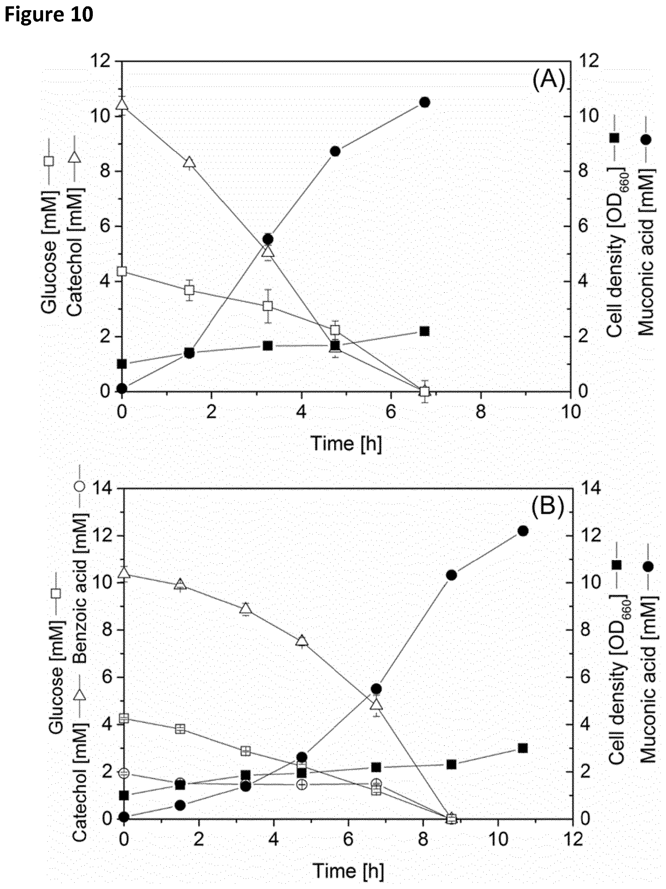

**Figure 10****:** Metabolic engineering of *Corynebacterium glutamicum* for *cis*-*cis*-muconic acid (MA) production from the lignin-aromatics catechol and phenol. The data comprise the cultivation profile of the second generation producer LIMA-2, using 10 mM catechol for production (A), of the first generation producer LIMA-1, using 10 mM catechol for production plus 2 mM benzoic acid for induction of *catA* expression (B), and of LIMA-2, using 5 mM phenol. In addition, glucose was added as growth substrate. The data represent the culture profiles until the depletion of the aromatic. The full culture profiles are given in the supplement (**Fig. 15**). The concentration of glucose was corrected to represent the amount consumed during this phase. The data comprise mean values and deviations from three biological replicates.

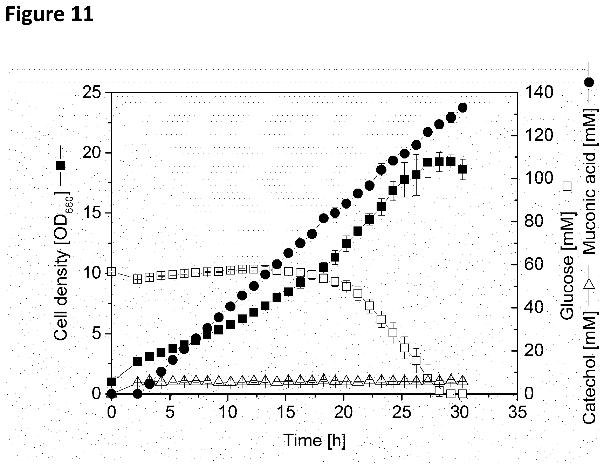

**Figure 11****:** Production of *cis-cis*-muconic acid (MA) with feeding of catechol. The production was conducted in a shake flask. Metabolically engineered *Corynebacterium glutamicum* LIMA-2 was grown on glucose minimal medium for the first 2 hours. Then, the feeding was started. Every hour, pulses with 5 mM catechol and 0.5 g L⁻¹ glucose, were added. In addition, the pH was controlled above 7.0 by addition manual control, adding appropriate volumes of 10 mM NaOH. The black arrow indicates the time point, when the glucose feed was stopped. The data represent mean values and standard deviations from three biological replicates.

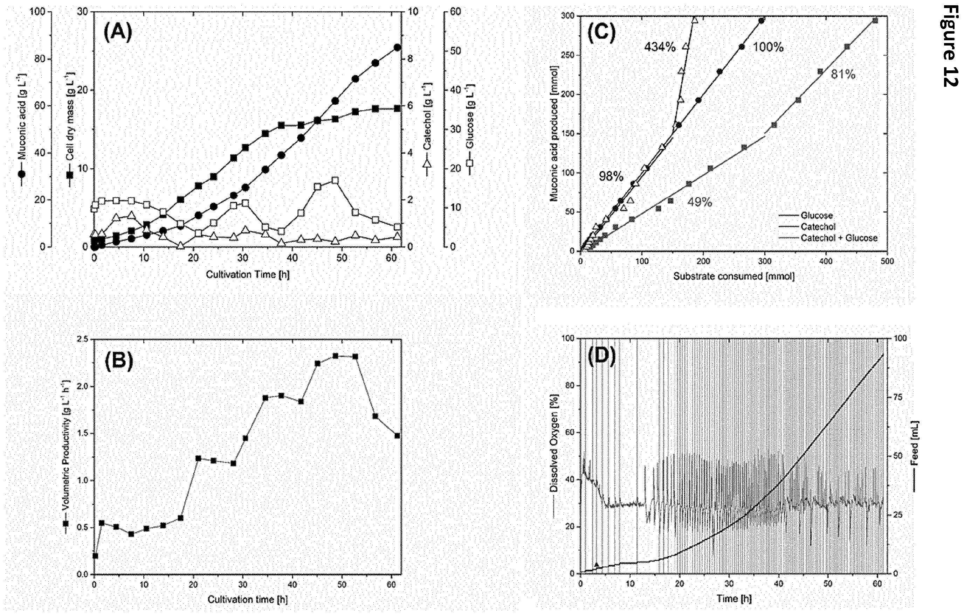

**Figure 12****:** Fed-batch production of *cis-cis*-muconic acid (MA) from catechol by metabolically engineered *Corynebacterium glutamicum* LIMA-2. Substrate consumption, growth and MA formation (A). Volumetric productivity (B). Yields for MA from catechol, and for MA from catechol plus glucose (C). Pulse-wise feeding of catechol (D). Glucose was added continuously to maintain the glucose level in the range between about 5 to 15 g L⁻¹ (A). The vertical lines vertical lines represent individual catechol feed pulses (D). The feed frequency was variably adjusted, depending on the signal of dissolved oxygen, which precisely indicated the time period for catechol depletion. As example, feeding was halted once during the initial phase, corresponding to transient catechol accumulation and was accelerated later in response to the faster conversion. The data represent mean values from two replicates.

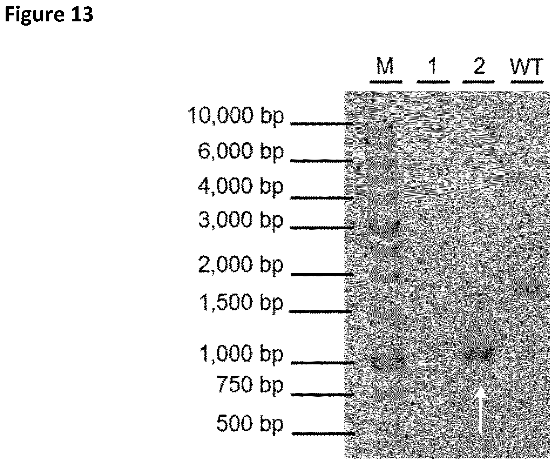

**Figure 13****:** Confirmation of the deletion of the *catB* gene in *Corynebacterium glutamicum* ATCC 13032, using colony PCR. To this end, *C*. *glutamicum* ATCC 13032, had been transformed with the integrative plasmid pClik int *sacB* Δ*catB,* followed by recombination and selection. The primers Δ*catB* TS1 FW and *ΔcatB* TS2 RV were used for the PCR **(Table 1).** The positive clone, indicated by the white arrow, revealed the small fragment, size expected for the deletion. It was designated *C*. *glutamicum* LIMA-1. M, 1 kb DNA ladder; 1, blank; 2, positive clone; WT, wild type.

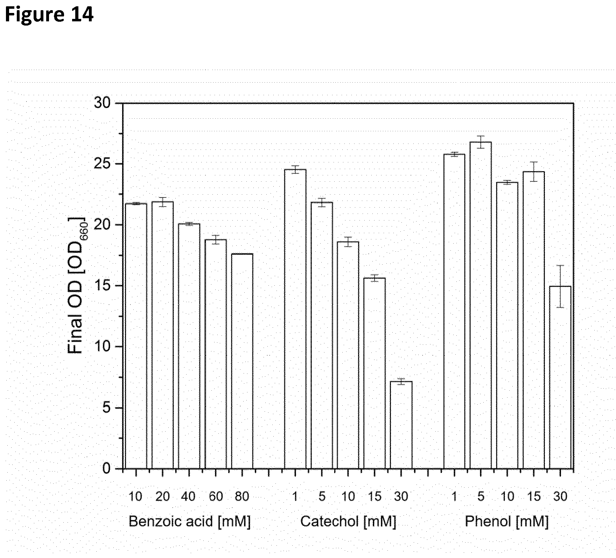

**Figure 14****:** Tolerance of *Corynebacterium glutamicum* LIMA-1 against benzoic acid (A), catechol (B), and phenol (C). In addition, glucose was added as growth substrate. The final cell concentration was measured after 24 hours of cultivation. The data represent mean values and standard deviations from three biological replicates.
**Figure 15****:** Kinetics and stoichiometry of *cis-cis*-muconic acid (MA) production, using the first generation producer *Corynebacterium glutamicum* LIMA-1. The aromatics benzoic acid (A), catechol (B) and phenol (C) were used for production. In addition, glucose was added as growth substrate. The data represent mean values and standard deviations from three biological replicates.

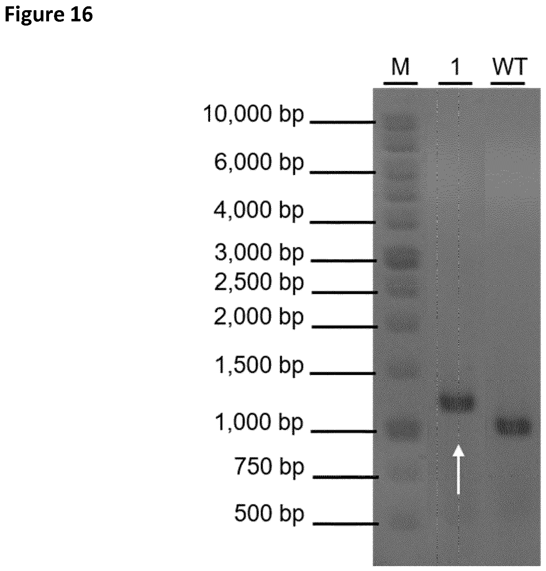

**Figure 16****:** Confirmation of overexpression of the *cat*A gene under control of the *tuf* promoter in *Corynebacterium glutamicum* LIMA-2, using colony PCR. To this end, *C*. *glutamicum* LIMA-1 had been transformed with the integrative plasmid pClik int *sacB P_{tuf}catA*, followed by recombination and selection. The primers *P_{ef-tu}-catA* TS1 FW and Pₑ*_{f-tu}catA* TS2 RV were used for the PCR **(Table 1).** The positive clone, indicated by the white arrow, revealed the increased fragment size, expected for the promoter exchange. M, 1 kb DNA ladder; 1, positive clone; WT, wild type.

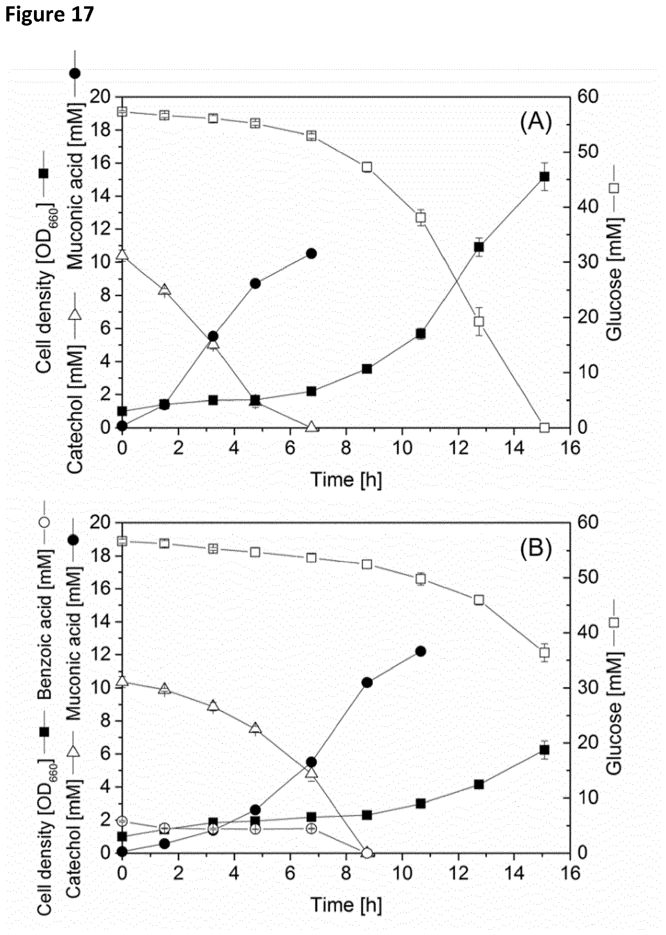

**Figure 17****:** Kinetics and stoichiometry of *cis-cis*-muconic acid (MA) production, using the second generation producer *Corynebacterium glutamicum* LIMA-2 on catechol (A) and the first generation producer *C*. *glutamicum* LIMA-1 on catechol and benzoic acid (B). Glucose was added as growth substrate. The data represent mean values and standard deviations from three biological replicates.

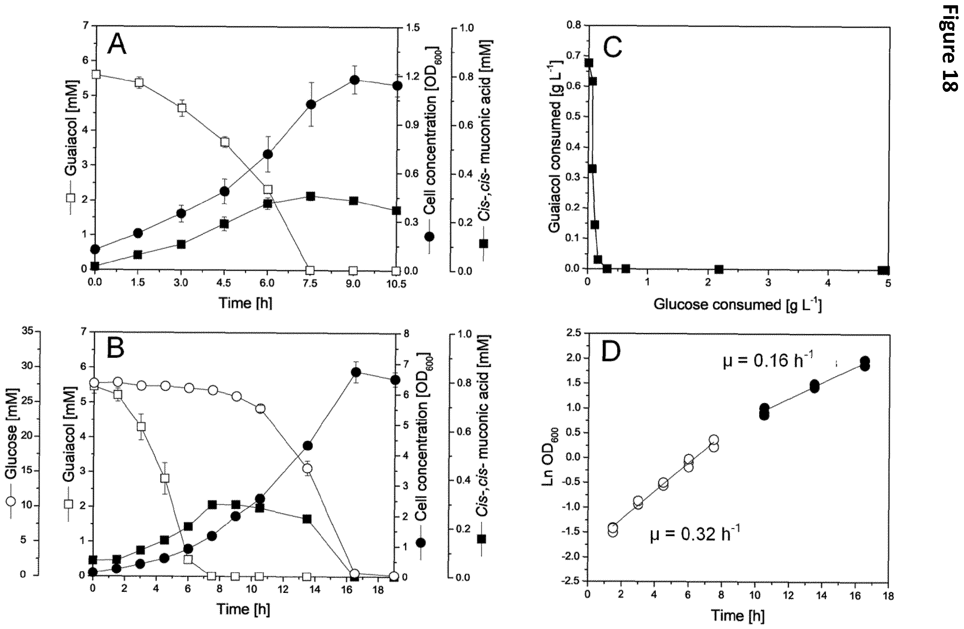

**Figure 18****:** Growth physiology of *Amycolatopsis* sp. ATCC 39116 on guaiacol (A) and on a mixture of guaiacol and glucose (B, C, D). The data reflect mean values and deviations from three replicates.

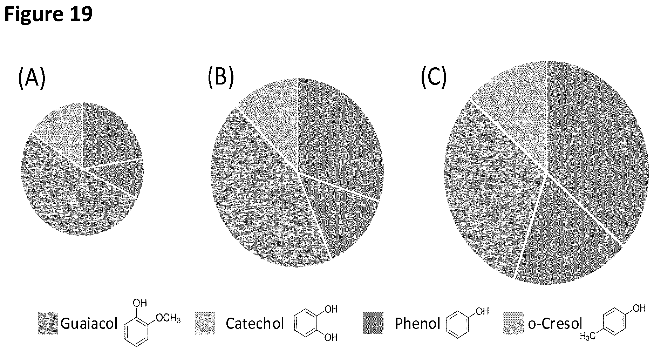

**Figure 19****:** Hydrothermal conversion of lignin into small aromatics. Softwood lignin, devoid of carbohydrates (Indulin AT), was treated in a pressure reactor in the presence of an aqueous phase for 20 minutes at 330°C and 130 bar (A), 350 °C and 165 bar (B), 370°C and 210 bar (C). After cooling of the reactor and clarification of the hydrolysate from insoluble particles, its analysis revealed guaiacol, catechol, phenol and o-cresol. The relative area for each condition reflects the relative fraction of the different aromatics. The circle size visualizes the total amount recovered. Hereby, the largest circle at 370 °C (C) corresponds to a yield of 12% and the relative yields at the other temperatures relate to the size of the corresponding circles (A, B).

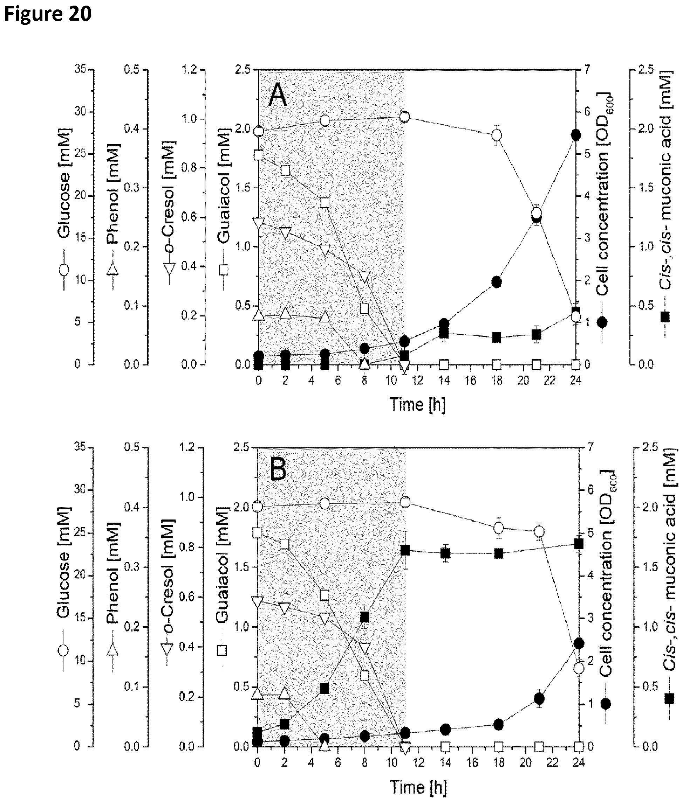

**Figure 20****:** Utilization of softwood lignin hydrolysate by the wildtype *Amycolatopsis* sp. ATCC 39116 (A) and the engineered producer *Amycolatopsis* sp. ATCC 39116 MUC-2 (B). The lignin hydrolysate was obtained by hydrothermal conversion, followed by aromatics enrichment using distillation. It was added to a mineral salt medium together with small amounts of glucose. The data reflect mean values and deviations from three replicates.

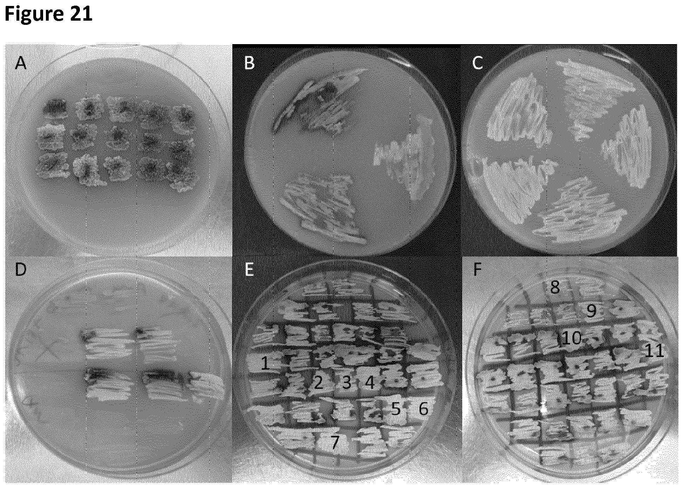

**Figure 21****:** Genetic engineering of *Amycolatopsis* sp. ATCC 39116 using conjugation in the transformation step and blue-white screening for verification of desired mutations. For genomic deletion of AATC3_020100009302, transformants were streaked in squares and overlaid with X-Gluc. This identified positive clones, which had integrated the plasmid pKG1132 MUC1 in their genome by a single crossover, by the blue color (A). Subsequent incubation without selection pressure allowed for the double crossover, which was again verified by blue-white screening (B, C). White colonies indicated the loss of the pKG1132 MUC1 plasmid and either exhibited the desired deletion or were re-modified to the original wildtype. The same procedure was also conducted for deletion of AATC3_020100018510, using the plasmid pKG1132 MUC2. Corresponding data are shown for the screening of single crossover mutants (D), and the subsequent screening for double crossover mutants (E, F).

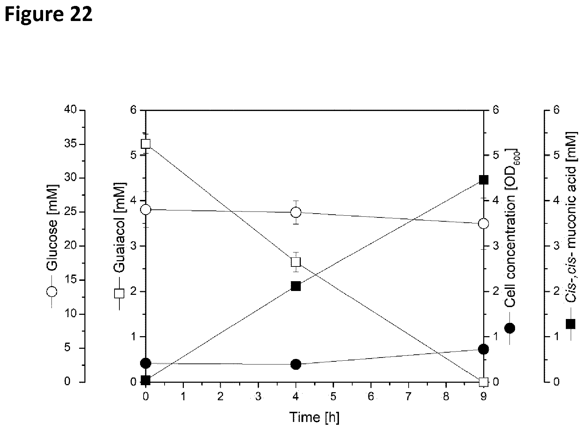

**Figure 22****:** Growth physiology the engineered producer *Amycolatopsis* species ATCC 39116 MUC-2 on a mixture of guaiacol and glucose.

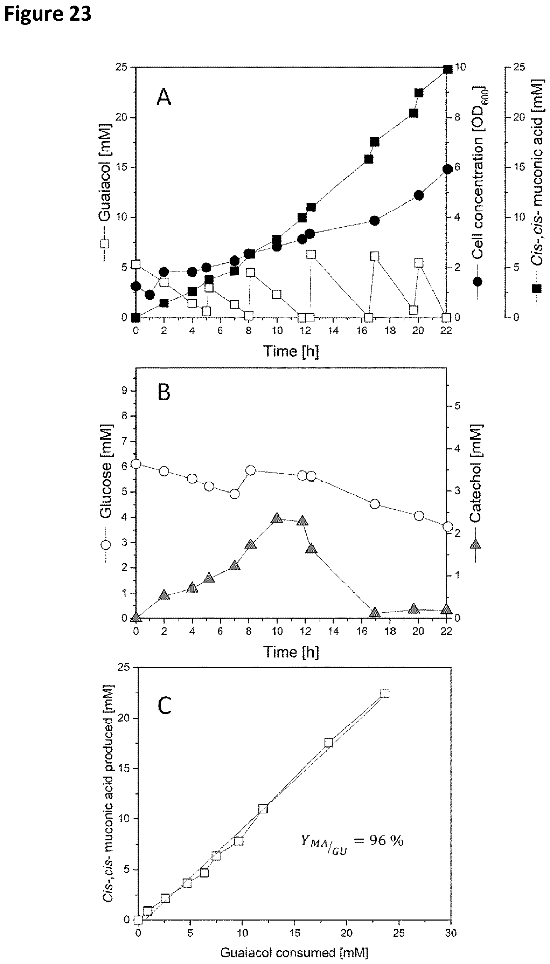

**Figure 23****:** Production performance the engineered producer *Amycolatopsis* species (ATCC 39116) MUC-2 in a fed-batch process. An initial batch phase on 5 mM guaiacol was followed by an extended feeding phase, adding further amounts of guaiacol pulse-wise. In addition, glucose was present as growth supporting nutrient.

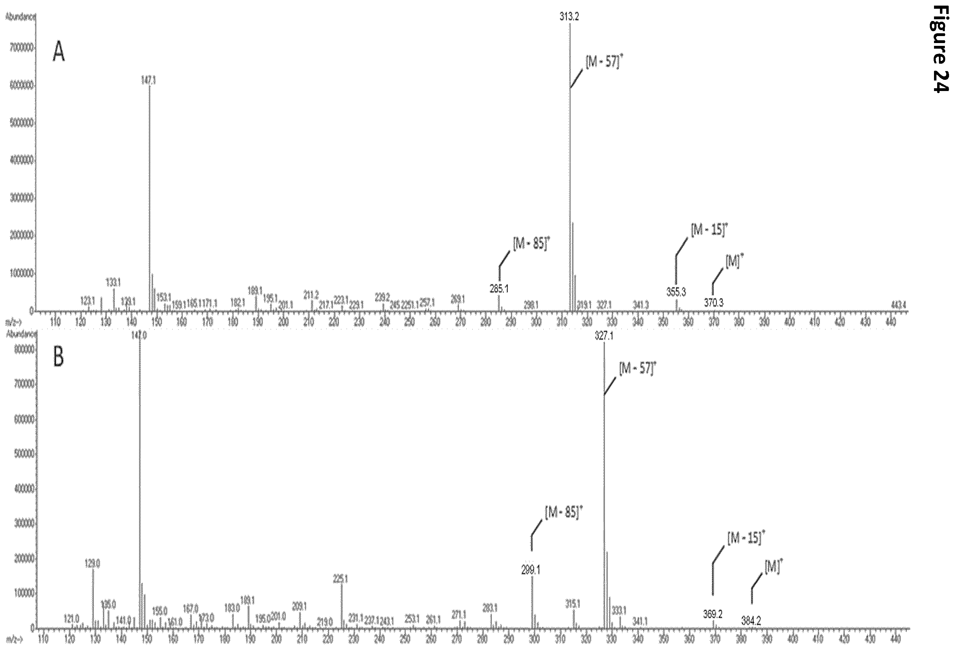

**Figure 24****:** Structural analysis of the metabolic product, formed by the engineered producer *Amycolatopsis* species (ATCC 39116) MUC-2 from *o*-cresol. GC-MS mass spectrum of *t*-butyl-dimethylsilyl derivatised pure muconate (MA) (A) and of the *t*-butyl-dimethylsilyl derivatised product, formed during the incubation of the cells on *o*-cresol (B). MA eluted after 9.5 minutes. The substance, formed from o-cresol eluted after 9.7 minutes. Regarding MA, the fragment ions correspond to the loss of a methyl group from the derivatization residue [M-15], to the loss of a *t*-butyl-dimethylsilyl group from the derivatization residue [M-57], and to the combined loss of a *t*-butyl-dimethylsilyl group from the derivatization residue and a CO group from one of the MA carboxylic groups [M-85]. Such a fragmentation pattern is typically observed for this type of derivates (Wittmann, C., 2007. Cell Fact. 6, 6.). The metabolite formed from o-cresol exhibits the same fragmentation pattern, whereby all fragment masses are shifted by a mass of 14. This identified the metabolite as methyl-muconate, likely 2-methyl muconate, given the position of the corresponding methyl group in the substrate.

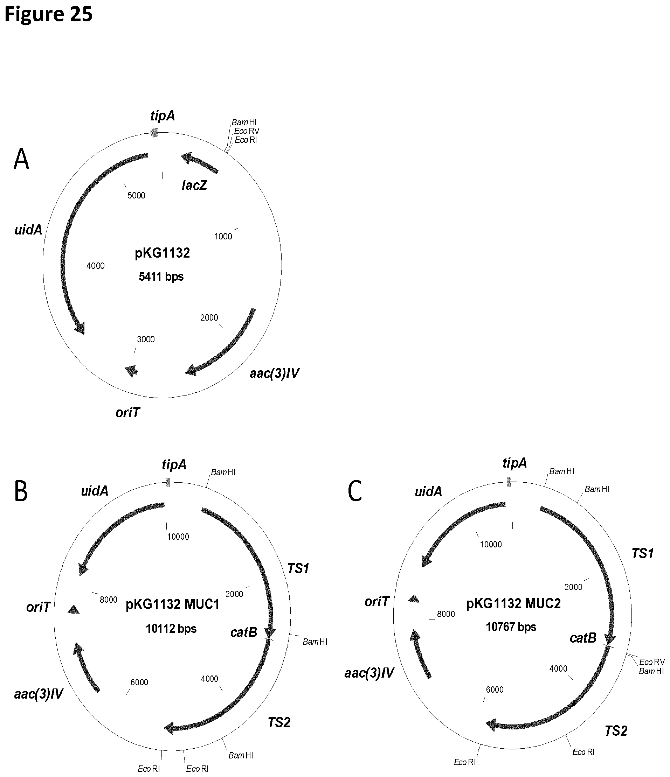

**Figure 25****:** Maps of the deletion vectors, generated and used in this work. The vectors comprise the backbone vector pKG1132 (A), the deletion vector pKG1132 MUC-1 for the deletion of AATC3_020100009302 (B), and the deletion vector pKG1132 MUC-2 for the deletion of AATC3_020100018510 (C). The β-glucuronidase gene (*uidA*) was cloned into the BamHI site of pKC1132, resulting in pKG1132. For gene deletion, 2.5 kbp upstream and downstream flanking regions were chosen as homology arms and cloned via Gibson assembly into the EcoRV site of pKG1132. Abbreviations: *aac(3)IV,* apramycin resistance gene; *tipA*, thiostrepton inducible promoter; *oriT*, origin of conjugal transfer; *uidA,* β-glucuronidase gene. The tipA promotor showed significant basal activity without induction, which was used for gene expression (Myronovskyi et al., 2011 Microbiol. 77, 5370-83.).

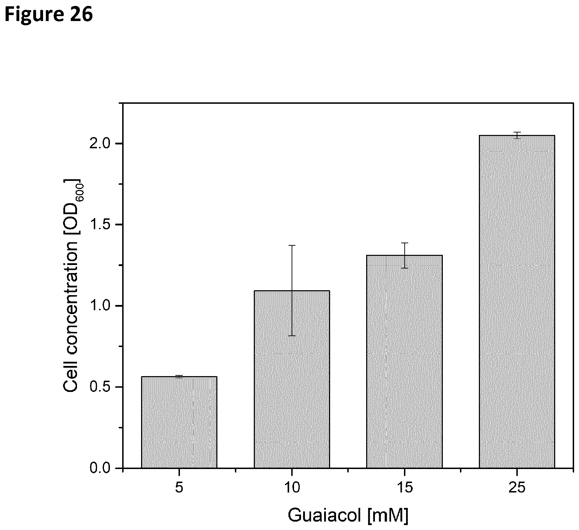

**Figure 26****:** Growth of *Amycolatopsis sp.* ATCC 39116 on different levels of guaiacol as sole source of carbon. The cell concentration was measured after 24 hours of incubation.

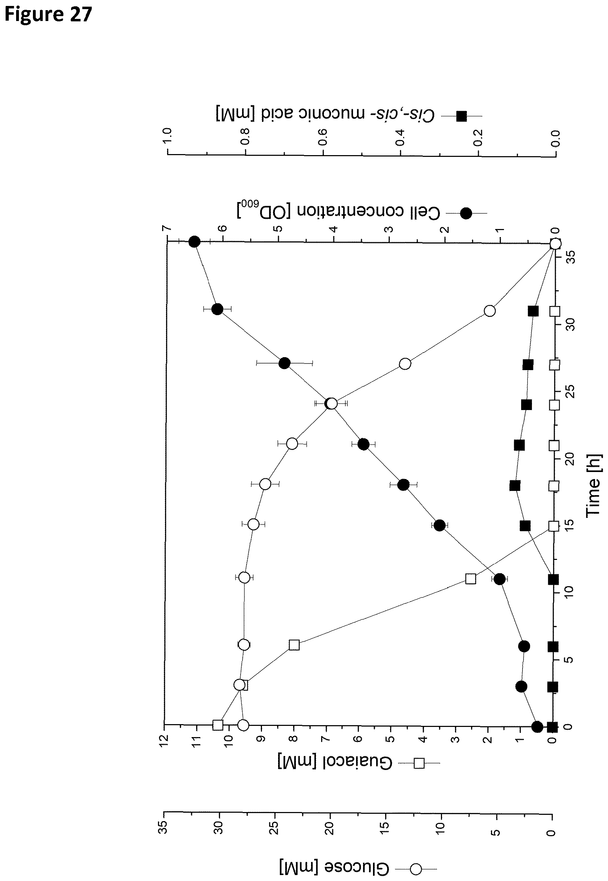

**Figure 27****:** Utilization of guaiacol by the engineered producer *Amycolatopsis* sp. ATCC 39116 MUC-1. The aromatic was added to a mineral salt medium together with glucose. The data reflect mean values and deviations from three replicates.

### Detailed description

The present invention refers to processes for using lignin, or optionally lignocellulose, as basis for various important biomolecules which are either intermediates in the preparation of further precursors for important biomolecules or which are commercially valuable biomolecules, e.g. polymers such as Nylon 6,6.

Lignin is subjected to fast pyrolysis according to a process according to the invention. More preferable, the amount of guaiacyl moieties in the lignin structure is at least 50% w/w, even more preferably at least 70% w/w, especially preferred at least 90% w/w.

In one aspect of the present invention, catechol is metabolically converted to *cis, cis-*muconic acid by the newly engineered *Pseudomonas putida* KT2440 BN6 strain which is also described in PCT/EP2016/070307 (published as WO 2017/037013 A1).

Other compounds for which *cis, cis*-muconic acid can be used as precursor are: Adipic acid (which again is a precursor of Nylon 6,6), terephthalic acid, hexamethylenediamine, caprolactam, caprolactone, 1,6-hexanediol, nylon-6,6 and nylon-6.

Thus, one aspect of the present invention refers to a method of producing a phenolic compound, preferably catechol, comprising the steps of
i) Subjecting lignin to a fast pyrolysis process;
ii) Condensing the resulting pyrolysis vapors by directly introducing the resulting pyrolysis vapors into at least one aqueous phase, resulting in at least one acquatic phase comprising a phenolic compound, preferably catechol, and at least one organic phase;
iii) Separating the resulting at least one acquatic phase comprising a phenolic compound, preferably catechol, and the resulting at least one organic phase.

Another aspect refers to a method of producing cis-cis muconic acid, comprising the steps of steps i) to iii) and further comprising the steps of
iiia) Optionally purifying catechol of the acquatic phase;
iv) Subjecting at least one acquatic phase comprising catechol of step iii) or purified catechol of step iiia) of said fast pyrolysis process to a biocatalytic conversion by contacting said acquatic phase with a biocatalyst, wherein said biocatalyst is a host cell which expresses an enzyme showing catechol-1,2-dioxygenase activity (EC 1.13.11.1) (catA) to prepare cis-cis muconic acid;
iva) Optionally purifying cis-cis muconic acid from the biocatalyst.

Another aspect refers to a method of producing adipic acid, comprising the steps i) to iii) according and further comprising the steps of
iiia) Optionally purifying catechol of the acquatic phase;
iv) Subjecting at least one acquatic phase comprising catechol of step iii) or purified catechol of step iiia) of said fast pyrolysis process to a biocatalytic conversion by contacting said acquatic phase with a biocatalyst, wherein said biocatalyst is a host cell which expresses an enzyme showing catechol-1,2-dioxygenase activity (EC 1.13.11.1) (catA) to prepare cis-cis muconic acid;
iva) Optionally purifying cis-cis muconic acid from the biocatalyst;
v) Preparing adipic acid by hydrogenation of cis-cis muconic acid.

Another aspect refers to a method of producing Nylon, comprising the steps i) to iii) and further comprising the steps of
iiia) Optionally purifying catechol of the acquatic phase;
iv) Subjecting at least one acquatic phase comprising catechol of step iii) or purified catechol of step iiia) of said fast pyrolysis process to a biocatalytic conversion by contacting said acquatic phase with a biocatalyst, wherein said biocatalyst is a host cell which expresses an enzyme showing catechol-1,2-dioxygenase activity (EC 1.13.11.1) (catA) to prepare cis-cis muconic acid;
iva) Optionally purifying cis-cis muconic acid from the biocatalyst;
v) Preparing adipic acid by hydrogenation of cis-cis muconic acid;
vi) Reacting hexamethylenediamine and adipic acid, preferably equivalent amounts of hexamethylenediamine and adipic acid, with water, preferably in a reactor;
vii) Crystallized the resulting product to produce nylon salt;
viii) Polymerize the nylon salt to nylon 6,6 in a reaction vessel, preferably either in batches or continuously.

*cis,* cis-muconic acid can also be used for the production of caprolactam. Good yield of around 55% can be obtained in the presence of Pd-Al₂O₃ (5 mol%) as catalyst, dissolved in dioxane together with ammonia (at, e.g. between 2 and 5 bar such as 3.4 bar) and hydrogen (at, e.g., between 20 and 50 bar, such as 34 bar) at between 200 °C and 350 °C such as 250 °C for 2 h (Beerthuis et al., 2015; Couldray et al., 2012). Thus, one embodiment also refers to a method of producing caprolactam comprising steps i) to iv) and optionally iiia) and/or iva) and further comprising the step of chemically converting *cis, cis*-muconic acid to caprolactam by using a catalyst, preferably an alumina-catalyst such as Pd-Al₂O₃ or Ru-Al₂O₃. Suitable solvents and reaction conditions are known to the skilled person, e.g. organic solvents such as dioxine, tetrahydrofuran and the like, pressures of between 2 and 50 bar and reaction temperatures between 200 °C and 350 °C.

*cis, cis*-muconic acid can also be converted in a reflux process with tetrahydrofuran and H₂O in the presence of I₂ as catalyst into *trans, trans*-muconic acid (WO 2010/148049) which can be converted to terephthalic acid. Via a thermal inverse electron demand Diels-Adler reaction, *trans, trans*-muconic acid and acetylene (e.g., 1 equivalent: 10 equivalents) can be converted to cyclohexa-2,5-diene-1,4-dicarboxylate (PI) (Burk et al., 2011). The formed PI can be easily oxygenated to terephthalic acid by the exposure to air. Thus, one embodiment also refers to a method of producing terephthalic acid comprising steps i) to iv) and optionally iiia) and/or iva) and further comprising the step of chemically converting *cis, cis*-muconic acid into *trans, trans*-muconic acid by using a catalyst, preferably I₂, and the step of converting *trans, trans*-muconic acid to terephthalic acid by oxygenating *trans, trans-*muconic acid, e.g., by exposure to air.

In one embodiment, the fast pyrolysis may include a combination of pre- treatment, feeding and pyrolysis conditions to convert lignin, or optionally lignocellulose, into valuable products. The pre-treatment involves mixing of the lignin, or optionally lignocellulose, with a low-cost clay additive that - in intimate contact with the lignin, or optionally the lignocellulose, - improves its feeding and pyrolysis behaviour, preferably in the presence of water. The mixing of the lignin, or optionally the lignocellulose, with the additive can be achieved by state of the art pelletising methods such as extrusion and subsequent drying of an aqueous slurry of the lignin, or optionally lignocellulose. Feeding of the (lignin- or optionally lignocellulose -additive) pellets into the reactor may be accomplished by using an adapted continuous screw feeder. A combination of feed-rate, reactor temperature and fluidisation gas velocity may ensure an efficient pyrolysis of the pelletised starting material in such a way that a maximum yield of valuable products is obtained.

In a specific embodiment, the fast pyrolysis process comprising:
a. pre-treating a mixture comprising lignin, or optionally lignocellulose, and a clay, wherein the pre- treatment comprises pelletising the mixture, to provide a pelletised starting material;
b. introducing the pelletised starting material obtained at a) to a reactor; and
c. pyrolysing the pelletised starting material in the reactor.

Preferably, the pyrolysing of the lignin, or optionally lignocellulose, preferably in form of pelletised starting material, in the reactor comprises pyrolysing the lignin, or optionally lignocellulose. Pyrolysis is the chemical decomposition of condensed substances by heating that occurs spontaneously at high enough temperatures without combustion. Pyrolysis is most commonly used for organic materials, being then one of the processes involved in charring. This chemical process is widely used in the chemical industry, for example, to produce charcoal, activated carbon, methanol and other chemicals from wood, to convert ethylene dichloride into vinyl chloride to make PVC, to produce coke from coal, to convert biomass into syn-gas, to turn waste into safely disposable substances, and for transforming medium-weight hydrocarbons from oil into lighter ones like gasoline. These specialised uses of pyrolysis may be called various names, such as dry distillation, destructive distillation, or cracking. Herein, pyrolysis especially refers to the process of heating materials without introduction of oxygen or air at a temperature in the range of about 300-700 °C, like 300-600 °C, such as 400-600 °C or even 450-550°C.

In another preferred embodiment, the pyrolysis is carried out in the absence of oxygen. More preferably in the absence of oxygen and halogen.

The content of lignin in the (dry) lignin, or optionally lignocellulose, is preferably more than 50 % w/w, more preferably more than 65 wt.%, most preferably more than 80 % w/w. For instance, in an embodiment the biomass comprises technical lignin (i.e. especially a material that contains typically > 90 % w/w lignin such as 95 % w/w or more or even 98 % w/w or more).

In a further embodiment, the starting material comprises lignin from the pulp and paper industry, e.g. in the form of black liquor derived lignin (e.g. from organosolv, soda, sulfite or Kraft pulping), or a lignin from the production of second generation bio fuels (like bio-ethanol). The processes of the invention are especially suitable for relatively pure lignins (> 80 wt.% lignin (dry base)) that are, e.g., prepared from biomass such as wood, straw, hulls and grass by techniques such as organosolv (a mixture of water and an appropriate organic solvent such as methanol, ethanol, organic acids, etc.), delignification and soda pulping. Examples are Alcell organosolv lignin (prepared from organosolv fractionation of a mixture of hardwoods), Granit soda pulping lignin (prepared from a mixture of wheat straw and Sarkanda grass) and organosolv wheat straw lignin (OWSL). The physical appearance of these lignins is a light (Granit) to dark brown (Alcell) powder (particle size < 0.1 mm) that easily melts at low temperatures (< 200°C). The lignins are virtually immiscible with water. In general, technical lignins from e.g. organosolv fractionation or from soda pulping are not easily miscible with water due to their hydrophobic character.

As described in WO 2011/159154, clay as additive provides a mixture that is relatively easily pelletisable and later thermolysable in a reactor, without substantial clogging of equipment. For instance, mixing the lignin or lignocellulose powder with some specific powdered (particle size < 0.1 mm) clays such as sepiolite, attapulgite and/or bentonite greatly enhances the miscibility with water and enables the preparation of an homogeneous aqueous lignin- or lignocellulose-clay slurry, e.g. using conventional homogenising equipment. The thickness of the slurry can be adjusted by the amount of water, preferably using a total amount of water of 30-70 % w/w of the total of lignin or optioanlly lignocellulose, clay, water and other components.

The lignin-, or optionally lignocellulose-, clay slurry can easily be pelletised by well-known techniques such as extrusion. The homogeneity of the lignin-, or optionally lignocellulose-, clay slurry may ensure an even distribution of clay and lignin, or optionally lignocellulose, in the resulting pellets or extrudates. To increase the mechanical strength of the pellets, a mild temperature treatment (preferably < 150°C in air or vacuum) can be applied to sinter the lignin-clay mixture. It is assumed that the clay acts as a sort of binder for the lignin. Other clays that possess a high level of porosity and water uptake capacity might be suitable as well. In addition from a binding effect, the clay may have a catalytic effect in the pyrolysis of lignin.

The clay is preferably a porous clay, especially a phyllo silicate clay, such as magnesium and/or aluminium phyllosilicates. Suitable clays include a hormite clay (such as a sepiolite or attapulgite clay), and a smectite clay (such as a montmorrilonite clay or another bentonite clay). Especially preferred are bentonite and/or sepiolite. In an embodiment, the clay comprises hydrotalcite clay. The term "clay" may in an embodiment refer to a combination of clays, such as a combination of bentonite and sepiolite. The clay may especially be a clay from the hormite group. Clays from the hormite group are, for example, attapulgite (Mgi.6Al0.6Si40io(OH).4H20 (= palygorskite), sepiolite (Mg₄Si₆0i5(OH)2.6H₂0), falcondoite, kaolinite, paramont- morillonite etc. Preferably, the clay used is sepiolite. The clays from the hormite group are known from the literature. Sepiolite and palygorskite are, for example, described by Galan (Clay Minerals (1996), 31, 443-453). Sepiolite is widely found in Spain.

Alternatively, the clay may also be from the smectite group. Bentonite is an absorbent aluminium phyllosilicate, generally impure clay from the smectite group, consisting mostly of montmorillonite (Na,Ca)₂(MgAl)₂Sᵢ₄0io.nH₂0). There are different types of bentonites and their names depend on the dominant elements, such as potassium (K), sodium (Na), calcium (Ca), and aluminium (Al). Bentonite usually forms from weathering of volcanic ash, most often in the presence of water. However, the term bentonite, as well as a similar clay called tonstein, have been used for clay beds of uncertain origin. For industrial purposes, two main classes of bentonite exist: sodium and calcium bentonite. Other smectites, such as saponite, beidellite, aliettite, hectorite, etc. (see www.mindata.org) can also be used.

In the pre-treatment, the lignin, or optionally lignocellulose, and the clay are mixed, in general together with water, to provide a paste or slurry, preferably a paste. The paste can be transformed to pellets following methods known in the art. Dependent upon the method chosen, the pre-treatment may include a heating before and/or after the pelletisation. Alternatively, the paste as such is subjected to thermolysis.

Lignin, or optionally lignocellulose, comprising biomass (typically containing 0 - 10 wt.% of moisture) and the clay (typically containing 0 - 10 wt.% of moisture) are preferably mixed in a weight ratio of 90: 10 to 10:90, preferably in a weight ratio of 80:20 to 20:80, based on the dry weight of the materials. Further, the amount of water of the mixture (before pelletisation and heating) is preferably in the range of about 30-70 % w/w, preferably in the range 40 - 60 % w/w relative to the total weight of the mixture of lignin, clay and water (and optional other components).

In a specific embodiment, the pre-treatment comprises (al) providing a paste of lignin, or optionally lignocellulose, clay and water (and optional other components), (a2) extruding the paste, (a3) providing particulate material, and (a4) drying the particulate material to provide the pelletised starting material. Instead of extruding using an extruder, the paste may also be pressed through a sieve with appropriate meshes. The pre-treatment advantageously provides a particulate material ("pelletised starting material"), that may be used for the pyrolysis. The pelletised starting material preferably comprises pellets having mean dimensions in the range of 0.25-10 mm, preferably 1-3 mm. Especially, at least 70 % w/w, more preferably at least 80 % w/w, even more preferably, at least 90 % w/w of the pelletised material consists of pellets having mean dimensions in the range of 0.25-10 mm or 1-3 mm, respectively. Optionally, too large and/or too small particles may be separated from the pelletised material, for instance by sieving or using a cyclone separator, or other methods known in the art.

The pelletised starting material may then be used in a thermal process, to provide bio-oil and a carbonaceous material, such as charcoal (herein also indicated as "bio char" since it originates from biological material). The thermal process is preferably applied in a fluid bed reactor e.g., (Dynamotive and RTI, USA), a circulating fluidized and transported bed reactor (e.g., Ensyn, USA; Fortum, FI), an ablative reactor (only at demo scale), a rotating cone reactor (e.g., BTG, NL), a vacuum reactor (e.g., Pyrovac, USA), and an auger reactor (e.g., KIT, DE and ROI, USA), especially preferred in a fluidised bed reactor.

Hence, the thermolysis is optionally performed in a fluidised bed reactor. The (pelletised) starting material is introduced in the fluidised bed reactor and fluidised. In this fluidised "state" the lignin, or optionally lignocellulose, is thermolysed (especially pyrolysed), resulting inter alia in pyrolysis vapors. The latter may not substantially be affected by the thermolysis, whereas the former may be transformed into char and bio-oils (and gasses).

The char particles can be retrieved from the reactor. The carbonaceous product (char) will float on the fluidised bed and can conveniently be collected by providing a collecting means just above the bed surface in the form of an overflow pipe or the like, connect to a receiving bin. The oil originating from the lignin pyrolysis products in the form of condensable organic vapours, water and aerosols that contain water and organics. This product mixture can be collected downstream the reactor by means of appropriate collecting equipment, including condensers for the condensable vapours and an electrostatic precipitator for the aerosols. Hence, disclosed is a process for the thermolysis of bio mass as described above, to provide bio char and bio oil from a lignin. In an embodiment, the process of the invention may be a continuous process (for the production of the pelletised end product and/or bio oil). Preferably, thermolysis is performed at a temperature in the range of 300-700 °C, especially in the range of 400-600 °C.

In one preferred embodiment, the pyrolysis is a continuous pyrolysis.

Preferably, the feed rates are in the range from 0,1 kg/h (kg/hour) to 100 kg/h.

In one preferred embodiment, the pyrolysis is carried out in a fluidized bed facility.

In one preferred embodiment, the granular material (bed inventory) for the fluidized bed reaction comprises silica sand (e.g., with a mean particle size from 0,01 mm to 0,7 mm such as from 0,01 mm to 0,5 mm, 0,025 mm to 0,3 mm .

The addition of salts to the reactor may shift the reaction equilibrium towards the intermediate compounds. Illustrative examples for useful salts that can be added to a (fast) pyrolysis reaction include alkali salts, e.g. Na₂SO₄, NaCl, KCI, CaCl₂, and CaSO₄.

Further, catalysts such as CaO, NaHCO₃, RbOH, CsOH, LiOH, Ca(OH)₂, CaCO₃, Na₂CO₃, K₂CO₃, KOH, Ni, ZrO₂, H₂SO₄, TiO₂, ZrO₂, Ru, Pt, Rh, Pd FeCl₃, and/or NiCl₂, NaOH, HCl can be added. Addition of hydrogen donor solvents such as tetralin, ethyl acetate, coal tar and reducing gas such as H₂, CO, and Ar can further be applied to increase the liquid reaction product.

In another embodiment, the pyrolysis is carried out under N₂-atmosphere, Ar-atmosphere, He-atmosphere or mixtures thereof.

In a preferred embodiment of the invention, the resulting pyrolysis vapors are introduced into an aqueous phase by bubbling, i.e. a line directly transports the vapors from the reactor into an aqueous phase.

Preferably, the temperature difference between the vapor resulting from the pyrolysis in the reactor and the temperature of the vapor being introduced into the water is less than 50 °C, more preferably less than 30 °C, more preferably less than 20 °C at the time point shortly (less than 1 sec) before the vapor is introduced into the aqueous phase.

In another preferred embodiment, the transport time of vapor between the pyrolysis, e.g., in a reactor, and the introduction into a (first) aqueous phase is less than 5 sec, more preferably less than 3 sec.

In yet another preferred embodiment, the water content in an aqueous phase is at least 90% w/w, more preferably at least 95% w/w, even more preferably at least 99% w/w especially preferred, the water content is at least 99,9 % w/w. Even more preferred, the aqueous phase comprises at least 99,9 % w/w water such as deionized water.

In yet another preferred embodiment, the water content in an aqueous phase is at least 90% w/w, more preferably at least 95% w/w and the additive is a buffer-system (i.e. buffer component(s) and, if necessary, amounts of base and/or acid to adjust a pH). In yet another embodiment, an aqueous phase consists of water or water and a buffer-system.

In yet another preferred embodiment, the pH of an aqueous phase is between 5 and 9, more preferably between 6 and 8.

In another preferred embodiment, the at least one aqueous phase has a temperature between 0 °C and 50 °C, preferably between 0 °C and 30 °C, more preferably between 0 °C and 10 °C.

In yet another embodiment, the at least one acquatic phase comprising catechol is in a temperature range between 0 °C and 80 °C, preferably between 0 °C and 50 °C.

Another preferred embodiment refers to a method according to the invention, wherein the amount of the aqueous phase(s) in which the pyrolysis vapors are introduced is at least 2 g, preferably at least 2.5 g, more preferably at least 4 g or even at least 5 g per g lignin to be pyrolysed.

Another preferred embodiment refers to a method according to the invention, wherein the amount of the aqueous phase in which the pyrolysis vapors are introduced is in a range from 2 to 8 g, more preferably from 2.5 to 6 g aqueous phase per g lignin to be pyrolysed .

In one preferred embodiment, the vapors of a fast pyrolysis are firstly introduced into a first aqueous phase and remaining vapors are introduced in a second aqueous phase resulting in two acquatic phases. In a further preferred embodiment, the two acquatic phases of step ii) are combined before step iii) or before step iv). In another preferred embodiment, only the first acquatic phase is used for step iii) or step iv).

In a preferred embodiment, the two aqueous phases are identical regarding the water content and additives.

Another preferred embodiment refers to a method according to the invention, wherein the biocatalyst is selected from the group consisting of bacteria, yeast, filamentous fungi, cyanobacteria, algae, and plant cells.

Another preferred embodiment refers to a method according to the invention, wherein the host cell is a bacterial host cell selected from the group consisting of *Bacillus* bacteria (e.g., *B*. *subtilis, B. megaterium*), *Acinetobacter* bacteria, *Norcardia* baceteria, *Xanthobacter* bacteria, *Escherichia* bacteria (e.g., *E. coli* (e.g., strains DH10B, Stbl2, DH5-alpha, DB3, DB3.1, DB4, DB5, JDP682 and ccdA-over (e.g., U.S. Application No. 09/518,188))), *Streptomyces* bacteria, *Erwinia* bacteria, *Klebsiella* bacteria, *Serratia* bacteria (e.g., S. *marcescens), Pseudomonas* bacteria (e.g., *P. aeruginosa, P. putida*), *Salmonella* bacteria (e.g., *S*. *typhimurium, S. typhi*), *Megasphaera* bacteria (e.g., *Megasphaera elsdenii*), photosynthetic bacteria (e.g., green non-sulfur bacteria (e.g., *Choroflexus* bacteria (e.g., *C*. *aurantiacus*), *Chloronema* bacteria (e.g., *C*. *gigateum*)), green sulfur bacteria (e.g., *Chlorobium* bacteria (e.g., *C*. *limicola)), Pelodictyon* bacteria (e.g., *P. luteolum*), purple sulfur bacteria (e.g., *Chromatium* bacteria (e.g., *C*. *okenii*)), and purple non-sulfur bacteria (e.g., *Rhodospirillum* bacteria (e.g., *R. rubrum)*), *Rhodobacter* bacteria (e.g., *R. sphaeroides, R. capsulatus*), and *Rhodomicrobium* bacteria (e.g., *R. vanellii*)), *Corynebacterium* bacteria (e.g. *Corynebacterium glutamicum* ATCC 13032), *Amycolatopsis* bacteria (e.g. *Amycolatopsis* sp. ATCC 39116). Corynebacterium glutamicum ATCC 13032 is also known as Corynebacterium glutamicum DSMZ 20300.

Another preferred embodiment refers to a method according to the invention, wherein the host cell is a yeast host cell selected from the group consisting of *Yarrowia* yeast (e.g., *Y. lipolytica* (formerly classified as Candida lipolytica)), *Candida* yeast (e.g., *C*. *revkaufi, C*. *pulcherrima, C*. *tropicalis, C*. *utilis*), *Rhodotorula* yeast (e.g., *R. glutinus, R. graminis*), *Rhodosporidium* yeast (e.g., *R. toruloides*), *Saccharomyces* yeast (e.g., *S*. *cerevisiae, S. bayanus, S. pastorianus, S. carlsbergensis*), *Cryptococcus* yeast, *Trichosporon* yeast (e.g., *T. pullans, T. cutaneum*), *Pichia* yeast (e.g., *P. pastoris*) and *Lipomyces* yeast (e.g., L. *starkeyii, L. lipoferus*).

Another preferred embodiment refers to a method according to the invention, wherein the host cell is a fungal host cell selected from the group consisting of *Aspergillus* fungi (e.g., *A. parasiticus, A. nidulans*), *Thraustochytrium fungi, Schizochytrium* fungi and *Rhizopus* fungi (e.g., R. arrhizus, R. oryzae, R. nigricans), e.g. an *A. parasiticus* strain such as strain ATCC24690, or an *A. nidulans* strain such as strain ATCC38163.

Another preferred embodiment refers to a method according to the invention, wherein the host cell is a non-genetically modified host cell.

Another preferred embodiment refers to a method according to the invention, wherein the host cell is a recombinant host cell comprising at least one heterologous gene.

Another preferred embodiment refers to a method according to the invention, wherein said at least one heterologous gene is stably integrated into the host cell's genome.

Another preferred embodiment refers to a method according to the invention, wherein said host cell comprises at least one (optionally heterologous) gene encoding a polypeptide having catechol 1,2-dioxygenase activity.

Another preferred embodiment refers to a method according to the invention, wherein said host cell comprises at least one (optionally heterologous) *catA* gene.

Another preferred embodiment refers to a method according to the invention, wherein said at least one (optionally heterologous) *catA* gene encodes a polypeptide comprising a sequence corresponding to SEQ ID No. 1, 108 or 122.

Another preferred embodiment refers to a method according to the invention, wherein said at least one (optionally heterologous) *catA* gene comprises a sequence corresponding to SEQ ID No. 2.

Another preferred embodiment refers to a method according to the invention, wherein said host cell comprises, operably linked to, e.g. upstream of, the at least one (optionally heterologous) gene, a promoter sequence corresponding to
i) SEQ ID No. 5 [Pem7]; or
ii) SEQ ID No. 6 [Pem7*]; or
iii) SEQ ID No. 7 [Ptuf]; or
iv) SEQ ID No. 8 [PrpoD]; or
v) SEQ ID No. 9 [Plac]; or
vi) SEQ ID No. 10 [PgyrB];
vii) SEQ ID No. 11; or
viii) SEQ ID No. 12; or
ix) SEQ ID No. 13; or
x) SEQ ID No. 14; or
xi) SEQ ID No. 15; or
xii) SEQ ID No. 16; or
xiii) SEQ ID No. 88 [Ptuf_1]; or
xiv) SEQ ID No. 89 [Ptuf_short]; or
xv) SEQ ID No. 90 [Ptuf_s_2]; or
xvi) SEQ ID No. 91 [Ptuf_s_3]; or
xvii) SEQ ID No. 92 [Ptuf_s_4]; or
xviii) SEQ ID No. 93 [Ptuf_s_5]; or
xix) SEQ ID No. 94 [Ptuf_s_6]; or
xx) SEQ ID No. 95 [Ptuf_s_7]; or
xxi) SEQ ID No. 96 [Ptuf_s_8]; or
xxii) SEQ ID No. 97 [Ptuf_s_9]; or
xxiii) SEQ ID No. 98 [Ptuf_s_10]; or
xxiv) SEQ ID No. 99 [Ptuf_s_11]; or
xxv) SEQ ID No. 100 [Ptuf_s_12]; or
xxvi) SEQ ID No. 101 [Pgro]; or
xxvii) SEQ ID No. 102 [Pgro_1]; or
xxviii) SEQ ID No. 103 [Pgro_2]; or
xxix) SEQ ID No. 104 [Pgro_4]; or
xxx) SEQ ID No. 105 [Pgro_5];
preferably SEQ ID No. 5 [Pem7].

Another preferred embodiment refers to a method according to the invention, wherein the at least one (optionally heterologous) gene is constitutively expressed.

Another preferred embodiment refers to a method according to the invention, wherein said host cell is further characterized in that it does not express a functional catB polypeptide, and/or in that it does not express a functional catC polypeptide, and/or in that it does not express a functional pcaB polypeptide. A preferred catB polypeptide is shown in SEQ ID No. 27, 109, 123, 124 or 125.

Another preferred embodiment refers to a method according to the invention, wherein said host cell is characterized by expressing a functional catA polypeptide and by not expressing a functional catB polypeptide.

A preferred embodiment refers to a method according to the invention, wherein said host cell expresses a functional catA polypeptide, said catA polypeptide being characterized in that it has (a) the amino acid sequence shown in SEQ ID No. 108; or (b) an amino acid sequence which has at least 40% identity to the amino acid sequence shown in SEQ ID No. 108 and having catechol-1,2-dioxygenase activity, e.g. SEQ ID No. 122; and does not express a functional catB polypeptide, said catB polypeptide being characterized in that it has (c) the amino acid sequence shown in SEQ ID No. 109; or (d) an amino acid sequence which has at least 25% identity to the amino acid sequence shown in SEQ ID No. 109 and having muconate cycloisomerase activity, e.g. SEQ ID No. 123, 124, or 125. Such a host cell is preferably a bacterial host cell, in particular *Corynebacterium glutamicum* ATCC13032 or *Amycolatopsis* sp. ATCC39116.

Another preferred embodiment refers to a method according to the invention, wherein the *cat8* gene, *catC* gene or *pcaB* gene is silenced, preferably knocked-down or knockedout, or deleted from the chromosome.

Another preferred embodiment refers to a method according to the invention, wherein said host cell is further characterized in that it does not express a functional catB polypeptide, and it does not express a functional catC polypeptide.

Another preferred embodiment refers to a method according to the invention, wherein said at least one heterologous gene is derived from *Pseudomonas, preferably Pseudomonas putida, more preferably Pseudomonas putida strain KT2440 BN6.*

Another preferred embodiment refers to a method according to the invention, wherein said host cell is selected from *Pseudomonas,* preferably *Pseudomonas putida,* more preferably *Pseudomonas putida* strain KT2440, strain KT2440 JD2S or strain KT2440 BN6, even more preferably *Pseudomonas putida* strain KT2440 BN6.

Another preferred embodiment refers to a method according to the invention, wherein said host cell is selected from *Corynebacterium,* preferably *Corynebacterium glutamicum,* more preferably *Corynebacterium glutamicum* ATCC 13032. The present inventors surprisingly found that *Corynebacterium,* preferably *Corynebacterium glutamicum,* more preferably *Corynebacterium glutamicum* ATCC 13032 is robust in the presence of phenolic compounds, such as catechol and produces high amounts of *cis, cis*-muconic acid as is shown in the Examples. Phenolic compounds, such as catechol are an outcome of the processes of the present invention. However, while other bacterial cells show diminished growth and metabolism in the presence of high catechol concentrations, in particular *Corynebacterium glutamicum* ATCC 13032 does not as shown in the Examples; see Example 2. By modifying *Corynebacterium,* preferably *Corynebacterium glutamicum,* more preferably *Corynebacterium glutamicum* ATCC 13032 so as to inactivate expression of a functional catB gene encoding a protein having muconate cycloisomerase activity, e.g. by disrupting the endogenous catB gene, the present inventors could improve the production of *cis,* cis-muconic acid. However, the present inventors could even further improve the production of *cis, cis*-muconic acid by decoupling the biosynthetic pathway to *cis, cis-*muconic acid production from its native regulation control by using a heterologous promoter for the expression of the catA gene encoding a protein having catechol-1,2-dioxygenase activity.

Thus, a preferred *Corynebacterium,* preferably *Corynebacterium glutamicum,* more preferably *Corynebacterium glutamicum* ATCC 13032 has its endogenous catA gene operably linked to a heterologous promoter and does not express a functional catB polypeptide. It is preferred that the heterologous promoter is a strong promoter which overexpresses the endogenous catA gene in comparison to the endogenous promoter of the catA gene. However, in the alternative, the endogenous catA gene may also be operably linked to its endogenous promoter.

Preferably, the catA gene of *Corynebacterium,* preferably *Corynebacterium glutamicum,* more preferably *Corynebacterium glutamicum* ATCC 13032 encodes a polypeptide having an amino acid sequence which has at least 70% identity to the amino acid sequence shown in SEQ ID No. 108 and has catechol-1,2-dioxygenase activity. More preferably, the catA gene encodes a polypeptide having the amino acid sequence shown in SEQ ID No. 108.

Preferably, the catB polypeptide of *Corynebacterium,* preferably *Corynebacterium glutamicum,* more preferably *Corynebacterium glutamicum* ATCC 13032 has an amino acid sequence which has at least 70% identity to the amino acid sequence shown in SEQ ID No. 109 and has muconate cycloisomerase activity. More preferably, the catB polypeptide has the amino acid sequence shown in SEQ ID No. 109.

Another preferred embodiment refers to a method according to the invention, wherein said host cell is selected from *Amycolatopsis,* preferably *Amycolatopsis* sp., more preferably *Amycolatopsis* sp. ATCC 39116 (Davis et al., 2012). The present inventors found that *Amycolatopsis,* preferably *Amycolatopsis* sp., more preferably *Amycolatopsis* sp. ATCC 39116 is robust in the presence of phenolic compounds, such as catechol or guaiacol and produces high amounts of *cis,* cis-muconic acid from guaiacol as is shown in the Examples. Phenolic compounds, such as catechol or guaiacol are an outcome of the processes of the present invention. When lignin is processed by way of the processes of the present invention, phenolic compounds are obtained. Dependent on the temperature applied in the pyrolysis process of the methods of the present invention, the amount of different phenolic compounds may vary. For example, if for the pyrolysis a temperature of lower than 400°C is used, such as 370°C, 350°C or 330°C, the amount of guaiacol among phenolic compounds is increased, e.g., it is up to 45%, if a temperature lower than 400°C is used, e.g. 330°C. A lower temperature requires less energy input which makes pyrolysis cheaper and/or more sustainable. Moreover, it is known that lignin from softwood has a large amount of guaiacol (up to 80% of the aromatic compounds of lignin) (Beckham et al. 2016) and thus, *Amycolatopsis,* preferably *Amycolatopsis* sp., more preferably *Amycolatopsis* sp. ATCC 39116 may be an ideal bacterial host cell for converting phenolic compounds, in particular guaiacol, obtainable from the processes of the present invention if, for example, lignin from softwood is used for the production of phenolic compounds.

However, while other bacterial cells show diminished growth and metabolism in the presence of high guaiacol concentrations, or do not use guaiacol, in particular *Amycolatopsis* sp. ATCC 39116 does not as shown in the Examples; see Example 3. By modifying *Amycolatopsis,* preferably *Amycolatopsis* sp., more preferably *Amycolatopsis* sp. ATCC 39116 so as to inactivate expression of a functional catB gene encoding a protein having muconate cycloisomerase activity, e.g. by disrupting the endogenous catB gene, the present inventors could improve the production of *cis, cis*-muconic acid from phenolic compounds, including in particular guaiacol. However, also other phenolic compounds such as phenol or cresol are used.

Much to their surprise, the present inventors identified at least three catB genes in *Amycolatopsis* sp. ATCC 39116. The amino acid sequence of them is shown in SEQ ID No. 123, 124 or 125, respectively. Accordingly, each of these catB genes may be individually inactivated, combinations of two of them (catB genes) may be inactivated or all three (catB genes) may be inactivated. The present inventors surprisingly observed that the catB gene encoding the amino acid sequence shown in SEQ ID No. 125, when it is inactivated, results in an *Amycolatopsis* host cell which does not further metabolize *cis, cis*-muconic acid after guaiacol is consumed. Accordingly, it is preferred that the catB gene encoding the amino acid sequence shown in SEQ ID No. 125 is disrupted in an *Amycolatopsis* host cell, e.g. when it is desired that an *Amycolatopsis,* preferably *Amycolatopsis* sp., more preferably *Amycolatopsis* sp. ATCC 39116 host cell should not further metabolize *cis, cis*-muconic acid after guaiacol is consumed.

By modifying *Amycolatopsis,* preferably *Amycolatopsis* sp., more preferably *Amycolatopsis* sp. ATCC 39116 so as to inactivate expression of a functional catB gene encoding a protein having muconate cycloisomerase activity, e.g. by disrupting the endogenous catB gene, the present inventors could improve the production of *cis, cis-*muconic acid, in particular *cis, cis*-muconic acid production on the basis of guaiacol.

Thus, a preferred *Amycolatopsis,* preferably *Amycolatopsis* sp., more preferably *Amycolatopsis* sp. ATCC 39116 expresses its endogenous catA gene and does not express a functional catB polypeptide.

It is also envisioned that another preferred *Amycolatopsis,* preferably *Amycolatopsis* sp., more preferably *Amycolatopsis* sp. ATCC 39116 has its endogenous catA gene operably linked to a heterologous promoter and does not express a functional catB polypeptide. It is preferred that the heterologous promoter is a strong promoter which overexpresses the endogenous catA gene in comparison to the endogenous promoter of the catA gene.

Preferably, the catA gene of *Amycolatopsis,* preferably *Amycolatopsis* sp., more preferably *Amycolatopsis* sp. ATCC 39116 encodes a polypeptide having an amino acid sequence which has at least 70% identity to the amino acid sequence shown in SEQ ID No. 122 and having catechol-1,2-dioxygenase activity. More preferably, the catA gene encodes a polypeptide has the amino acid sequence shown in SEQ ID No. 122

Preferably, the catB polypeptide of *Amycolatopsis,* preferably *Amycolatopsis* sp., more preferably *Amycolatopsis* sp. ATCC 39116 has an amino acid sequence which has at least 70% identity to the amino acid sequence has shown in SEQ ID No. 123, 124 or 125 and has muconate cycloisomerase activity. More preferably, the catB polypeptide has the amino acid sequence shown in SEQ ID No. 123, 124 or 125.

It is preferred that *Amycolatopsis,* preferably *Amycolatopsis* sp., more preferably *Amycolatopsis* sp. ATCC 39116 does not express a functional catB polypeptide having the amino acid sequence shown in SEQ ID No. 123 and a functional catB polypeptide having the amino acid sequence shown in SEQ ID No. 124.

It is preferred that *Amycolatopsis,* preferably *Amycolatopsis* sp., more preferably *Amycolatopsis* sp. ATCC 39116 does not express a functional catB polypeptide having the amino acid sequence shown in SEQ ID No. 123 and a functional catB polypeptide having the amino acid sequence shown in SEQ ID No. 125.

It is preferred that *Amycolatopsis,* preferably *Amycolatopsis* sp., more preferably *Amycolatopsis* sp. ATCC 39116 does not express a functional catB polypeptide having the amino acid sequence shown in SEQ ID No. 124 and a functional catB polypeptide having the amino acid sequence shown in SEQ ID No. 125. Interestingly, such a cell, in addition to muconate also produces methyl-muconate from o-cresol and/or m-cresol. It would have been expected that o-cresol and/or m-cresol are converted into catechol, but the production of methyl-catechol was observed which is then converted into methyl-muconate.

It is preferred that *Amycolatopsis,* preferably *Amycolatopsis* sp., more preferably *Amycolatopsis* sp. ATCC 39116 does not express a functional catB polypeptide having the amino acid sequence shown in SEQ ID No. 123, a functional catB polypeptide having the amino acid sequence shown in SEQ ID No. 124, and a a functional catB polypeptide having the amino acid sequence shown in SEQ ID No. 125.

Another preferred embodiment refers to a method according to the invention, wherein, the cis-cis-muconic acid derived after step iv) or iva) is white in color.

Another preferred embodiment refers to a method according to the invention, wherein the yield in cis-cis-muconic acid (in mol) from catechol is greater than 80%, or greater than 85% based on the amount in mol of catechol.

### (Fast) pyrolysis reaction

The skilled artisan will readily understand that the exact reaction conditions of the fast pyrolysis will vary depending on the organic educt, the size and properties of the reaction container, and the desired nature and yield of intermediate product that is to be obtained. Reaction conditions can be adjusted, e.g. by the addition of salts, solvents (e.g. methanol, phenol or p-cresol), different concentrations of organic educt (e.g. lignin), and various retention times.

The present invention is considered to be particular advantageous for converting lignin into cis-cis-muconic acid, adipic acid or nylon 6,6 via catechol. This process is also termed "lignin processing" hereinafter. A preferred protocol for the first step in lignin processing, i.e. lignin conversion using (fast) pyrolysis, is described in the following. The present inventors have discovered that in order to convert the organic educt lignin into the intermediate product catechol, reaction temperatures 300°C and above, such as about 400°C, about 450°C, about 500°C, about 550°C, about 600°C may be favorable. Reaction temperatures of between about 450°C and about 550°C may be particularly preferred. The skilled person will readily be able to adjust the retention time in order to obtain a desired amount of catechol, e.g. depending on the size of the reactor. A steady process is also conceivable. Exemplary retention times applied in the methods of the invention may in general be between 1 and 30 sec, such as between 2 and 20 sec.

### Lignin educt

One of the most important benefits of the means and methods of the invention is that they can be applied to a great variety of organic educts as long as they comprise lignin. Biomass and its constituents are particularly envisaged for use as feedstock in the methods of the invention and generally include biological material derived from living, or recently living organisms, such as waste from wood processing industry (e.g. sawdust, cut-offs, bark, etc), waste from paper and pulp industry, agricultural waste (straw etc.), urban wood waste (wooden pallets, packing material, etc.). While it is in general possible and envisaged herein to subject any of the aforementioned biomass resources to the inventive method as described herein, the use constituents isolated from biomass may be advantageous when production of a certain intermediate product with few by-products is desired. Biomass constituents envisaged for use according to the methods of the invention include, without limitation lignin, lignocellulose and other lignin comprising combinations. However, samples with a lignin content of at least 50% w/w, more preferably at least 80% w/w, even more preferably at least 90% w/w such as at least 95% w/w or even at least 99% w/w are preferred.

### Lignin

It is particularly envisaged herein to provide means and methods for further processing of lignin. Lignin is a cross-linked amorphous copolymer synthesized from random polymerization of aromatic monomers, in particular the three primary phenylpropane monomers p-coumaryl alcohols, coniferyl alcohols, and sinapyl alcohols containing zero, one, and two methoxyl groups, respectively.

The relative amount of desired intermediate product in the reaction product may vary depending on the organic educt and the reaction conditions.

E.g., for various lignin compounds, catechol is envisaged to be present in an acquatic phase resulting from the fast pyrolysis reaction according to the invention in an amount of 0.5% w/w or more, such as 1% w/w or more, 1.5% w/w or more, 2.0% w/w or more, for example up to 10.0% w/w compared to the amount of lignin, which was processed via (fast) pyrolysis. Further components may be phenol, cresol and/or guaiacol. The term "cresol" as used herein generally comprises m-cresol, p-cresol and o-cresol and can, in one embodiment, be present in an acquatic phase resulting from the fast pyrolysis reaction according to the invention in an amount of 0.025% w/w or more, such as 0,05% w/w or more, 0,1% w/w or more, 0,2% w/w or more, for example up to 0,5% w/w compared to the amount of lignin, which was processed via (fast) pyrolysis . In another embodiment, phenol is envisaged to be present in an acquatic phase resulting from the fast pyrolysis reaction according to the invention in an amount of 0.02% w/w or more, such as 0,05% w/w or more, 0,1% w/w or more, 0,2% w/w or more, for example up to 0,5% w/w compared to the amount of lignin, which was processed via (fast) pyrolysis. In yet another embodiment,guaiacol is envisaged to be present in an acquatic phase resulting from the fast pyrolysis reaction according to the invention in an amount of 0.01% w/w or more, such as 0,015% w/w or more, 0,1% w/w or more, 0,2% w/w or more, for example up to 0,5% w/w.

It is envisaged that the step of purifying an intermediate product in an acquatic phase from step (iii) of the method of the invention may involve separating said intermediate product from the acquatic phase or other components in said acquatic phase. Separation of said intermediate product includes complete separation (i.e. purification), and partial separation of said product. "Complete separation" means that a product is yielded in essentially pure form (i.e. without the presence of other by-products or solvents). "Partial separation" means that other by-products or solvents are present.

The step of iiia) purifying an intermediate product may involve a variety of process steps depending on the characteristics of the intermediate product to be recovered, the presence and nature of potential by-products and the desired purity of the intermediate product. E.g., obtaining the intermediate product, preferably catechol, may involve distillation of the reaction product obtained after sub- and/or supercritical fluid-assisted conversion. As it is well-known in the art, distillation is a process of separating components from a liquid mixture by selective evaporation and condensation. Distillation includes e.g. simple distillation, fractional distillation, steam distillation (also referred to as "steam bath distillation" herein).

Steam bath distillation may be accomplished as set out in the appended examples. Other methods for separating the intermediate product and/or by-product and/or salts from other components of the reaction (e.g. catalyst, solvent, and/or remaining educt) are also conceivable, and include, e.g., filtration (such as vacuum filtration, for instance using PTFE membranes), affinity chromatography, ion exchange chromatography, solvent extraction, filtration, centrifugation, electrophoresis, hydrophobic interaction chromatography, gel filtration chromatography, chromatofocusing, differential solubilization, preparative disc-gel electrophoresis, isoelectric focusing, HPLC, reverse-phase HPLC, and countercurrent distribution.

Intermediate products obtained in step (iii) or (iiia) of the inventive method may vary depending on the organic educt and reaction parameters.

Notably, as mentioned previously many of the intermediate products exemplified herein also themselves constitute potential organic educts susceptible to further conversion or re-polymerization. As set out elsewhere herein, reaction parameters such as reaction temperature, pressure and reaction time, can be readily adjusted in order to shift the reaction towards favorable intermediate products.

The present invention also refers to a mean to produce catechol from lignin, or optionally lignocellulose, comprising a pyrolysis reactor, at least one line leading pyrolysis vapors into a water reservoir, preferably the container comprising the water reservoir is cooled. Optionally, this means comprises a second line leading from the first container comprising a water reservoir into a second water reservoir, preferably the second container comprising the second water reservoir is cooled. The second line is attached to the first container so that the exit through the line is above water level of the water reservoir in said container.

### Biocatalyst

The intermediate product obtained in step iii) or iiia) of the inventive method is subjected to biocatalytic conversion, i.e. contacted with a biocatalyst, in particular a host cell that produces the desired organic product. Contacting the intermediate product will, as will be well understood by the person skilled in the art, be conducted under conditions that allow the biocatalyst to catalyze production of the desired organic product from the intermediate product. The exact conditions, including concentration of the intermediate product, concentration and growth state of the biocatalyst, culture conditions including culture medium composition, pH, temperature, aeration, agitation and container, will depend greatly on the biocatalyst, the intermediate product and the organic product to be obtained and will be readily ascertainable by the skilled person in the art.

A preferred host cell includes any suitable host cell that is capable of producing the desired organic product from the intermediate product it is supplied with. The skilled person will readily acknowledge that feasibility of using a given host cell as a biocatalyst in the methods of the invention primarily depends on whether the host cell comprises the genetic constitution required to catalyze production of the desired end product. E.g., the host cell preferably expresses enzymes capable of converting the intermediate product (e.g., catechol) obtained in step (ii) of the invention into the desired organic end product (e.g., cis-cis-muconic acid). Polypeptides required for production of the desired organic end product (which may include, e.g., enzymes catalyzing the conversion and proteins required for import and/or export of the reactants into or out of the cell, respectively), will also be referred to as "polypeptides of interest" or "POI" herein. Genes encoding said polypeptides of interest are also termed "genes of interest" or "GOI" herein.

The host cell may be a prokaryotic or eukaryotic host cell and may be selected from bacteria, yeast, filamentous fungi, cyanobacteria, algae, and plant cells.

The host cell is envisaged to be a single cell organism, which is typically capable of dividing and proliferating. A host cell can include one or more of the following features: aerobe, anaerobe, filamentous, non-filamentous, monoploid, dipoid, auxotrophic and/or nonauxotrophic.

Suitable prokaryotic host cells include Gram negative or Gram positive bacteria and may be selected from, e.g., Bacillus bacteria (e.g., B. subtilis, B. megaterium), Acinetobacter bacteria, Norcardia baceteria, Xanthobacter bacteria, Escherichia bacteria (e.g., E. coli (e.g., strains DH10B, Stbl2, DH5-alpha, DB3, DB3.1 ), DB4, DB5, JDP682 and ccdA-over (e.g., U.S. Application No. 09/518,188), Streptomyces bacteria, Erwinia bacteria, Klebsiella bacteria, Serratia bacteria (e.g., S. marcescens), Pseudomonas bacteria (e.g., P. aeruginosa, P. putida), Salmonella bacteria (e.g., S. typhimurium, S. typhi), Megasphaera bacteria (e.g., Megasphaera elsdenii).

Bacteria also include, but are not limited to, photosynthetic bacteria (e.g., green non-sulfur bacteria (e.g., Choroflexus bacteria (e.g., C. aurantiacus), Chloronema bacteria (e.g., C. gigateum)), green sulfur bacteria (e.g., Chlorobium bacteria (e.g., C. limicola), Pelodictyon bacteria (e.g., P. luteolum), purple sulfur bacteria (e.g., Chromatium bacteria (e.g., C. okenii))), and purple non-sulfur bacteria (e.g., Rhodospirillum bacteria (e.g., R. rubrum), Rhodobacter bacteria (e.g., R. sphaeroides, R. capsulatus), and Rhodomicrobium bacteria (e.g., R. vanellii)).

Any suitable yeast may be selected as a host cell, including without limitation Yarrowia yeast (e.g., Y. lipolytica (formerly classified as Candida lipolytica)), Candida yeast (e.g., C. revkaufi, C. pulcherrima, C. tropicalis, C. utilis), Rhodotorula yeast (e.g., R. glutinus, R. graminis), Rhodosporidium yeast (e.g., R. toruloides), Saccharomyces yeast (e.g., S. cerevisiae, S. bayanus, S. pastorianus, S. carlsbergensis), Cryptococcus yeast, Trichosporon yeast (e.g., T. pullans, T. cutaneum), Pichia yeast (e.g., P. pastoris), Kluyveromyces yeast (e.g. K. marxianus), and Lipomyces yeast (e.g., L. starkeyii, L. lipoferus).

Any suitable fungus may be selected as a host cell, including without limitation Aspergillus fungi (e.g., A. parasiticus, A. nidulans), Thraustochytrium fungi, Schizochytrium fungi and Rhizopus fungi (e.g., R. arrhizus, R. oryzae, R. nigricans). The fungus may for example be an A. parasiticus strain such as strain ATCC24690, or an A. nidulans strain such as strain ATCC38163.

Eukaryotic host cells from non-microbial organisms can also be utilized as host cells in accordance with the present invention. Examples of such cells, include, without limitation, insect cells (e.g., Drosophila (e.g., D. melanogaster), Spodoptera (e.g., S. frugiperda Sf9 or Sf21 cells) and Trichoplusa (e.g., High-Five cells); nematode cells (e.g., C. elegans cells); avian cells; amphibian cells (e.g., Xenopus laevis cells); reptilian cells; and mammalian cells (e.g., NIH3T3, 293, CHO, COS, VERO, C127, BHK, Per-C6, Bowes melanoma and HeLa cells).

The aforementioned host cells are commercially available, for example, from Invitrogen Corporation, (Carlsbad, CA), American Type Culture Collection (Manassas, Virginia), and Agricultural Research Culture Collection (NRRL; Peoria, Illinois).

Suitable host cells are selected for their capability of converting the provided substrate (i.e. intermediate product) into the desired organic end product. Therefore, the host cell must be capable of channeling the substrate in and preferably of channeling the product out of the cell. In addition, the host cell should be tolerant to both the substrate and especially the accumulated product. Advantageously, the host cell can cope with the pH and temperature changes occurring during cultivation in the presence of the substrate. Thus, the host cell preferably provides for a high reaction rate, high yield and purity of the end product.

Depending on the organic product obtained from the host cell, it may be beneficial to use a host cell which is non-pathogenic, in particular non-pathogenic for humans, for example when the organic end product obtained from said host cell is intended for further processing in the pharmaceutical, cosmetic or food industry.

It is equally conceivable that the host cell is a genetically modified (i.e. recombinant) or a non-genetically modified host cell.

### Non-genetically modified host cells

Non-genetically modified host cells (also referred to as non-genetically modified organism or non-GMO herein) are host cells whose genetic material has not been altered using recombinant DNA technology techniques in contrast to genetically modified host cells (also termed "GMO" herein). The term "non-GMO" includes both wild-type host cells and host cells comprising mutations.

The use and creation of GMOs is governed by varying national regulations and guidelines. In particular when producing food products, use of non-GMOs is typically preferable.

Said non-GMO is preferably capable of catalyzing the conversion of the intermediate product obtained from step (ii) of the inventive method to the desired organic end product. That is, said non-GMO preferably comprises endogenous genes encoding for the polypeptide(s) of interest required for biocatalytic conversion of the intermediate products into the desired organic end products according to the methods of the invention.

As regards "lignin processing" as described herein, host cells comprising endogenous genes encoding for polypeptides having catechol-1,2-dioxygenase activity and are thus conceivable for use as non-genetically modified host cells include without limitation Acinetobacter sp. (e.g. A. calcoaceticus PHEA-2, A. gyllenbergii NIPH 230, A. junii CIP 64.5, A. Iwoffii NCTC 5866 = CIP 64.10, A. oleivorans DR1, A. radioresistens DSM 6976 = NBRC 102413 = CIP 103788, A. schindleri CIP 107287, A. schindleri CIP 107287), Amycolatopsis mediterranei U32, Amycolatopsis sp. ATCC39116, Arthrobacter sp., Aspergillus sp. (e.g. A. niger CBS 513.88, A. oryzae RIB40), Bordetella holmesii ATCC 51541, Bradyrhizobium sp. (e.g. B. diazoefficiens USDA 110, B. genosp. SA-4 str. CB756), Burkholderia sp. (e.g. B. cenocepacia J2315, B. glumae BGR1, B. mallei ATCC 23344, B. multivorans, B. pseudomallei K96243, B. xenovorans LB400), Candida dubliniensis CD36, Corynebacterium glutamicum ATCC 13032, Cupriavidus metallidurans CH34, Delftia acidovorans SPH-1, Enterobacter aerogenes KCTC 2190, Herbaspirillum seropedicae SmR1, Klebsiella pneumoniae subsp. pneumoniae HS11286, Mycobacterium smegmatis str. MC2 155, Neorhizobium galegae bv. orientalis str. HAMBI 540, Neurospora crassa OR74A, Pseudomonas sp. (e.g. P. aeruginosa PAO1, P. fluorescens SBW25, P. fragi B25, P. putida KT2440, P. stutzeri A1501), Ralstonia sp. (e.g. R. eutropha H16, R. pickettii 12J), Rhizobium sp. (e.g. R. etli CFN 42, R. leguminosarum bv. trifolii CB782), Rhodococcus sp. (e.g. R. erythropolis PR4), R. fascians NBRC 12155 = LMG 3623, R. jostii RHA1), Sinorhizobium sp. (e.g. S. fredii NGR234, S. meliloti 1021, S. wenxiniae), Sphingomonas sp. KA1, Thermus thermophilus HB8, Verticillium albo-atrum VaMs.102.

As set out elsewhere herein, host cells will typically be selected for ease of handling, tolerance to culture conditions, and (high) substrate concentrations, insensitivity towards accumulated end product and capability of producing the end product at high reaction rates in high yields and purity. Particularly envisaged as non-GMO host cells for use in lignin processing as described herein are host cells selected from *Pseudomonas,* preferably *P*. *putida,* and more preferably from *P. putida* strain KT2440.

### Recombinant host cell

Recombinant host cells (also referred to as genetically modified organisms or GMOs) are also envisaged for use in accordance with the methods of the invention. Recombinant host cells are host cells whose genetic material has been altered using recombinant DNA technologies. It is in particular envisaged that recombinant host cells comprise at least one heterologous nucleic acid sequence.

The heterologous nucleic acid sequence may e.g. be a heterologous gene regulation element, or a heterologous gene. Useful heterologous genes in the context of the present invention encode polypeptides of interest, i.e. polypeptides aiding in the production of the desired organic end product from the intermediate product obtained in step (ii) of the method of the invention. E.g., in the lignin processing method as contemplated herein, the intermediate product is envisaged to be catechol, and a host cell comprising at least one (optionally heterologous) gene encoding a polypeptide having catechol-1,2-dioxygenase activity is envisaged, in particular a *catA* gene and/or a *catA2* gene. Further heterologous genes that may advantageously be present in the host cell include genes for the metabolic funneling of aromats, e.g. phenol and cresol.

### Heterologous nucleic acid sequence

The term "heterologous" or "exogenous" nucleic acid sequence is used herein to refer to a nucleic acid sequence not naturally occurring in, i.e. foreign to, the host cell. In other words, a heterologous nucleic acid sequence is not found in wild-type host cells. The term includes nucleic acid sequences such as heterologous regulatory sequences (e.g. promoters) and heterologous genes. The heterologous nucleic acid sequences may be derived from another "donor" cell, or be a synthetic or artificial nucleic acid sequences. Heterologous gene(s) in the context of the present invention are in particular envisaged to encode for the polypeptide(s) of interest required for catalyzing conversion of said compound into a product (i.e. the desired organic end product), and optionally also for channeling in of the substrate (i.e. the intermediate product), and exporting the product from the host cell.

### Preparation

Recombinant host cells can be prepared using genetic engineering methods known in the art. The process of introducing nucleic acids into a recipient host cell is also termed "transformation" or "transfection" hereinafter. The terms are used interchangeably herein.

Host cell transformation typically involves opening transient pores or "holes" in the cell wall and/or cell membrane to allow the uptake of material. Illustrative examples of transformation protocols involve the use of calcium phosphate, electroporation, cell squeezing, dendrimers, liposomes, cationic polymers such as DEAE-dextran or polyethylenimine, sonoporation, optical transfection, impalefection, nanoparticles (gene gun), magnetofection, particle bombardement, alkali cations (cesium, lithium), enzymatic digestion, agitation with glass beads, viral vectors, or others. The choice of method is generally dependent on the type of cell being transformed, the nucleic acid to be introduced into the cell and the conditions under which the transformation is taking place.

### Transient expression

A nucleic acid molecule encoding a polypeptide of interest (for instance a polypeptide having catechol-1,2-dioxygenase activity) and an operably linked regulatory sequence such as a promoter may be introduced into a recipient host cell either as a non-replicating DNA or RNA molecule, which may be a linear molecule or a closed covalent circular molecule. Such molecules are incapable of autonomous replication, and the expression of the gene occurs through the transient expression of the introduced sequence.

### Stable expression

The heterologous nucleic acid sequence, in particular gene (for instance a gene encoding for a polypeptide having catechol-1,2-dioxygenase activity such as a *catA* or *catA2* gene) may be stably integrated into the host cell's genome. Permanent (stable) expression of the gene encoding the polypeptide of interest may be achieved by integration of the introduced DNA sequence into the host cell chromosome. Stable expression may also be achieved by providing the gene of interest in a vector capable of autonomously replicating in the host cell.
The vector employed for delivery of the heterologous nucleic acid sequence to be expressed stably is envisaged to be capable of integrating the desired gene sequences into the host cell chromosome, or of autonomously replicating within the host cell; thereby ensuring maintenance of the heterologous nucleic acid sequence in the host cell and stable integration into the host cell's genome. Cells with DNA stably integrated into their genomes can be selected by also introducing one or more markers into the vector, e.g. providing for prototrophy to an auxotrophic host, biocide (e.g. antibiotics or heavy metal) resistance, or the like. The selectable marker gene sequence can either be directly linked to the DNA gene sequences to be expressed, or introduced into the same cell by co-transfection. Additional elements may also be needed for optimal synthesis of mRNA. These elements may include splice signals, as well as transcription promoters, enhancers, and termination signals.

### Vector

The heterologous nucleic acid molecule of interest can be delivered to the host cell in the form of a vector, e.g. a plasmid or viral vector. If said heterologous nucleic acid molecule is e.g. a DNA molecule and comprises a gene of interest, it is envisaged that said vector comprises regulatory sequences that allow for the expression of said gene of interest. The vector may or may not comprise sequences enabling autonomous replication of said vector in the host cell, depending on whether transient or stable expression of the gene is intended, as explained above.

Any of a wide variety of vectors may be employed for this purpose. The person skilled in the art will readily understand that selection of a particular vector include depends, e.g., on the nature of the host cell, the intended number of copies of the vector, whether transient or stable expression of the gene of interest is envisaged, and so on.

Illustrative examples of vectors conceivable for use in accordance with the invention include, without limitation, viral origin vectors (M13 vectors, bacterial phage λ vectors, baculovirus vectors, adenovirus vectors, and retrovirus vectors), high, low and adjustable copy number vectors, eukaryotic episomal replication vectors (pCDM8), and prokaryotic expression vectors such as pcDNA II, pSL301, pSE280, pSE380, pSE420, pTrcHisA, B, and C, pRSET A, B, and C (Invitrogen, Inc.), pGEMEX-1, and pGEMEX-2 (Promega, Inc.), the pET vectors (Novagen, Inc.), pTrc99A, pKK223-3, the pGEX vectors, pEZZ18, pRIT2T, and pMC1871 (Pharmacia, Inc.), pKK233-2 and pKK388-1 (Clontech, Inc.), and pProEx-HT (Life Technologies, Inc.) and variants and derivatives thereof. Vectors can also be eukaryotic expression vectors such as pFastBac, pFastBac HT, pFastBac DUAL, pSFV, and pTet-Splice (Life Technologies, Inc.), pEUK-CI, pPUR, pMAM, pMAMneo, pBI101, pBI121, pDR2, pCMVEBNA, and pYACneo (Clontech), pSVK3, pSVL, pMSG, pCH110, and pKK232-8 (Pharmacia, Inc.), p3'SS, pXTI, pSG5, pPbac, pMbac, pMC1neo, and pOG44 (Stratagene, Inc.), and pYES2, pAC360, pBlueBacHis A, B, and C, pVL1392, pBsueBaclll, pCDM8, pcDNAI, pZeoSV, pcDNA3 pREP4, pCEP4, and pEBVHis (Invitrogen, Inc.) and variants or derivatives thereof are also conceivable.

Further vectors of interest include pUC 18, pUC 19, pBlueScript, pSPORT, cosmids, phagemids, fosmids (pFOSI), YAC's (yeast artificial chromosomes), BAC's (bacterial artificial chromosomes), pBAC 108L, pBACe3.6, pBeloBACl1 (Research Genetics), PACs, P1 (E. coli phage), pQE70, pQE60, pQE9 (Qiagen), pBS vectors, PhageScript vectors, BlueScript vectors, pNH8A, pNH16A, pNH18A, pNH46A (Stratagene), pcDNA3 (InVitrogen), pGEX, pTrsfus, pTrc99A, pET-5, pET-9, pKK223-3, pKK233-3, pDR540, pRIT5 (Pharmacia), pSPORT1, pSPORT2, pCMVSPORT2.0, pSV-SPORT1 (Life Technologies, Inc.), and the vectors described in Provisional Patent Application No. 60/065,930, filed Oct. 24, 1997, and variants or derivatives thereof.

It will be acknowledged that the vector may comprise regulatory sequences as exemplified elsewhere herein, preferably operably linked to, e.g. upstream of, the gene of interest.

After the introduction of the vector, recipient cells can be grown in a selective medium, which selects for the growth of vector-containing cells. Expression of the heterologous gene(s) of interest (e.g. a *catA* or *catA2* gene) is envisaged to result in the production of a polypeptide of interest (e.g. a polypeptide having catechol-1,2-dioxygenase activity).

### Catechol-1,2-dioxygenase activity

As described in the foregoing, the (optionally heterologous) gene of interest preferably encodes a polypeptide of interest having a desired capability that beneficially enables biocatalytic conversion of the intermediate product into the organic end product according to the methods of the invention. In particular as regards the "lignin processing" method as described herein, the host cell preferably expresses at least one (optionally heterologous) gene encoding a polypeptide having catechol-1,2-dioxygenase activity. Said (optionally heterologous) gene may be a *catA* gene or a *catA2* gene. It is also envisioned herein to use host cells comprising both at least one *catA* gene and at least one *catA2* gene.

Catechol-1,2-dioxygenase (EC 1.13.11.1) catalyzes intradiol (or ortho-) cleavage of catechol as, thereby producing cis-cis muconic acid. Catechol-1,2-dioxygenase activity can be easily assessed spectrophotometrically by measurement of the increase in absorbance at λ = 260 nm, corresponding to the formation of cis,cis-muconic acid as reported by Silva et al. Braz J Microbiol. 2013; 44(1): 291-297.

### CatA and CatA2

Said gene of interest encoding a polypeptide having catechol-1,2-dioxygenase activity is envisaged to be a *catA* or *catA2* gene. The protein encoded by a *catA* gene or a *catA2* gene may be identified in a database as a catechol-1,2-dioxygenase.

The term "gene of interest" and "polypeptide of interest", in particular "catA" and/or "catA2", includes variants. The term "variant" or with reference to a nucleic acid or polypeptide refers to polymorphisms, i.e. the exchange, deletion, or insertion of one or more nucleotides or amino acids, respectively, compared to the predominant form of the respective nucleic acid or polypeptide. In the context of the present invention, a "variant" may refer to a contiguous sequence of at least about 50, such as about 100, about 200, or about 300 amino acids set forth in the amino acid sequence of a protein named herein (cf. e.g. below), or the corresponding full-length amino acid sequence, with the proviso that said alteration is included in the respective amino acid sequence. In case the mutation leads to a premature stop codon in the nucleotide sequence encoding the protein, the sequence may even be shorter than the corresponding wild type protein.

Variants of genes of interest as described herein, in particular catA and/or catA2, may be orthologs. An ortholog, or orthologous gene, is a gene with a sequence that has a portion with similarity to a portion of the sequence of a known gene, but found in a different species than the known gene. An ortholog and the known gene originated by vertical descent from a single gene of a common ancestor.

As used herein a variant or ortholog of the *catA* or *catA2* gene is envisaged to encode a protein having catechol-1,2-dioxygenase activity and having at least about 60 %, at least about 65 %, at least about 70 %, at least about 75 %, at least about 80 % or at least about 90 %, including at least 95%, at least 97%, at least 98%, at least 99%, or at least 99.5% identity or 100% sequence identity with a known *catA* gene, in particular PP_3713 of P. *putida* KT2440, or a known *catA2* gene, in particular PP_3166 of P. *putida* KT2440, respectively.

Variants substantially similar to known POIs, in particular catA and/or catA2 polypeptides, are preferred. A sequence that is substantially similar to a catA or catA2 polypeptide may have at least about 60 %, at least about 65 %, at least about 70 %, at least about 75 %, at least about 80 % or at least about 90 %, including at least 95%, at least 97%, at least 98%, at least 99%, or at least 99.5% identity or 100% sequence identity with the sequence of a known catA or catA2 or polypeptide, respectively.

By "% identity" is meant a property of sequences that measures their similarity or relationship. Identity is measured by dividing the number of identical residues by the total number of residues and gaps and multiplying the product by 100. Preferably, identity is determined over the entire length of the sequences being compared. "Gaps" are spaces in an alignment that are the result of additions or deletions of amino acids. Thus, two copies of exactly the same sequence have 100% identity, but sequences that are less highly conserved, and have deletions, additions, or replacements, may have a lower degree of identity. Those skilled in the art will recognize that several computer programs are available for determining sequence identity using standard parameters, for example Blast (Altschul, et al. (1997) Nucleic Acids Res. 25:3389-3402), Blast2 (Altschul, et al. (1990) J. Mol. Biol. 215:403-410), and Smith-Waterman (Smith, et al. (1981) J. Mol. Biol. 147:195-197). The term "mutated" or "mutant" in reference to a nucleic acid or a polypeptide refers to the exchange, deletion, or insertion of one or more nucleotides or amino acids, respectively, compared to the naturally occurring nucleic acid or polypeptide.

"Sequence identity" or "% identity" refers to the percentage of residue matches between at least two polypeptide or polynucleotide sequences aligned using a standardized algorithm. Such an algorithm may insert, in a standardized and reproducible way, gaps in the sequences being compared in order to optimize alignment between two sequences, and therefore achieve a more meaningful comparison of the two sequences. Sequence comparisons can be performed using standard software programs such as the NCBI BLAST program.

In the context of the invention, the expression "position corresponding to another position" (e.g., regions, fragments, nucleotide or amino acid positions, or the like) is based on the convention of numbering according to nucleotide or amino acid position number and then aligning the sequences in a manner that maximizes the percentage of sequence identity. Because not all positions within a given "corresponding region" need be identical, nonmatching positions within a corresponding region may be regarded as "corresponding positions." Accordingly, as used herein, referral to an "amino acid position corresponding to amino acid position [X]" of a specified protein sequence represents, in addition to referral to amino acid positions of the specified protein sequence, referral to a collection of equivalent positions in other recognized protein and structural homologues and families.

Thus, when a position is referred to as a "corresponding position" in accordance with the disclosure it is understood that nucleotides/amino acids may differ in terms of the specified numeral but may still have similar neighbouring nucleotides/amino acids. Such nucleotides/amino acids which may be exchanged, deleted or added are also included in the term "corresponding position".

Specifically, in order to determine whether an amino acid residue of the amino acid sequence of a polypeptide of interest, e.g. a catA or catA2 polypeptide different from a known host cell, corresponds to a certain position in the amino acid sequence of the known host cell, a skilled artisan can use means and methods well-known in the art, e.g., alignments, either manually or by using computer programs such as BLAST2.0, which stands for Basic Local Alignment Search Tool or ClustalW or any other suitable program which is suitable to generate sequence alignments. Accordingly, a known wild-type catA or catA2 polypeptide (or nucleic acid encoding the same) may serve as "subject sequence" or "reference sequence", while the amino acid sequence of a catA or catA2 polypeptide (or nucleic acid sequence encoding the same) different from the wild-type can serve as "query sequence". The terms "reference sequence" and "wild type sequence" are used interchangeably herein.

As set out above, a host cell employed in the methods of the invention -in particular in the lignin processing method as described herein- may comprise a *catA* gene. The *catA* gene may be an endogenous gene or a heterologous gene. Said *catA* gene may be under the control of an endogenous promoter, either a wild-type promoter or a mutated promoter, or a heterologous promoter. Additionally or alternatively, the host cell may comprise a *catA2* gene. The *catA2* gene may be an endogenous gene or a heterologous gene. Said *catA2* gene may be under the control of an endogenous promoter, either a wild-type promoter or a mutated promoter, or a heterologous promoter. Said promoter may be different from the promoter that controls expression of the *catA* gene. The *catA* gene may be under the control of a promoter that is similar or identical to the promoter that controls the *catA2* gene.

It is in particular envisaged that the host cell may comprise a (optionally heterologous) *catA* gene and a (optionally heterologous) *catA2* gene. Thus, the host cell may comprise an endogenous *catA* gene and an endogenous *catA2* gene. A host cell comprising a heterologous *catA* gene and a heterologous *catA2* gene is also conceivable. Also envisaged herein are host cells comprising an endogenous *catA* gene and a heterologous *catA2* gene, and *vice versa.*

Host cells comprising at least one endogenous gene encoding a polypeptide having catechol-1,2-dioxygenase activity, such as a *catA* and/or *catA2* gene as described herein, include, without limitation, *Acinetobacter sp.* (e.g. *A. calcoaceticus* PHEA-2, *A*. *gyllenbergii* NIPH 230, *A. junii* CIP 64.5, *A. Iwoffii* NCTC 5866 = CIP 64.10, *A*. *oleivorans* DR1, *A. radioresistens* DSM 6976 = NBRC 102413 = CIP 103788, *A. schindleri* CIP 107287, *A*. *schindleri* CIP 107287), *Amycolatopsis mediterranei* U32, *Amycolatopsis sp.* ATCC39116, *Arthrobacter sp., Aspergillus sp.* (e.g. *A. niger* CBS 513.88, *A. oryzae* RIB40), *Bordetella holmesii* ATCC 51541, *Bradyrhizobium sp.* (e.g. *B. diazoefficiens* USDA 110, *B. genosp.* SA-4 str. CB756), *Burkholderia sp.* (e.g. *B. cenocepacia* J2315, *B*. *glumae* BGR1, *B. mallei* ATCC 23344, *B*. *multivorans, B. pseudomallei* K96243, *B*. *xenovorans* LB400), *Candida dubliniensis* CD36, *Corynebacterium glutamicum* ATCC 13032, *Cupriavidus metallidurans* CH34, *Delftia acidovorans* SPH-1, *Enterobacter aerogenes* KCTC 2190, *Herbaspirillum seropedicae* SmR1, *Klebsiella pneumoniae subsp. pneumoniae* HS11286, *Mycobacterium smegmatis str.* MC2 155, *Neorhizobium galegae bv. orientalis str.* HAMBI 540, *Neurospora crassa* OR74A, *Pseudomonas sp.* (e.g. *P. aeruginosa* PAO1, *P. fluorescens* SBW25, *P. fragi B25, P. putida* KT2440, *P. stutzeri* A1501), *Ralstonia sp.* (e.g. *R. eutropha* H16, *R. pickettii* 12J), *Rhizobium sp.* (e.g. *R. etli* CFN 42, *R. leguminosarum bv. trifolii* CB782), *Rhodococcus* sp. (e.g. *R. erythropolis* PR4), *R. fascians* NBRC 12155 = LMG 3623, *R. jostii* RHA1), *Sinorhizobium sp.* (e.g. *S*. *fredii* NGR234, *S*. *meliloti* 1021, *S*. *wenxiniae*), *Sphingomonas sp.* KA1, *Thermus thermophilus* HB8, *Verticillium albo-atrum VaMs.102.*

Particularly preferred host cells for use in lignin processing as described herein are selected from Pseudomonas, preferably P. putida, and more preferably from P. putida strain KT2440 BN6.

As set out herein, the GOI encoding a polypeptide of interest, e.g. a polypeptide having catechol-1,2-dioxygenase activity, may be encoded by a heterologous gene. Said heterologous gene may be a *catA* gene or a *catA2* gene. The host cell comprising the heterologous gene is also termed "recipient host cell" herein, whereas the host cell from which the heterologous gene is obtained is also referred to as "donor host cell": Suitable donor host cells include host cells comprising an endogenous gene encoding for a polypeptide of interest, e.g. with regards to the lignin processing method described herein, a polypeptide having catechol-1,2-dioxygenase activity such as a catA polypeptide and/or a catA2 polypeptide. The skilled person will readily acknowledge that heterologous genes encoding for polypeptides of interest, e.g. polypeptides having catechol-1,2-dioxygenase activity, can advantageously be obtained from host cells expressing said gene endogenously, e.g. host cells expressing an endogenous catechol-1,2-dioxygenase as listed above. Exemplary donor cells can be selected from *Pseudomonas,* preferably P. *putida,* more preferably P. *putida* strain KT2440 BN6. The heterologous gene encoding the polypeptide of interest (for instance a polypeptide having catechol-1,2-dioxygenase activity) can be introduced using into the recipient host cell using recombinant DNA technology as described elsewhere herein. Any of the various host cells specified herein is in principle suitable as a recipient host cell. E.g., host cells not expressing an endogenous *catA* and/or *catA2* gene may be selected as recipient host cells.

The *catA* gene is in particular envisaged to be the gene PP_3713 encoding the catA polypeptide of *Pseudomonas putida,* strain KT2240 BN6, with Uniprot accession No. Q88GK8 (Version 79 of 04 Feb 2015), and may also be referred to as PP_3713. Variants of PP_3713 may also be used. It is further envisaged that said the *catA* gene may encode for a polypeptide comprising a sequence corresponding to SEQ ID No. 1. Said *catA* gene may comprise a sequence corresponding to SEQ ID No. 2. In BN6, the catA is induced by Pem7, the latter constitutive promotor replaced the promotor catR which is present in the wild type.

The *catA2* gene is in particular envisaged to be the gene PP_3166 encoding the catA2 polypeptide of *Pseudomonas putida,* strain KT2240, with Uniprot accession No. Q88I35 (Version 70 of 22 Jul 2015). Variants of PP_3166 may also be used. It is envisaged that said the *catA2* gene may encode for a polypeptide comprising a sequence corresponding to SEQ ID No. 3. Said *catA2* gene may comprise a sequence corresponding to SEQ ID No. 4.

In accordance with the foregoing, it is envisaged that the host cell may comprise at least one (optionally heterologous) *catA* gene, optionally comprising a sequence corresponding to SEQ ID No. 2; and/or at least one (optionally heterologous) *catA2* gene, optionally comprising a sequence corresponding to SEQ ID No. 4. Said host cell may thus express a (optionally heterologous) catA polypeptide comprising a sequence corresponding to SEQ ID No. 1; and/or a (optionally heterologous) catA2 polypeptide comprising a sequence corresponding to SEQ ID No. 3.

### Further genes

It is further envisaged that the host cell may be equipped with further (optionally heterologous) genes which advantageously aid in converting (by-)products obtained during sub- and/or supercritical fluid-assisted conversion. An exemplary (by-)product would be protocatechuate, a key intermediate in degradation of the lignin and other aromic compounds, such as vanillate, benzoate, coumarate and ferulate. Protocatechuate decarboxylase (AroY, EC 4.1.1.63) is an enzyme which catalyzes the conversion of protocatechuate to catechol and therefore enables the use of multiple aromatic compounds as carbon sources. Furthermore the presence of 4 hydroxybenzoate decarboxylase subunit B (KpdB, EC 4.1.1.61) was shown to increase the activity of AroY (T. Sonoki et al. J Biotechnol. 2014 Dec 20;192 Pt A:71-7.)

### AroY

In particular with regards to lignin processing, it is thus envisioned that the host cell may further comprise an (optionally heterologous) gene encoding for a polypeptide having protocatechuate decarboxylase (EC 4.1.1.63) activity. An illustrative example is the AroY polypeptide of *Klebsiella pneunomia,* strain A170-10, with Uniprot accession No. B9A9M6 (version 14 of 24 July 2015) or a variant thereof. Said polypeptide may comprise a sequence corresponding to SEQ ID No. 17. The gene may be an *AroY* gene of *K. pneunomia,* strain A170-10 comprising a sequence corresponding to SEQ ID No. 18 or a variant thereof. E.g., an exemplary codon-optimized version of the *AroY* gene according to SEQ ID No. 33 may also be used.

### KpdB

Additionally, the host cell may comprise an (optionally heterologous) gene encoding for a polypeptide having 4 hydroxybenzoate decarboxylase subunit B activity (KpdB, EC 4.1.1.61). An illustrative example is the KpdB polypeptide of *Klebsiella pneunomia* NBRC 114940, with Uniprot accession No. X5I148 (version 5 of 22 July 2015) or a variant thereof. Said polypeptide may comprise a sequence corresponding to SEQ ID No. 19. The gene may be the *kdpb* gene of *K. pneunomia* NBRC 114940 comprising a sequence corresponding to SEQ ID No. 20 or a variant thereof. E.g., an exemplary codon-optimized version of the *kpdb* gene according to SEQ ID No. 34 may be used.

### pheA

Phenol and cresol are the two main by-products that accumulate during sub- and/or supercritical fluid-assisted conversion of lignin.

Thus, for the conversion of phenol, in particular with regards to lignin processing as described herein, it is envisioned that the host cell may further comprise an (optionally heterologous) gene encoding for a polypeptide having phenol 2-monooxygenase activity. An illustrative example is the pheA polypeptide of P. *putida* sp. EST1001 with Uniprot Acc. No. Q52159 (version 54 of 24 June 2015) comprising a sequence corresponding to SEQ ID No. 21. Said polypeptide may be encoded by a gene comprising a sequence corresponding to SEQ ID No. 22 or a variant thereof.

### pcmh

For the conversion of cresol, in particular with regards to lignin processing as described herein, it is envisioned that the host cell may further comprise an (optionally heterologous) gene encoding for a polypeptide having p-cresol methylhydroxylase activity. An illustrative example is the pcmh polypeptide comprising the pchF subunit of *Pseudomonas putida* (*Arthrobacter siderocapsulatus*) with Uniprot Acc. R9WN81 (version 12 of May 27, 2015) and the pchC subunit of *Pseudomonas putida* (*Arthrobacter siderocapsulatus*) with Uniprot Acc. No. P09787 (version 94 of April 1, 2015). Said pchF subunit may comprise a sequence corresponding to SEQ ID No. 23. Said pchC subunit may comprise a sequence corresponding to SEQ ID No. 25. The pchF subunit may be encoded by a gene comprising a sequence corresponding to SEQ ID No. 24 or a variant thereof. The pchC subunit may be encoded by a gene comprising a sequence corresponding to SEQ ID No. 26 or a variant thereof.

Other genes useful for processing of (by-)products are also conceivable and can be selected by the skilled person in the art depending on the intermediate product(s) obtained after sub- and/or supercritical fluid assisted conversion and the desired organic products to be obtained.

The genes described herein may be operable linked to an (optionally heterologous) promoter, said promoter may comprise a sequence corresponding to SEQ ID No. 5, 6, 7, 8, 9, 10, 11, 12, 13, 14, 15 or 16. An optional construct comprising aroY and kdpb operably linked to, e.g. upstream of, suitable promoter sequences is disclosed in SEQ ID No. 35. An optional construct comprising pheA and pcmh operably linked to, e.g. upstream of, suitable promoter sequences is disclosed in SEQ ID No. 36.

### Regulatory sequences

The terms "expression" and "expressed", as used herein, are used in their broadest meaning, to signify that a sequence included in a nucleic acid molecule and encoding a peptide/protein is converted into its peptide/protein product. Thus, where the nucleic acid is DNA, expression refers to the transcription of a sequence of the DNA into RNA and the translation of the RNA into protein. A nucleic acid molecule, such as DNA, is said to be "capable of expressing" a peptide/protein if it contains nucleotide sequences which contain transcriptional and translational regulatory information and such sequences are operably linked to nucleotide sequences which encode the polypeptide. An operable linkage is a linkage in which a nucleotide sequence encoding a polypeptide of interest is linked to one or more regulatory sequence(s) such that expression of said nucleotide sequence can take place. Thus, a regulatory sequence operably linked to a coding sequence is capable of effecting the expression of the coding sequence, for instance in an *in vitro* transcription/ translation system or in a cell when the vector is introduced into the cell. A respective regulatory sequence need not be contiguous with the coding sequence, as long as it functions to direct the expression thereof. Thus, for example, intervening untranslated yet transcribed sequences may be present between a promoter sequence and the coding sequence and the promoter sequence can still be considered "operably linked" to the coding sequence.

The term "regulatory sequence" includes controllable transcriptional promoters, operators, enhancers, silencers, transcriptional terminators, 5' and 3' untranslated regions which interact with host cellular proteins to carry out transcription and translation and other elements that may control gene expression including initiation and termination codons. The regulatory sequences can be native (endogenous), or can be foreign (heterologous) to the cell. The precise nature of the regulatory regions needed for gene sequence expression may vary from organism to organism, but shall in general include a promoter region which, in prokaryotes, contains both the promoter (which directs the initiation of RNA transcription) as well as the DNA sequences which, when transcribed into RNA, will signal synthesis initiation. The term "promoter" as used herein, refers to a nucleic acid sequence that operates gene expression. For example, in prokaryotes, the promoter region contains both the promoter (which directs the initiation of RNA transcription) as well as the DNA sequences which, when transcribed into RNA, will signal synthesis initiation. Such regions will normally include those 5'-non-coding sequences involved with initiation of transcription and translation, such as the TATA box, capping sequence or the CAAT sequence. Promoter regions vary from organism to organism, but are well known to persons skilled in the art.

The promoter operably linked to and thus driving the expression of the gene of interest in the host cell may be an endogenous, i.e. wild-type or mutated, promoter, or a heterologous promoter. Two nucleic acid sequences (such as a promoter region sequence and a sequence encoding a *catA* or *catA2* polypeptide) are said to be operably linked if the nature of the linkage between the two DNA sequences does not (1) result in the introduction of a frame-shift mutation, (2) interfere with the ability of the promoter region sequence to direct the transcription of a gene sequence encoding the polypeptide of interest, in particular a *catA* or *catA2* polypeptide, or (3) interfere with the ability of the gene sequence of a polypeptide of interest, in particular a *catA* or *catA2* polypeptide to be transcribed by the promoter region sequence.

Thus, a promoter region is operably linked to a gene if the promoter is capable of driving transcription of said gene.

### Promoters

Promoters are regarded as molecular tools that enable the modulation and regulation of expression of genes of interest in homologous organisms as well as in heterologous organisms. In order to allow for fast and efficient conversion of the intermediate product, advantageously promoters allowing for standardized and constitutive expression (i.e. continuous gene transcription) control the expression of the genes of interest (i.e. genes involved in intermediate product processing). Said promoters can be heterologous promoters or endogenous promoters. It is in particular envisaged that such promoters enable constitutive and/or standardized expression of the downstream genes, and preferably abolish the need for induction of the genes of interest. The promoter may also be equipped with a regulatory sequence/element that makes the promoter inducible and/or repressible.

E.g., with regards to lignin processing, constitutive promoters are envisaged to operably linked to, e.g. upstream of, the (optionally heterologous) *catA* gene and/or the (optionally heterologous) *catA2* gene. It is envisioned that constitutive expression of preferably *catA* and *catA2* under the control of said promoters allows for efficient conversion of catechol into the desired end product cis-cis-muconic acid, thereby preventing accumulation of the (toxic) intermediate product. If further (optionally heterologous) genes are present in the host cell that allow for conversion of other (by-)products of sub- and/or supercritical fluid-assisted conversion, a constitutive promoter may also be pesent or introduced operably linked to, e.g. upstream of, said genes.

Suitable promoters may be strong constitutive promoters, such as promoters naturally controlling the expression of housekeeping genes which typically constitutive genes that are transcribed at a relatively constant level as their products are typically needed for maintenance of the cell and their expression (PrpoD, PgyrB, Ptuf and PgroES) is usually unaffected by experimental conditions.

Promoter strength may be tuned to be appropriately responsive to activation or inactivation. The promoter strength may also be tuned to constitutively allow an optimal level of expression of a gene of interest or of a plurality of gene of interest. Strength of expression can, for example, be determined by the amount/yield of organic end product production and/or by quantitative reverse transcriptase PCR (qRT-PCR).

Illustrative examples of a strong constitutive promoter include, but are not limited to, the T7 promoter, the T5 promoter, the *Escherichia coli* lac promoter, the trc promoter, the tac promoter, the recA promoter, the adenyl methyltransferase (AMT) promoters AMT-1 and AMT-2, and synthetic promoters derived from the foregoing promoters or e.g. Pcp7 as disclosed in Spexard et al (Biotechnol Lett (2010) 32, 243-248).

The present inventors further provide a promoter library comprising particulary suitable promoters for regulating the expression of the genes of interest, especially *catA* and/or *catA2.* Said promoters favorably allow constitutive expression, and preferably strong and standardized expression, of *catA* and/or *catA2,* and are envisaged to comprise a sequence corresponding to
(i) SEQ ID No. 5 [Pem7]; or
(ii) SEQ ID No. 6 [Pem7*]; or
(iii) SEQ ID No. 7 [Ptuf]; or
(iv) SEQ ID No. 8 [PrpoD]; or
(v) SEQ ID No. 9 [Plac]; or
(vi) SEQ ID No. 10 [PgyrB]; or
(vii) SEQ ID No. 11; or
(viii) SEQ ID No. 12; or
(ix) SEQ ID No. 13; or
(x) SEQ ID No. 14; or
(xi) SEQ ID No. 15; or
(xii) SEQ ID No. 16, or
(xiii) SEQ ID No. 88 [Ptuf_1]; or
(xiv) SEQ ID No. 89 [Ptuf_short]; or
(xv) SEQ ID No. 90 [Ptuf_s_2]; or
(xvi) SEQ ID No. 91 [Ptuf_s_3]; or
(xvii) SEQ ID No. 92 [Ptuf_s_4]; or
(xviii) SEQ ID No. 93 [Ptuf_s_5]; or
(xix) SEQ ID No. 94 [Ptuf_s_6]; or
(xx) SEQ ID No. 95 [Ptuf_s_7]; or
(xxi) SEQ ID No. 96 [Ptuf_s_8]; or
(xxii) SEQ ID No. 97 [Ptuf_s_9]; or
(xxiii) SEQ ID No. 98 [Ptuf_s_10]; or
(xxiv) SEQ ID No. 99 [Ptuf_s_11]; or
(xxv) SEQ ID No. 100 [Ptuf_s_12]; or
(xxvi) SEQ ID No. 101 [Pgro]; or
(xxvii) SEQ ID No. 102 [Pgro_1]; or
(xxviii) SEQ ID No. 103 [Pgro_2]; or
(xxix) SEQ ID No. 104 [Pgro_4]; or
(xxx) SEQ ID No. 105 [Pgro_5].

In particular with regards to lignin processing, it is thus envisaged to employ a host cell such as *P*. *putida* comprising at least one (optionally heterologous) *catA* gene and/or at least one (optionally heterologous) *catA2* gene as described elsewhere herein, each or any of said genes under the control of an (optionally heterologous) promoter comprising a sequence corresponding to SEQ ID No. 5 [Pem7]; or SEQ ID No. 6 [Pem7*]; or SEQ ID No. 7 [Ptuf]; or SEQ ID No. 8 [PrpoD]; or SEQ ID No. 9 [Plac]; SEQ ID No. 10 [PgyrB], or SEQ ID No. 11; or SEQ ID No. 12; or SEQ ID No. 13; or SEQ ID No. 14; or SEQ ID No. 15; or SEQ ID No. 16; SEQ ID No. 88 [Ptuf_1]; or SEQ ID No. 89 [Ptuf_short]; or SEQ ID No. 90 [Ptuf_s_2]; or SEQ ID No. 91 [Ptuf_s_3]; or SEQ ID No. 92 [Ptuf_s_4]; or SEQ ID No. 93 [Ptuf_s_5]; or SEQ ID No. 94 [Ptuf_s_6]; or SEQ ID No. 95 [Ptuf_s_7]; or SEQ ID No. 96 [Ptuf_s_8]; or SEQ ID No. 97 [Ptuf_s_9]; or SEQ ID No. 98 [Ptuf_s_10]; or SEQ ID No. 99 [Ptuf_s_11]; or SEQ ID No. 100 [Ptuf_s_12]; or SEQ ID No. 101 [Pgro]; or SEQ ID No. 102 [Pgro_1]; or SEQ ID No. 103 [Pgro_2]; or SEQ ID No. 104 [Pgro_4]; or SEQ ID No. 105 [Pgro_5].

### Endogenous promoters

Host cells comprising endogenous genes of interest and endogenous promoters (i.e., non-genetically modified host cells) are thus easy to work with and may be advantageous in a variety of applications. For example, in the lignin processing method described herein, *Pseudomonas sp.,* e.g., *Pseudomonas putida* comprising an endogenous *catA* gene and an endogenous *catA2* gene, both under the control of endogenous promoters, can be utilized.

### Heterologous promoters

The promoter may, however, also be heterologous to the host cell. A heterologous promoter can be introduced operably linked to, e.g. upstream of, the gene(s) of interest into the genome of a host cell which naturally harbors said genes using common genetic engineering techniques. The heterologous promoter may also be introduced operably linked to, e.g. upstream of, heterologous genes of interest and inserted as an expression cassette/unit into the genome of a host cell. The expression cassette may also be present in an extrachromosomal element such as a vector, e.g. a plasmid. Culture conditions

Host cells and recombinant host cells may be provided in any suitable form. For example, such host cells may be provided in liquid culture or solid culture (e.g., agar-based medium), which may be a primary culture or may have been passaged (e.g., diluted and cultured) one or more times. Host cells also may be provided in frozen form or dry form (e.g., lyophilized). Host cells may be provided at any suitable concentration.

Host cells are preferably cultured under conditions that allow production of the desired organic end product, e.g. cis-cis-muconic acid in the lignin processing method as described herein. Suitable conditions are within the routine knowledge of the skilled artisan. The term "cultivation of cells" or "culturing of cells" in medium in the context of the host cells of the present invention generally refers to the seeding of the cells into a culture vessel, to the growing of the cells in medium in the logarithmic phase until a sufficient cell density is established and/or to the maintenance of the cells in medium, respectively. Culturing can be performed in any container suitable for culturing cells.

The skilled person will readily understand that culture conditions will vary depending on the host cell, and the characteristics of the intermediate product and organic end product. Suitable conditions for culturing the host cell typically include culturing the same in an aqueous medium that is suitable for sustaining cell viability and cell growth and allows the host cell to produce the desired organic product. For instance, in the lignin processing method provided herein, suitable culture conditions that enable the biocatalyst, in particular *Pseudomonas sp.,* preferably *P. putida* and more preferably P. putida KT2440, to convert the substrate catechol into the desired organic product cis-cis-muconic acid, may comprise E-2 minimal medium with glucose as a carbon source (pH 7) and a reaction temperature of about 30°C as described in the appended examples. Also, in order to express the necessary enzymes, in particular *catA2* situated in the ben operon, expression of the catA2 polypeptide may require induction. Thus, addition of an agent for induction, e.g. benzoic acid, may be required.

### Preferred host cells

As described herein, the present inventors found that for the production of cis, cis-muconic acid from phenolic compounds, it is advantageous if a host cell expresses a functional catA, but does not express a functional catB polypeptide. Accordingly, disclosed is a host cell, preferably a bacterial host cell, wherein said host cell is characterized by expressing a functional catA polypeptide and by not expressing a functional catB polypeptide. A particularly preferred bacterial host cell is *Corynebacterium glutamicum* ATCC13032 or *Amycolatopsis* sp. ATCC39116. When used herein, terms like "does not express a functional gene (as described herein)", "does not express a functional polypeptide (as described herein)", "not expressing a functional gene (as described herein)", or "not expressing a functional polypeptide (as described herein)" or grammatical variations thereof means that a host cell as described herein is modified so as to inactivate or disrupt expression of a gene or polypeptide as described herein. Inactivation or disruption may be achieved by means and methods commonly known in the art, e.g. by knocking out a gene of interest, by insertion mutagenesis, by introducing a stop codon into the open reading frame, by inactivating the promoter, etc.

It is preferred that said host cell expresses a functional catA polypeptide, said catA polypeptide being characterized in that it has (a) the amino acid sequence shown in SEQ ID No. 108; or (b) an amino acid sequence which has at least 40% identity to the amino acid sequence shown in SEQ ID No. 108 and having catechol-1,2-dioxygenase activity, e.g. SEQ ID No. 122; and does not express a functional catB polypeptide, said catB polypeptide being characterized in that it has (c) the amino acid sequence shown in SEQ ID No. 109; or (d) an amino acid sequence which has at least 25% identity to the amino acid sequence shown in SEQ ID No. 109 and having muconate cycloisomerase activity, e.g. SEQ ID No. 123, 124, or 125. Such a host cell is preferably a bacterial host cell, in particular *Corynebacterium glutamicum* ATCC13032 or *Amycolatopsis* sp. ATCC39116.

Disclosed is also *Corynebacterium,* preferably *Corynebacterium glutamicum,* more preferably *Corynebacterium glutamicum* ATCC 13032 having its endogenous catA gene operably linked to a heterologous promoter and which does not express a functional catB polypeptide. It is preferred that the heterologous promoter is a strong promoter which overexpresses the endogenous catA gene in comparison to the endogenous promoter of the catA gene.

Preferably, the catA gene of *Corynebacterium,* preferably *Corynebacterium glutamicum,* more preferably *Corynebacterium glutamicum* ATCC 13032 encodes a polypeptide having an amino acid sequence which has at least 70% identity to the amino acid sequence shown in SEQ ID No. 108 and has catechol-1,2-dioxygenase activity. More preferably, the catA gene encodes a polypeptide having the amino acid sequence shown in SEQ ID No. 108.

Preferably, the catB polypeptide of *Corynebacterium,* preferably *Corynebacterium glutamicum,* more preferably *Corynebacterium glutamicum* ATCC 13032 has an amino acid sequence which has at least 70% identity to the amino acid sequence shown in SEQ ID No. 109 and has muconate cycloisomerase activity. More preferably, the catB polypeptide has the amino acid sequence shown in SEQ ID No. 109.

Disclosed is *Amycolatopsis,* preferably *Amycolatopsis* sp., more preferably *Amycolatopsis* sp. ATCC 39116 which expresses its endogenous catA gene and does not express a functional catB polypeptide.

It is also envisioned that *Amycolatopsis,* preferably *Amycolatopsis* sp., more preferably *Amycolatopsis* sp. ATCC 39116 has its endogenous catA gene operably linked to a heterologous promoter and does not express a functional catB polypeptide. It is preferred that the heterologous promoter is a strong promoter which overexpresses the endogenous catA gene in comparison to the endogenous promoter of the catA gene.

Preferably, the catA gene of *Amycolatopsis,* preferably *Amycolatopsis* sp., more preferably *Amycolatopsis* sp. ATCC 39116 encodes a polypeptide having an amino acid sequence which has at least 70% identity to the amino acid sequence shown in SEQ ID No. 122 and having catechol-1,2-dioxygenase activity. More preferably, the catA gene encodes a polypeptide has the amino acid sequence shown in SEQ ID No. 122

Preferably, the catB polypeptide of *Amycolatopsis,* preferably *Amycolatopsis* sp., more preferably *Amycolatopsis* sp. ATCC 39116 has an amino acid sequence which has at least 70% identity to the amino acid sequence has shown in SEQ ID No. 123, 124 or 125 and has muconate cycloisomerase activity. More preferably, the catB polypeptide has the amino acid sequence shown in SEQ ID No. 123, 124 or 125.

It is preferred that *Amycolatopsis,* preferably *Amycolatopsis* sp., more preferably *Amycolatopsis* sp. ATCC 39116 does not express a functional catB polypeptide having the amino acid sequence shown in SEQ ID No. 123 and a functional catB polypeptide having the amino acid sequence shown in SEQ ID No. 124.

It is preferred that *Amycolatopsis,* preferably *Amycolatopsis* sp., more preferably *Amycolatopsis* sp. ATCC 39116 does not express a functional catB polypeptide having the amino acid sequence shown in SEQ ID No. 123 and a functional catB polypeptide having the amino acid sequence shown in SEQ ID No. 125.

It is preferred that *Amycolatopsis,* preferably *Amycolatopsis* sp., more preferably *Amycolatopsis* sp. ATCC 39116 does not express a functional catB polypeptide having the amino acid sequence shown in SEQ ID No. 124 and a functional catB polypeptide having the amino acid sequence shown in SEQ ID No. 125. Interestingly, such a cell, in addition to muconate also produces methyl-muconate from o-cresol and/or m-cresol. It would have been expected that o-cresol and/or m-cresol are converted into catechol, but the production of methyl-catechol was observed which is then converted into methyl-muconate.

It is preferred that *Amycolatopsis,* preferably *Amycolatopsis* sp., more preferably *Amycolatopsis* sp. ATCC 39116 does not express a functional catB polypeptide having the amino acid sequence shown in SEQ ID No. 123, a functional catB polypeptide having the amino acid sequence shown in SEQ ID No. 124, and a a functional catB polypeptide having the amino acid sequence shown in SEQ ID No. 125.

### Cell culture medium

Illustrative examples of a suitable cell culture medium, for example for culturing a bacterial host such as a *Pseudomonas* sp. host or a *Burkholderia sp.* host, include, but are not limited to, Luria-Bertani (LB) complex medium, Inkas-medium, phosphate-limited protease peptone-glucose-ammonium salt medium (PPGAS), Minimal medium E (MME), nitrogen-limited minimal medium or mineral salt medium. The media used may include a factor selected from growth factors and/or attachment factors or may be void of such a factor. It may be sufficient to add such a factor only to the media used for the seeding of the cells and/or the growing of the cells, for example under logarithmic conditions. The media may contain serum or be serum-free. A variety of carbon sources may be used such as a monosaccharide, e.g. glucose, a disaccharide, e.g. sucrose, an alcohol, e.g. glycerol, an alkane, e.g. n-hexane, a fatty acid such as caprylic acid (also termed octanoate), or mixtures thereof. The bacterial host cell may for instance be in the logarithmic growth phase or in the stationary phase.

Suitable cell culture media may further include salts, vitamins, buffers, energy sources, amino acids and other substances. Any medium may be used that is suitable to sustain cell viability and in which the selected host cell is capable of producing the desired organic end product (e.g. cis-cis-muconat), as explained above.

### Downstream metabolization

The host cells may further be characterized in that they do not express genes that catalyze downstream metabolization of the desired organic end product. As the host cells are employed for production of a specific desired target compound, further processing and degradation of the same should advantageously be avoided.

As will be acknowledged by the skilled artisan, genes encoding downstream processing factors for a given organic product will typically be present in cells that are capable of processing said organic product. E.g., in *Pseudomonas putida,* the *catB* and *catC* genes encode enzymes that catalyze consecutive reactions in the catechol branch of the beta-ketoadipate pathway synthesis of 5-oxo-4,5-dihydro-2-furylacetate from catechol. Another *P. putida* gene catalyzing downstream metabolization of catechol is *pcaB* which converts cis, cis-muconic acid to carboxy muconolactone in the structurally related protocatechuate branch. Other host cells capable of processing catechol may also comprise functional *catB* and/or *catC* and/or *pcaB* genes that may advantageously be removed or "turned off" in order to allow for accumulation of cis-cis-muconate..

Particularly in the lignin processing method according to the invention, and in order to avoid further processing of the desired end product cis-cis-muconate in the host cell, it is thus envisaged that the host cell does not express a functional *catB* polypeptide and/or that the host cell does not express a functional *catC* polypeptide and/or that the host cell does not express a functional *pcaB* polypeptide. It is therefore envisioned that said host cell does not comprise a functional *catB* gene and/or a functional *catC* gene and/or a functional *pcaB* gene, respectively.

The *catB* gene is in particular envisaged to encode a catB polypeptide having muconate cycloisomerase activity (EC 5.5.1.1), i.e. which is capable of synthesizing (S)-5-oxo-2,5-dihydro-2-furylacetate from cis-cis-muconic acid. An illustrative example of a catB polypeptide is the catB polypeptide of *Pseudomonas putida,* strain KT2240, with Uniprot accession No. Q88GK6 (version 67 of 22 July 2015). Said catB polypeptide may comprise a sequence corresponding to SEQ ID No. 27. An illustrative example of a *catB* gene is PP_3715 (SEQ ID No. 28). The term *catB* gene and *catB* polypeptide also comprises variants as defined elsewhere herein.

The *catC* gene is in particular envisaged to be encode a catC polypeptide having muconolactone Delta-isomerase activity (E.C. 5.3.3.4), i.e. which is capable of synthesizing 5-oxo-4,5-dihydrofuran-2-acetate from (S)-5-oxo-2,5-dihydrofuran-2-acetate. An illustrative example is the catC polypeptide of *Pseudomonas putida,* strain KT2240, with Uniprot accession No. Q88GK7 (version 67 of 22 July 2015). Said catC polypeptide may comprise a sequence corresponding to SEQ ID No. 29. An illustrative example of a *catC* gene is PP_3714 (SEQ ID No. 30). The term *catC* gene and catC polypeptide also comprises variants as defined elsewhere herein.

The *pcaB* gene is in particular envisaged to encode a pcaB polypeptide having 3-carboxy-cis,cis-muconate cycloisomerase activity, i.e. which is capable of synthesizing carboxy muconolactone from cis-cis-muconic acid. An illustrative example is the pcaB polypeptide of *Pseudomonas putida,* strain KT2240, with Uniprot accession No. Q88N37 (version 89 of 22 July 2015). Said pcaB polypeptide may comprise a sequence corresponding to SEQ ID No. 31. An illustrative example of a *pcaB* gene is PP_1379 (SEQ ID No. 32). The terms *"pcaB* gene" and "pcaB polypeptide" also comprises variants as defined elsewhere herein. A host cell "not comprising a functional *cat8*/*catClpcaBgene"* may either lack an endogenous catB/catC/pcaB gene, or it naturally comprises an endogenous *catB*/*catC*/*pcaB* gene, which is however silenced, preferably knocked-down or knocked-out, or deleted from the host cell chromosome. The skilled person is well aware of suitable methods for silencing endogenous genes, e.g. by manipulating the promoter region at a gene. It is preferred that the endogenous gene is knocked-down or knocked-out using by way of known methods, e.g. by recombinase techniques. Alternatively, the endogenous gene may be deleted from the chromosome by allelic substitution etc.

The term "silenced" is used herein to generally indicate that the expression of a gene is suppressed or inhibited as ascertainable e.g. by a reduced level of production or accumulation of the transcript or a processed product, for example of an mRNA, or of a translation product of the mRNA.

The level of expression of *catBIcatClpcaB* may be reduced by at least about 10%, by at least about 15%, by at least about 20%, by at least about 25%, at least about 30%, at least about 35%, at least about 40%, at least about 45%, at least about 50%, at least about 55%, at least about 60%, at least about 65%, at least about 70%, at least about 75%, at least about 80%, at least about 85% or more, including about 90% or more, about 95% or more including about 100%.

Genes encoding for downstream metaboliation of the desired organic products may for instance be knocked-out, i.e. made inoperable, resulting in an inhibition of gene expression, or translation of a non-functional protein product. Knock-out techniques are well-known in the art and include, e.g., introduction of one or more mutations into the *catBIcatClpcaB* gene, or into a regulatory sequence to which the respective gene is operably linked. Other methods include recombination techniques, e.g. resulting in the insertion of a foreign sequence to disrupt the gene or a deletion from the host cell's genome. Such a *catBIcatClpcaB* gene may be partially or fully inactivated, disrupted or otherwise blocked.

A knock-down of the *catBIcatClpcaB gene,* resulting in a reduced expression of said gene(s), is also conceivable. Several methods for gene knock-down are known in the art, and may involve either genetic modification or treatment with a reactant (the latter resulting in a transient knock-down). Genetic modifications resulting in a gene knock-down include, e.g., the incorporation of mutations into the target gene or a regulatory element operably linked thereto. In order to knock-down an endogenous gene, a heterologous molecule, such as a nucleic acid molecule, can be introduced into the host cell and upon introduction into a host cell reduces the expression of a target gene, typically through transcriptional and/or post-transcriptional silencing. Said reactant may be a nucleic acid molecule may be a silencing RNA, e.g. so-called "antisense RNA". Said antisense RNA typically includes a sequence of at least 20 consecutive nucleotides having at least 95% sequence identity to the complement of the sequence of the target nucleic acid, such as the coding sequence of the target gene, but may as well be directed to regulatory sequences of target genes, including the promoter sequences and transcription termination and polyadenylation signals. Other reactants useful for knock-down of target genes include small interfering RNAs (siRNAs), aptamers, Spiegelmers^{®}, nc-RNAs (including anti-sense-RNAs, L-RNA Spiegelmer, silencer RNAs, micro-RNAs (miRNAs), short hairpin RNAs (shRNAs), small interfering RNAs (siRNAs), repeat-associated small interfering RNA (rasiRNA), and molecules or an RNAs that interact with Piwi proteins (piRNA). Such non-coding nucleic acid molecules can for instance be employed to direct mRNA degradation or disrupt mRNA translation. A respective reactant, in particular RNA molecule, may in principle be directly synthesized within the host cell, or may be introduced into the host cell.

A different means of silencing exogenous DNA that has been discovered in prokaryotes is a mechanism involving loci called 'Clustered Regularly Interspaced Short Palindromic Repeats', or CRISPRs. Proteins called 'CRISPR-associated genes' (cas genes) encode cellular machinery that cuts exogenous DNA into small fragments and inserts them into a CRISPR repeat locus. When this CRISPR region of DNA is expressed by the cell, the small RNAs produced from the exogenous DNA inserts serve as a template sequence that other Cas proteins use to silence this same exogenous sequence. The transcripts of the short exogenous sequences are used as a guide to silence these foreign DNA when they are present in the cell.

Another technology involves the use of transcription activator-like effector nucleases (TALENs). TALENs are nucleases that have two important functional components: a DNA binding domain and a DNA cleaving domain. The DNA binding domain is a sequence-specific transcription activator-like effector sequence while the DNA cleaving domain originates from a bacterial endonuclease and is non-specific. TALENs can be designed to cleave a sequence specified by the sequence of the transcription activator-like effector portion of the construct. Once designed, a TALEN is introduced into a cell as a plasmid or mRNA. The TALEN is expressed, localizes to its target sequence, and cleaves a specific site. After cleavage of the target DNA sequence by the TALEN, the cell uses non-homologous end joining as a DNA repair mechanism to correct the cleavage. The cell's attempt at repairing the cleaved sequence can render the encoded protein non-functional, as this repair mechanism introduces insertion or deletion errors at the repaired site.

The capability of the host cell to degrade cis-cis-muconic acid to downstream products, in particular (S)-5-oxo-2,5-dihydro-2-furylacetate (in case of *catB* silencing or deletion) and/or 5-oxo-4,5-dihydrofuran-2-acetate (in case of *catC* silencing or deletion) and/or carboxy muconolactone (in case of *pcaB* silencing or deletion) may thus be reduced in comparison to a wild type cell, including entirely absent.

### Purification of organic compounds (e.g. cis-cis-muconic acid)

The host cells may be removed, for example by way of centrifugation or filtration, before recovering the one or more organic end products produced in a method according to the invention. E.g., host cells may be recovered, e.g. concentrated, captured, harvested and/or enriched in/on a separation or filter unit. For example, host cells as employed in the method according to the present invention may be enriched before they are collected and/or are concentrated before they are collected and/or are captured before they are collected. Enriching may, for example, be achieved by batch centrifugation, flow through centrifugation and/or tangential flow filtration.

The organic end product, e.g. cis-cis-muconic acid, may be advantageously secreted from the host cell, so that its formation can be easily analysed and/or monitored by standard techniques of cell culture broth analysis, including chromatographic techniques such as HPLC.

### Hydrogenation of cis-cis muconic acid

Methods for preparing adipic acid by hydrogenation of cis-cis muconic acids are well known in the art. Preferably, the hydrogenation is a chemical hydrogenation, for example, hydrogenation via Pd/C catalysis (Vardon et al, Energy Environ. Sci. 2015, (8), 617-628).

### Production of nylon 6,6

Production of Nylon starting from adipic acid is also well known in the art. For example, adipic acid can in a first step be transformed into adipoyl chloride followed by a condensation reaction with hexane-1,6-diamine to form Nylon 6,6.

### Step 1: Adipic acid to adipoyl dichloride

The following protocol is notlimiting but clearly demonstrate transfermotion of adipic acid to adipoyl chloride. Ten mmol (1.46 g) of adipic acid is placed in a 100-mL round-bottom flask equipped with a condenser, gas trap for HCL, and stir bar. Add 3.57 g (2.17 cc. 0.03 moles) of thionyl chloride (sulfurous dichloride) at once. The mixture is heated gently over heating mantle and stir plate held at 50-60° C. After about four hours, reaction is complete and evolution of hydrogen chloride has ceased. The flask is connected to a downward condenser tube and heated under diminished pressure by a heating mantle to remove any excess thionyl chloride. The light yellow residue of adipoyl dichloride is ready for use.

### Step 2: the Polyamide

### Procedure

Note: this *reaction* involves toxic vapors - use the ventilation hood at all times.

Disposal: Wash the nylon thoroughly with water and place in a waste basket for disposal. The liquid wastes should be poured into special designated containers.

Pour 10 mL of a 5% aqueous solution of hexamethylenediamine (hexane-1,6-diamine) into a 50-mL beaker. Add 10 drops of 20% sodium hydroxide solution. Carefully add 10 mL of a 5% solution of adipoyl chloride in cyclohexane to the solution by pouring it down the wall of the slightly tilted beaker. Two layers will form and a polymer film will immediately form at the liquid-liquid interface.

Using a copper-wire hook (6 inches, bent at one end), gently free the walls of the beaker from polymer strings. Hook the mass at the center and slowly raise the wire so that polyamide forms continuously, producing a rope that can be drawn out for many feet. The strand can be broken by pulling it faster. Rinse the rope several times with water and lay it on a paper towel to dry. With the piece of wire, vigorously stir the remainder of the two-phase system to form additional polymer.

Decant the liquid and wash the polymer thoroughly with water. Allow the polymer to dry. Do not discard in the sink; use a waste container.

### Caprolactam

cis, cis-MA can be used for the production of caprolactam. For example, good yield (around 55%) was obtained in the presence of Pd-Al₂O₃ (5 mol%) as catalyst, dissolved in dioxane together with ammonia (3.4 bar) and hydrogen (34 bar) at 250 °C for 2 h [Beerthuis et al., 2015; Couldray et al., 2012]. Directly from adipic acid, a yield of 64% caprolactam was generated in the presence of 5% Ru-Al₂O₃ (5 mol%) as catalyst, solved in tetrahydrofuran with ammonia (3.4 bar) and hydrogen (69 bar) at 250°C for 2 h see, e.g., Beerthuis R, Rothenberg G, Shiju NR (2015) Green Chemistry 17 (3):1341-1361 and Coudray L, Bui V, Frost JW, Schweitzer D (2012) WO2012141997.

### Terephthalic acid

trans, trans-MA and cis, trans-MA can be converted to terephthalic acid. Via a thermal inverse electron demand Diels-Adler reaction.

### Examples

### Lignin feedstocks

To comply with the main botanical families of lignocellulosic biomass various lignin types were taken into account. Kraft lignin of softwood named Lignoboost (KL1-SW) (Innventia, Sweden). Kraft lignin of softwood named Indulin AT (Meadwestvaco, US) (KL2-SW) and soda lignin of mixed wheat straw/ Sarkanda grass named Protobind^{™} 1000 (Greenvalue, CH) (SL-W) were obtained commercially. Three organosolv lignins were extracted from spruce (OS1-SW), poplar (OS2-HW), and wheat straw (OS3-W) using an acid-catalyzed ethanol-based organosolv process (Constant *et al.,* 2016; Wildschut *et al.,* 2013). The straw, poplar and spruce lignin were prepared via in-house organosolv fractionation (treatment with water/ ethanol (40/60 w/w) at 200°C for 30 minutes.

### LIBRA lignin pyrolysis

Four lignin types (KL1-SW, KL2-SW, OS1-SW, and SL-W) were pyrolyzed at different conditions (see Table 4) using a fully automated bubbling fluidized bed reactor. A solid feed rate of 10 g/min was applied. To specify in more detail KL1.1-SW and OS1-SW were pyrolyzed at 450°C, KL1.2-SW and SL-W at 550°C. KL1.3-SW was also pyrolyzed at 550°C with the additional presence of hydrogen at a ratio of 10%. The gas was bubbled (1 kg/h) at atmospheric pressure. The hot lignin pyrolysis vapours were first filtered at high temperature to remove fine particulate material (char and ash). Subesequenly, the hot pyrolysis vapours were quenched in 2 sequential 0°C impingers (Fig. 3). Most of the reaction water and part of the organic degradatives are condensed in these two impingers. Organic degradatives in aerosol form are not captured but precipitated / condensed in a downstream located electrostatic precipitator (ESP). Finally, low boiling organics such as methanol, acetone and acetic acid are trapped in the freeze condenser downstream the ESP. As a result, two-phase liquid products with an upper aqueous layer and a bottom organic layer were obtained. The phases were separated by decantation.

Four lignin types (KL1-SW, KL2-SW, OS1-SW, and SL-W) were pyrolyzed at different conditions (see Table 4) using a fully automated bubbling fluidized bed reactor. A solid feed rate of 10 g/min was applied. To specify in more detail KL1.1-SW and OS1-SW were pyrolyzed at 450°C, KL1.2-SW and SL-W at 550°C. KL1.3-SW was also pyrolyzed at 550°C with the additional presence of hydrogen at a ratio of 10%. The gas was bubbled (1 kg/h) at atmospheric pressure. The hot lignin pyrolysis vapours were first filtered at high temperature to remove fine particulate material (char and ash). Subesequenly, the hot pyrolysis vapours were quenched in 2 sequential 0°C impingers, each containing 250 ml of ice-chilled water. (Fig. 3). Most of the reaction water and part of the organic degradatives are condensed in these two impingers. Organic degradatives in aerosol form are not captured but precipitated / condensed in a downstream located electrostatic precipitator (ESP). Finally, low boiling organics such as methanol, acetone and acetic acid are trapped in the freeze condenser downstream the ESP. As a result, two-phase liquid products with an upper aqueous layer and a bottom organic layer were obtained. The phases were separated by decantation.

In accordance with De Wild P.J., Huijgen W.J., Gosselink R.J. Lignin pyrolysis for profitable lignocellulosic biorefineries. Biofuels Bioprod. Bioref. 8, (2014) 645-657, pyrolysing of lignin yielded pyrolysis vapours. These vapours of mainly gases and aerosols that -upon condensing- resulted in approximately 19% acquatic phase and 24%organic phase based on the amount of lignin which was pyrolised.

The acquatic phase was collected in the two impingers (optionally comprising an aqueous phase before the vapours were introduced). The organic phase was partially collected in the two impingers and partially in the downstream located ESP. This because part of the lignin-derived organics are in the form of aerosols that are not effectively extracted / collected in the water-filled impingers.

After fast pyrolysis, among others an acquatic and an organic phase are formed from lignin (added new Table 4). In the organic phase most catechol is solved (Table 4). The idea was to extract catechol from both phases, by blowing the pyrolysis gas immediately through water after thermochemical depolymerization. However, ECN did not provide a water solution, as intended, for the pyrolysis gas (Table 4). As a result, it is not clear whether the additional presence of water acts as an extraction solution for catechol in case it can also be solved in the organic phase. However, in view of the good property of water to solve catechol, it is most likely possible. Catechol can be solved up to 451 g L⁻¹ at 20°.

When sample KL1.2-SW is examined, the concentration of catechol in the acquatic phase can be raised theoretically asymptotically from 21 g L⁻¹ catechol (from solely the acquatic phase) to 75 g L⁻¹ (from the acquatic and organic phase) (Added figure). Increased concentration of catechol reduces energy requirements for further processing. Because of the simulated initial presence of 200 ml of water, the concentration of catechol increases as more lignin is processed. Over time, the volume of the hydrophobic and hydrophilic phase also increase the total volume. Based on the simulation carried out, it is therefore estimated that a ratio of 0,5 to 3,2 g of aqueous phase per g of lignin is reached. If needed, the ratio of the aqueous phase to lignin can be increased further when the process is continued. To increase the effect, possibly it would be good to remove the organic phase during the process by decantation. Based on the data obtained a maximum yield for catechol of 3.7% can be calculated for KL1.2-SW present in the hydrophilic and hydrophobic phase.

The processed lignin varies between 140 to 450 g (Table 4).

**Table 4.**

| Lignin Type | Amount of lignin fed to the reactor (g) | Pyrolysis at (°C) | Fluidization Gas | Amount of organic phase (g) | Catechol conc in organic phase (mM) | Amount of aqueous phase (g) |
|---|---|---|---|---|---|---|
| KL1.1-SW | 140 | 450°C | N₂ | 22,4 | 385,07 | 13 |
| KL1.2-SW | 450 | 550°C | N₂ | 82,5 | 486,79 | 87,1 |
| KL1.3-SW | 450 | 550°C | N₂/H₂ | 69,2 | 600,31 | 83,2 |
| KL2.1-SW | 450 | 550°C | N₂ | 76,1 | 452,27 | 96,4 |
| OS1.1-SW | 140 | 450°C | N₂ | 26,6 | 413,22 | 25,2 |
| SL1.1-W | 140 | 450°C | N₂ | 23,8 | 236,13 | 29,4 |

| | | Concentration in aqueous phase (mM) (before/ after steam bath distillation) | | | | |
|---|---|---|---|---|---|---|
| | | Catechol | Phenol | Guaiacol | *m, p*-Cresol | *o*-Cresol |
| KL1.1-SW | | 78.20/5.69 | 12.53/0.00 | 8.81/0.12 | 2.84/0.04 | 7.90/0.01 |
| KL1.2-SW | | 185.31/5.90 | 6.41/1.98 | 1.97/0.02 | 2.53/0.04 | 23.69/0.78 |
| KL1.3-SW | | 183.54/6.92 | 112.63/3.36 | 1.32/0.00 | 9.07/0.07 | 24.27/0.66 |
| KL2.1-SW | | 116.85/6.30 | 3.86/0.03 | 14.33/0.00 | 6.35/0.00 | 13.01/0.07 |
| OS1.1-SW | | 176.01/5.57 | 102.10/2.21 | 4.80/0.28 | 2.84/0.03 | 8.83/0.25 |
| SL1.1-W | | 44.87/4.87 | 22.82/2.24 | 5.29/0.95 | 4.92/0.11 | 2.19/0.16 |

Because catechol can easily be extracted by water based on its solubility, it was possible to concentrate this compound. Besides the two impingers, the fluidized bed reactor was furthermore equipped with a cyclone as a char knock-out pot (KO-pot) before the recovery of the liquid product, and afterwards an electrostatic precipitator (ESP) to collect aerosol vapors and a freeze condenser (FC) to obtain the condensed fraction. Non-condensable pyrolysis gases (merely CO, CO₂ and CH₄) were monitored on-line with a micro-GC and ABB NDIR monitor set-up. The FC fraction was an aqueous liquid with a very pungent smell. The ESP fraction was a homogeneous and rather viscous liquid with a smoky smell. The combined aqueous phases, containing increased concentrations of catechol from the two impingers was used for further biochemical processing.

### Steam bath distillation

For steam bath distillation, 1 mL of the aqueous phase of the pyrolysis oil was diluted with 50 mL MQ-water. This was filled in a 500 mL round-bottomed flask with boiling granules. The flask was placed in an oil bath that was heated to 120°C for 2 hours. The steam for the distillation process was generated by boiling water in a 5 L flask. The distillate was cooled down in a 1 L flask. The residue of the pyrolysis oil from the 500 mL flask was transferred in an argon-purged bottle and stored at -20°C.

### Example 1: Strain development and cultivation conditions

### Strain and cultivation conditions

The bacterial strains used in this study are listed in Table 1. Unless otherwise stated bacteria were usually grown in LB (10 g/l tryptone, 5.0 g/l yeast extract, 5 g/l NaCl, dissolve in H₂O and autoclave). Batch cultivations were done in Erlenmeyer flasks that were shaken at 200 rpm. *Escherichia coli* cells were grown at 37°C while *Pseudomonas putida* was cultured at 30°C. Selection of *P. putida* cells was performed by plating onto M9 minimal medium with citrate (2 g/L) as a sole carbon source. The following four stock solutions were prepared and autoclaved separately: 10× stock solution of M9: weight 42.5 g Na₂HPO₄ 2H₂O, 15 g KH₂PO₄, 2.5 g NaCl and 5 g NH₄Cl and dissolve in 500 ml of H₂O, 120.37 g/L MgSO₄, 200 g/L citrate (as selective carbon source for *Pseudomonas*), and an 16 g/L agar solution. The components were diluted in sterile water to final concentrations of 1× M9 salts, 0.24 g/L MgSO₄, 20 g/l citrate and where required, 14 g/L agar. If needed, additionally antibiotics were added at the following final concentration: ampicillin (Amp) 100 µg/ml for *E. coli* cells and at 500 µg/ml for *P. putida,* kanamycin (Km) 50 µg/ml. Other supplements were added in following concentrations: 5-bromo-4-chloro-3-indolyl-β-D-galactopyranoside (X-gal) 80 µg/ml; isopropyl-β-D-1-thiogalactopyrano side (IPTG) 0.48 g/L; 3-methyl-benzoate (3MB) 2.25 g/L; 102.69 g/L sucrose. The cultivation of *P. putida* or *E. coli* cells were monitored during growth by OD₆₀₀ using UV-1600PC Spectrophotometers (Radnor, Pennsylvania, USA).

### Strain development and Cloning Targeting Sequence into pEMG

The pEMG plasmid was used to perform modifications in the genome of P. putida KT2440. The procedure is based on the homologous recombination forced by double-strand breaks in the genome of the *P. putida* after cleavage in vivo by I-Scel. (encoded on the plasmid pSW-I). Transient expression of the nuclease is controlled in pSW-I by Pm, a promoter induced in presence of 3-methylbenzoate-inducible.

The deletion of catB/catC and pcaB (KT2440 JD2S and BN14, respectively), the integration of Pem7, Pem7* upstream of catA (BN6 an BN12, respectively), catA2 downstream of Pcat:catA (BN15) and a copy of Pcat:catA:catA2 (BN18 and BN19) in P. putida were performed one after another as follows:

For the genetic modifications of P. putida KT2440 JD1 and JD2S, KT2440 BN6-BN19 (table 1) the upstream (TS1) and downstream (TS2) regions flanking the region to be deleted/inserted and the insertion Pem7, Pem7*, catA2 and Pcat: catA: catA2 were amplified separately using Phusion High-Fidelity Polymerase (Thermo Fisher) (Primer listed in table 3). For the deletion of ΔcatB/catC and pcaB, the resulting products (TS1 and TS2) were joined together by using Gibson assemply. For the deletion of ΔcatB/catC and pcaB, the resulting products (TS1 and TS2) were joined together by using Gibson assemply. The fused fragment was ligated into Smal linearized plasmid pEMG, resulting in pEMG-ΔcatB/catC and pEMG-ΔpcaB -for the construction of KT2440 JD2S and KT2440 BN14. For the insertion of Pem7 and Pem7* upsteam of catA, catA2 downstream of catA and the copy of Pcat: catA: catA2, TS1, TS2 and the to be inserted fragments were ligated into Smal digested pEMG via Gibson assembly. Each of the resulting plasmids pEMG-ΔcatB/catC, pEMG-ΔpcaB, pEMG-Pem7, pEMG-Pem7*, pEMG-catA2 and pEMG-Pcat:catA:catA2 were transformed separately into E. coli DH5αλpir via electroporation and the culture was plated onto LB-Km plates supplemented with Xgal and IPTG to discriminate potential positive clones by visual screening. Putative positive clones were checked for the presence of the TS1/TS2 insertions by colony PCR using pEMG-F/pEMG-R (see table 3) and confirmed by sequencing the corresponding plasmids. The pEMG derivates were isolated form *E. coli* DH5αλ*pir* with Miniprep Kit (Quiagen) and transformed into *E. coli* CC118I*pir* for the delivery into P. *putida* KT2440 via mating as described in (de Lorenzo and Timmis, Methods Enzymol., 1994; 235:386-405; Martinez-Garcia and de Lorenzo, Environ Microbiol., 2011, 13(10):2702-16).

The bacterial mixtures were resuspended in 10 mM MgSO₄ and appropriate dilutions plated onto M9 citrate plus kanamycin. Since pEMG-derived plasmids cannot proliferate in *P. putida,* KmR clones raised after conjugation can grow only by co-integration of the construct in the genome of the recipient strain. The delivery of the pSW-I plasmid into competent *P. putida* was done by electroporation of 50 ng of pSW-I in the Km resistant cells as described in (Martinez-Garcia and de Lorenzo, Environ Microbiol., 2011, 13(10):2702-16) and plated onto LB-Km 50 µg/ml + Amp 500 µg/ml. The induction of the I-Scel enzyme in cointegrated clones that harbors the pSW-I plasmid was started by adding 15 mM 3-methylbenzoate in a 5 ml LB-Amp medium. The culture was incubated for 14 h at 30 °C and plated on LB-Amp 500 plates. The loss of cointegrated plasmid were checked by selecting kanamycin sensitive clones on LB-Kan 50 µg/ml plates. Deletions and insertions into the genome were generally confirmed by PCR with primer that hybridize upstream of TS1-F and downstream of TS2-R in the genome. The curation of pSW-I from P. putida was achieved by several passages of the deleted clone in LB without antibiotics.

### Strain development and Cloning Targeting Sequence into pJNNmod

The pJNNmod plasmid was used for the episomal expression of *catA* under control of PGro and PGro_2 in *P. putida* BN15. The construction of pJNNmod-PGro:catA and pJNNmod-PGro_2:catA leading to BN20 and BN21, respectively, was performed as follows:

For the genetic modifications of BN20 and BN21 (table 1) *catA* and the promoter PGro and PGro_2 were amplified separately using Phusion High-Fidelity Polymerase (Thermo Fisher) (Primer catA-F and catA-F: *catA* : TS1_catB/C-F/TS1_catB/C-R; PGro-F and PGro-R: PGro and PGro_2; listed in table 3).For the insertion of *catA* with each promoter (PGro and PFro_2) in the plasmid pJNNmod, the fragments were ligated into Smal digested PJNNmod via Gibson assembly. Each of the three resulting plasmids pJNNmod-Gro:catA and pJNNmod-Gro_2:catA were transformed separately into *E. coli* DH5αλ*pir* via electroporation and the culture was plated onto LB-Amp plates. Putative positive clones were checked for the presence of the promoter/catA insertions by colony PCR using pJNNmod-F/pJNNmod-R (see table 3) and confirmed by sequencing the corresponding plasmids (plasmids see table 2).

**Table 1. Strains**

| **Strain** | **Description/relevant characteristics** | **Reference** |
|---|---|---|
| *E. coli* | | |
| DH5α | *supE44, ΔlacU169 (ϕ80 lacZΔM15), hsdR17 (rk-mk+), recA1, endA1, thi1, gyrA, relA* | Hanahan and Meselson (Methods Enzymol. 1983; 100:333-42) |
| DH5αλpir | *λpir* lysogen of DH5α | Martinez-Garcia and de Lorenzo (Environ Microbiol. 2011; 13(10):2702-16) |
| CC118 | Δ(*ara-leu*), *araD,* Δ*lac*X174, *galE, galK, phoA, thi1, rpsE, rpoB, argE (Am), recA1,* lysogenic λ*pir* | de Lorenzo and Timmis (Methods Enzymol. 1994; 235:386-405) |
| HB101 | SmR, *hsdR-M+, pro, leu, thi, recA* | Sambrook et al. (Molecular cloning. A laboratory manual, 2nd Ed. New York: Cold spring harbor laboratory press, 1989) |
| *P. putida* | | |
| KT2440 | mt-2 derivative cured of the TOL plasmid pWW0 | Bagdasarian et al. (Gene. 1981 ,16(1-3):237-47) |
| JD2S | KT2440 Δ*catB*/*C* | unpublished |
| BN6 | KT2440 Δ*catB*/*C* Pem7:catA | This application |
| JD1 | KT2440 Δ*catR* | Van Duuren et al. (J Biotechnol. 2011;156(3):163-72) |
| BN12 | KT2440 Δ*catB*/*C* Pem7*:catA | unpublished |
| BN14 | KT2440 Δ*catB*/*C* Δ*pcaB* Pem7:catA | unpublished |
| BN15 | KT2440 Δ*catB*/*C* Pcat:*catA:catA2* | unpublished |
| BN18 | JD2 Pcat:*catA:catA2* | unpublished |
| BN19 | BN15 Pcat:catA:catA2 | unpublished |
| BN20 | pJNNmod-PGro:catA | unpublished |
| BN21 | PJNNmod-PGro_2:*catA* | unpublished |

**Table 2. Plamids**

| **Plamids** | **Genome** | **Reference** |
|---|---|---|
| pSW-I | ApR, oriRK2, xylS, Pmlscel (transcriptional fusion of I-scel to Pm) | Wong and Mekalanos (Proc Natl Acad Sci USA., 2000; 97(18):10191-6) |
| pEMG | KmR, *ori*R6K, *lacZ*α with two flanking I-Scel sites | Martinez-Garcia and de Lorenzo (Environ Microbiol. 2011; 13(10):2702-16) |
| pRK600 | CmR; oriColE1, RK2 mob+, tra+ | de Lorenzo and Timmis (Methods Enzymol. 1994; 235:386-405) |
| pSEVA247C | neo,R, pRO1600/ColE1, CFP | Silva-Rocha et al. (Nucleic Acids Res. 2013 Jan; 41) |
| pSEVA247R | neo,R, pRO1600/ColE1, RFP | Silva-Rocha et al. (Nucleic Acids Res. 2013 Jan; 41) |
| pJNNmod | P_{TAC}, *laclq,* ColE1 origin of replication | Rodrigues et al. (Metab Eng. 2013; 20:29-41) |

*

**Table 3**

| **Primer** | **Sequence** | **Function** | **SEQ ID NO:** |
|---|---|---|---|
| pEMG-F | CCATTCAGGCTGCGCAACTGTTG | Vector primer pEMG | 37 |
| pEMG-R | CTTTACACTTTATGCTTCCGGC | Vector primer pEMG | 38 |
| pSW-F | GGACGCTTCGCTGAAAACTA | Check of plasmid curation | 39 |
| pSW-R | AACGTCGTGACTGGGAAAAC | Check of plasmid curation | 40 |
| Check-F | GGCACATCGAACACGCTGTAGTTG | Confirm *catB*/*C* deletion | 41 |
| Check-R | CCTCCAGGGTATGGTGGGAGATTC | Confirm *catB*/*C* deletion | 42 |
| TS1_catB/C-F | TGAACGCTTCGCCAGCCAACT ACCTTCGCCAGCC | Amplification TS1 *catB*/*C* | 43 |
| TS1_catB/C-R | GCTCGATACCCAGGCCAGCAGGCCA GCA | Amplification TS1 *catB*/*C* | 44 |
| TS2_catB/C-F | CATATGTGTTGCCAGGTCCCGTCAGG TC | Amplification TS2 *catB*/*C* | 45 |
| TS2_catB/C-R | AAAAACATATGCAGCTCAAGGCCGAC GAAAAGG | Amplification TS2 *catB*/*C* | 46 |
| TS1_Pem7-F | TGAATTCGAGCTCGGTACCCTGGGC GATGTGCAGCAGCTC | Amplification TS1 Pem7 | 47 |
| TS1_Pem7-R | CGATGATTAATTGTCAACAACGTGCTT ACCTCGTATTGTTC | Amplification TS1 Pem7 | 48 |
| TS2_Pem7-F | TTAAAGAGGAGAAATTAAGCATGACC GTGAAAATTTCCCACA | Amplification TS2 Pem7 | 49 |
| TS2_Pem7-R | GTCGACTCTAGAGGATCCCCTCGAAG TACGAATAGGTGCCC | Amplification TS2 Pem7 | 50 |
| Pem7-F | GCCTGACAAGAACAATACGAGGTAAG CACGTTGTTGACAATTAATCATCGG | Amplification of Pem7 | 51 |
| Pem7-R | GTCGGCAGTGTGGGAAATTTTCACGG TCATGCTTAATTTCTCCTCTTTAACCT AGGG | Amplification of Pem7 | 52 |
| Ptuf_s-F | CAAGCTTAGGAGGAAAAACAAACTGG AAGCGGTGTCAAAG | Amplification short Ptuf | 56 |
| Ptuf_s-R | TCCTCGCCCTTGCTCACCATGCTTAA TTTCTCCTCTTTGTGGCCGGCATTCTA TTTGTC | Amplification short Ptuf | 57 |
| Ptuf_sM-F | AACTGGAAGCGGTGTCAAAGC | Mutagenesis short Ptuf | 58 |
| Ptuf_sM-R | GTGGCCGGCATTCTATTTG | Mutagenesis short Ptuf | 59 |
| Ptuf-F | CAAGCTTAGGAGGAAAAACACCGCTT CACAGGGAACACCA | Amplification short Ptuf | 60 |
| Ptuf-R | CCTCGCCCTTGCTCACCATCGATACA ATCCTCCGCAGAAG | Amplification short Ptuf | 61 |
| Ptuf_M-F | CCGCTTCACAGGGAACAC | Mutagenesis Ptuf | 62 |
| Ptuf_M-R | CGATACAATCCTCCGCAGAAG | Mutagenesis Ptuf | 63 |
| PGro-F | CAAGCTTAGGAGGAAAAACAGAAGGA CCGGGGCCGCGCAA | Amplification of PGroES | 64 |
| Pgro-F | TCCTCGCCCTTGCTCACCATTGTCGA TCTCTCCCAAATTG | Amplification of PGroES | 65 |
| TS1_pcaB-F | TGAATTCGAGCTCGGTACCCACACCG CGGGCATGACCGCC | Amplification TS1 for pcaB deletion (BN14) | 66 |
| TS1_pcaB-R | GTGCGCCACAGCGGTCTCCTGCAGC GTCCTTAATCATCAT | Amplification TS1 for pcaB deletion (BN14) | 67 |
| TS2_pcaB-F | ATGATGATTAAGGACGCTGCAGGAGA CCGCTGTGGCGCAC | Amplification TS2 for pcaB deletion (BN14) | 68 |
| TS2_pcaB-R | GTCGACTCTAGAGGATCCCCCTGGG CAAAGCCCGGGGTGA | Amplification TS2 for pcaB deletion (BN14) | 69 |
| TS1_catA2-F | TAATCTGAATTCGAGCTCGGTACCCC GTTGGCCGGTGCCACCGTC | Amplification TS1 catA2 Integration (BN15) | 70 |
| TS1_catA2-R | GTTCACGGTCATGCTTAATTTCTCCTC TTTTCAGCCCTCCTGCAACGCCC | Amplification TS1 catA2 Integration (BN15) | 71 |
| TS2_catA2-F | GTTCGAGGTTATGTCACTGT | Amplification TS2 catA2 Integration (BN15) | 72 |
| TS2_catA2-R | TGCAGGTCGACTCTAGAGGATCCCC GGCGGGCAGATCCTGTGCGTAG | Amplification TS2 catA2 Integration (BN15) | 73 |
| CatA2-F | GGGCTGAAAAGAGGAGAAATTAAGCA TGACCGTGAACATTTCCCA | Amplification catA2 (BN15) | 74 |
| CatA2-R | AAATCACAGTGACATAACCTCGAACT CAGGCCTCCTGCAAAGCTC | Amplification catA2 (BN15) | 75 |
| TS1_Pcat:catA /2-F | TAATCTGAATTCGAGCTCGGTACCCC GCGCCTGAACGCCGGGCAG | Amplification TS1 for Pcat:catA:catA2 Integration (BN18/19) | 76 |
| TS1_Pcat:catA /2-R | TCTCCCACCATACCCTGGAGGTCTGA CACACCATGCCCACAGGGG | Amplification TS1 for Pcat:catA:catA2 Integration (BN18/19) | 77 |
| TS2_Pcat:catA | GCCGCGAGCTTTGCAGGAGGCCTGA | Amplification TS2 for Pcat:catA:catA2 | 78 |
| /2-F | TCATATGGCCTGTTGCTCGA | Integration (BN18/19) | |
| TS2_Pcat:catA /2-R | TGCAGGTCGACTCTAGAGGATCCCCT GACCACCTTGCAACAGGTG | Amplification TS2 for Pcat:catA:catA2 Integration (BN18/19) | 79 |
| Pcat:catA/2-F | CAGACCTCCAGGGTATGGTG | Amplification of Pem7 | 80 |
| Pcat:catA/2-R | TCAGGCCTCCTGCAAAGCTC | Amplification of Pem7 | 81 |
| catA-F | GTCGACTCTAGAGGATCCCCTCAGCC CTCCTGCAACGCCC | Amplification of catA (BN20/21) | 82 |
| catA-R | ATGACCGTGAAAATTTCCCA | Amplification of catA (BN20/21) | 83 |
| PGro-F | ATATGTCGAGCTCGGTACCCGAAGGA CCGGGGCCGCGCAA | Amplification of PGro and PGro_2 (BN20/21) | 84 |
| PGro-R | TGGGAAATTTTCACGGTCATTGTCGA TCTCTCCCAAATTG | Amplification of PGro and PGro_2 (BN20/21) | 85 |
| pJNN-F | CGCGAATTGCAAGCTGATCC | Check primer forward | 86 |
| PJNN-R | CTCTCATCCGCCAAAACAGC | Check primer reverse | 87 |

| **Construct** | **SEQ ID NO:** |
|---|---|
| pEMG-ΔcatB catC | **53** |
| pEMG-ΔpcaB | **54** |
| pEMG-pEM7 | **55** |

For the modified strains, especially strain BN6, the deletion of *catB*/*catC* (KT2440 JD2S) and the integration of Pem7 upstream of *catA* in *P. putida* (KT2440 BN6) were performed one after another. For the genetic modifications of *P. putida* KT2440 JD2S and KT2440 BN6 (table 1) the upstream (TS1) and downstream (TS2) regions, flanking the region to be deleted and inserted, respectively, and the insertion Pem7 were amplified separately using Phusion High-Fidelity Polymerase (Thermo Fisher). The following primers were used: for ΔcatB/C: TS1_catB/C-F/ TS1_catB/C-R and TS2_catB/C-F/ TS2_catB/C-R and for the integration of Pem7: TS1_Pem7-F/ TS1_Pem7-R and TS2_Pem7-F/ TS2_Pem7-R; and Pem7- F/ Pem7-R (table 3). First, the pEMG plasmid was linearized with the endonuclease Smal. For the deletion of Δ*catB*/*catC*, the products (TS1 and TS2) were fused and ligated into the restriction site Smal, via the Gibson assembly. Equally, for the insertion of Pem7 upstream of *catA,* TS1, TS2 and the promoter to be inserted (Pem7) were fused followed by a ligation into Smal digested pEMG via Gibson assembly. Each of the two resulting plasmids pEMG-Δ*catB*/*catC* or pEMG-Pem7 were transformed separately into *E*. *coli* DH5αλpir via electroporation and the culture was plated onto LB-Km plates supplemented with Xgal and IPTG to discriminate potential positive clones by visual screening. Putative positive clones were checked for the presence of the TS1/TS2 insertions by colony PCR using pEMG-F/pEMG-R and confirmed by sequencing the corresponding plasmids.

The pEMG derivates were isolated form *E. coli* DH5αλpir with the Miniprep Kit (Quiagen) and transformed into *E. coli* CC118λpir. The latter strain was used for the delivery of the plasmid into P. *putida* KT2440 by conjugation based on triparental mating supported by *E. coli* HB101 (pRK600) as described in de Lorenzo and Timmis, 1994 and Martinez-Garcia and de Lorenzo, 2011. The bacterial mixtures were resuspended in 10 mM MgSO₄ and appropriate dilutions of the pellet were plated onto M9 citrate (as selective carbon source for *Pseudomonas*) plus kanamycin. Since pEMG-derived plasmids cannot proliferate in *P. putida,* Km^{R} clones raised after conjugation can grow only by co-integration of the construct in the genome of the recipient strain. The delivery of the pSW-I plasmid into competent *P. putida* was done by electroporation of 50 ng of pSW-I in the Km resistant cells and plated onto LB-Km + Amp. The sucrose solution was used as wash/electroporation buffer during the electroporation. The presence of the plasmid was confirmed by PCR using pSW-F/pSW-R. The induction of the I-Scel enzyme in cointegrated clones that harbours the pSW-I plasmid was started by adding 15 mM 3-methylbenzoate in a 5 mL LB-Amp medium. The culture was incubated for 14 h. at 30 °C and plated on LB-Amp plates. The loss of cointegrated plasmid were checked by selecting kanamycin sensitive clones on LB-Kan plates. The deletion of *catB*/*catC* in *P. putida* KT2440 (JD2S) and insertion of Pem7 in KT2440 BN6 was confirmed by PCR using Check-F/ Check-R and TS1-Pem7-F/ TS2-Pem7-R, respectively. The curation of pSW-I from *P. putida* JD2S was achieved by several passages in LB without antibiotics.

*Pseudomonas putida* BN6 was grown on glucose (10mM) and E-2 mineral medium (Hartmans *et al.,* 1989) in the presence of the aqueous phase of the pyrolysis oil from the described technical lignins. The strains were grown with a diluted catechol concentration of 1.5 mM from the different samples. Under the same conditions the strain was additionally grown in the presence of purified catechol from steam bath distillation. The cultivation took place in 48 well flower plates in the BioLector (m2p-laps, Germany). Every strain was tested in triplicate. From an agar plate a two sequential pre-culture was inoculated, before the cultivation was started. The cultivation temperature was 30 °C, with a humidity of 85 %, and the shaking frequency was set at 1300 rpm. The cultivations lasted for about 24 hours.

### Application of promoter with higher activity to increase the catechol conversion rates in P. putida

Increased production performance of *P. putida* production strains caused by a promoter upstream of catA with increased promoter activity could be demonstrated in P. putida BN6 (SEQ ID No. 5 [Pem7]) with a conversion rate of 5.5 mmol g-1 h-1 versus BN12 (SEQ ID No. 5 [Pem7*] with a conversion rate of 7.11 mmol g-1 h-1. Hence, Pem7* can be applied as heterologous promoter in P. putida to express genes like catA at a high level leading to an significant increase in the catechol conversion rate compared to the original promoter (see figure 2).

### Production of cis-cis muconic acid

*P. putida* KT2440 JD2S was grown at 30°C and pH 7. From a frozen stock at -80°C first a batch cultivation of 5 mL was grown in LB for 22 h. Subsequently, 1.33 mL was used to inoculate a 60 mL batch cultivation with E-2 mineral medium (Hartmans S, Smits JP, van der Werf MJ, Volkering F, de Bont JAM. Appl Eniviron Microbiol 55, 2850-2855 (1989)) and 10 mM glucose. The cultivation was grown for 26 h. 14 mL of the second cultivation was used to inoculate a 660 mL batch cultivation of a similar medium with the additional presence of 1 mM benzoate. The latter culture was grown for 23 h and sequentially used as pre-culture for the fermentation. The Erlenmeyer flasks of the batch cultivations were shaken at 100 rpm. A pH-stat fed-batch cultivation was performed in a 72L bioreactor (Braun Biostat UD, Germany) with a start working volume of 30 L in a similar medium as the pre-culture without the presence of benzoate. After the addition of the pre-culture the oxygen saturation was kept at >50% in the culture by sparging with air at a rate of 15-25 L min⁻¹ and an adjusted stirring speed. The pH was regulated with 20% NaOH during the batch culture phase. After 16 h the fed-batch phase was started by the addition of 1 mM benzoate in combination with the feeding pattern of E-2 mineral medium with 1.4 M glucose and 72 g L⁻¹ ammonium sulfite. A biomass formation rate of 0.04 h⁻¹ was installed. Since the maintenance factor increases during the process and to ensure growth its value was increased with exponent 0.02 per hour (van Duuren JBJH, Puchalka J, Mars AE, Buecker R, Eggink G, Wittmann C, Martins dos Santon VAP. BMC Biotechnol 13, 93 (2014)). During the operation as a pH-stat, the addition of benzoate was coupled to the pH-regulation by titrating with a solution containing E-2 mineral medium with 1.2 M benzoic acid and 2.4 M sodium hydroxide (van Duuren J.B.J.H., Wijte D., Karge B., Martins dos Santos V.A.P., Yang Y., Mars A.E., Eggink G. Biotechnol Prog 28 (2012)). The physiologic activity of the bacterial cells could be followed accurately by the stirrer speed, which was maximum after 70 h (Fig. 3). The dry cell weight increased at an average rate of 0.04 h⁻¹. After 78 h the addition of benzoate was stopped. The last 9 hours of the fermentation the pH was again corrected with 20% NaOH to avoid the presence of benzoate in the medium. To reduce the resource demands the feeding rate of glucose was coherently reduced to the linear rate of 19.84 mL h⁻¹. To control foam in the reactor at a low level, in total 85 mL Antifoam 204 (Sigma-Aldrich, USA) was added to the medium regulated by a measurement probe. Based on the described process after 87 h a broth of 42.5 L with 1695 g *cis, cis*-muconic acid was obtained. The cells were separated from the medium by microfiltration. For this process step at 1.5 bar and 15°C a UFI-TEC cross-flow micro-filtration system (UFI-TEC, Germany) equipped with 4*TAMI 0.2 µm membranes (TAMI Industries, France) was used at a duration of 1.75 h. The biomass was concentrated in a supernatant of 4.3 L, which was discarded. In a next step, 2.1 L 32% HCl was added to 38.2 L medium. By reducing the pH-value below the second pKa value of the dissolved *cis,* cis-muconic acid at a pH of 2.7, the compound becomes very insoluble in water (Table 5). 1451.6 g of *cis, cis-*muconic acid could be acquired by overnight precipitation. 1.11 g of *cis*, cis-muconic acid stayed in solution. The solid *cis*, *cis*-muconic acid was sequentially washed with 45L and 20L water. Besides impurities in the water streams of 45 L and 21 L, 16.65 g and 8.45 g *cis*, *cis-*muconic acid was dissolved, respectively. From the remaining 17.1 L *cis, cis*-muconic acid suspension the compound was dried by spry drying for 9.38 h with 50°C air and a 120°C surface. In total 1153 g *cis*, *cis*-muconic acid was obtained. The purity of the white powder was determined (Table 5).

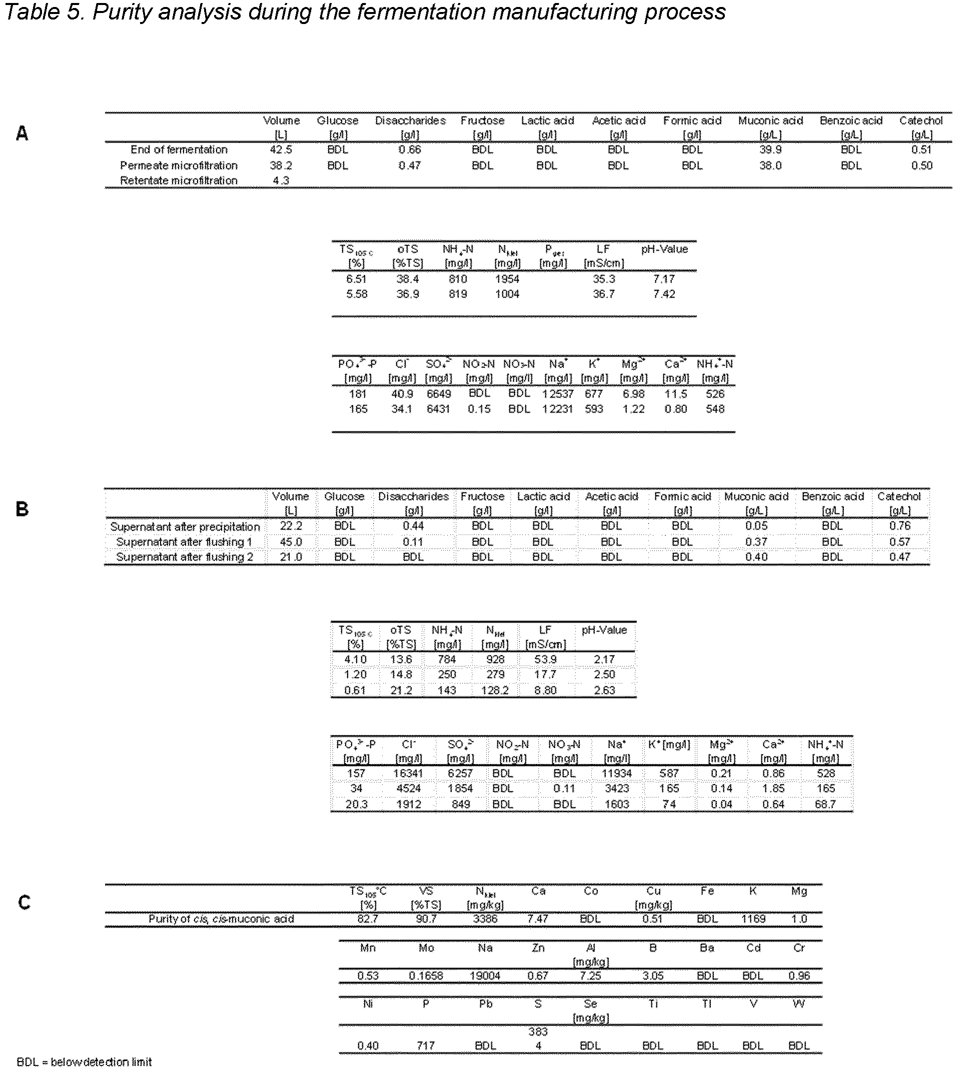

### Sample analysis

To determine the concentrations of catechol, phenol, guaiacol and cresol all solutions were analyzed via UHPLC (Agilent Technologies, USA). The flow rate of eluent A (0.025% H₃PO₄ in water) and B (acetonitrile) were 1 ml/min and the oven temperature was set to 25 °C. A Merck Purospher STAR RP-18 column (Merck Millipore, USA) was used for separation and a DA-detector (G4212A, Agilent Technologies) for quantification. The aromatic compounds were quantified at 210 nm, *cis,* cis-muconic acid at 260 nm. In addition, aliquots of the pyrolysis water from the lignin pyrolysis were dissolved in iso-propanol and subsequently analyzed with gas chromatography coupled with mass spectrometric detection (GC-MS), using an internal standard in combination with calibration standards. The internal standard contained deuterated ethylbenzene, resorcinol and phenol. The GC/MS method was developed for the analysis of isopropanol (IPA) dissolved polar compounds from the thermal conversion of biomass via split injection (split ratio 1:25, injector temperature 275°C) on a gas chromatographic separation column (40°C → 245°C, ramp 10°C/min, He carrier gas flow 1 ml/min). The equipment consists of a TRACE GC ULTRA/ DSQII gas chromatograph with Xcalibur data processing software. The WAX column was a Zebron ZB-WAX plus or a ZB-WAX (length of 30 m, internal diameter 0.25 mm) with a Hydroguard precolumn (internal diameter 0.53 mm). The film thickness of the stationary phase was 0.25 µm. Further details can be found in de Wild *et al.,* 2009 and de Wild *et al.,* 2011.

### Conclusion

To reduce the heterogeneity and recalcitrance of lignin for commercial application, pyrolysis, especially the LIBRA pyrolysis, process is a promising procedure. In this research it was shown that based on first phase pyrolysis oil separation into an aqueous and organic phase by the addition of water (i.e. direct introduction of pyrolysis vapors into water before the vapors condense) it becomes possible to valorize the extracted hydrophilic aromatic catechol based on an integrated biochemical process.

Catechol is a secondary thermal conversion product of guaiacol (Lawson and Klein, 1985; Townsend *et al.,* 1988). A high guaiacol and catechol yield is a strong indication of the potential of the particular lignin for its conversion into catechol-rich lignin pyrolysis oil. Four lignins OS1-SW, OS2-HW, OS3-W, and KL1-SW were characterized by analytical pyrolysis with GC separation and mass spectrometric detection to estimate per lignin the relative yield of guaiacol and catechol. Approximately 0.5 mg of each of the lignins was pyrolyzed at 500°C. Based on the GC-MS results it was defined that coniferous lignins provide the highest relative yields of guaiacol and catechol. This is in concordant with the fact that coniferous lignin almost exclusively consists of interlinked guaiacyl moieties, in contrast to deciduous and herbaceous lignins. When the processed lignins are compared, based on the obtained GC-MS spectrums, coniferous lignin is the most promising lignin, followed by herbaceous and deciduous lignin to obtain increased concentrations of catechol.

On the basis of the LIBRA lignin pyrolysis process in combination with its downstream as shown in Figure 2 the various fractions were obtained. The obtained acquatic phase of KL1.2-SW and KL2.1-SW were analyzed with GC-MS. The following substances were present in the aqueous phase from KL1.2-SW and absent in the aqueous phase from KL2.1-SW: methylacetate, hydroxyacetaldehyde, furfural, 2(5H)furanon, 5-hydroxymethyl furfural (5-HMF), 4-hydroxybenzaldehyde, 4-hydroxyacetophenone, resorcinol and levoglucosan. Except for the methylacetate, benzaldehyde and the acetophenone these chemicals in general are pyrolysis products from the thermochemical degradation of carbohydrates. This indicates that the KL1-SW contains more carbohydrate impurities when compared to the KL2-SW. Especially levoglucosan and furfural are a strong indicator (Greenhalf *et al.,* 2012).

Jiang *et al.* described that Furfural and 5-HMF are inhibitors in fermentation processes. Because from KL2.1-SW the most preferred acquatic phase could be obtained, which was solely directly suitable for biochemical conversion by a metabolically engineered *P. putida* KT2440 BN6 strain, steam batch distillation was performed to produce a purified aqueous residue fraction with all samples. Based on HPLC measurements it was shown that steam bath distillation is an appropriate method to fractionize the acquatic phase from the (fast) pyrolysis process with lignin. Besides catechol phenol was simultaneously purified by boiling the sample at 120°C. Of all processed samples, the main compounds that accumulated at an increased concentration in the acquatic phase were catechol, phenol, guaiacol and cresol (Table 4). The molar presence of all four aromatics were measured before and after distillation. Catechol is a key-component in the metabolism of *P. putida* KT2440 BN6. Since the acquatic phase is considered a waste product and because it can easily be decanted from the organic phase, the developed route is regarded cost-effective for industrial application. The other three aromatic compounds are described to be a precursor for catechol when converted metabolically. Based on metabolic engineering it can therefore be expected that the total yield can be increased in future processes (Vardon *et al.,* 2015, Jiménez *et al.,* 2002, Kali *et al.,* 2002, Shingler *et al.,* 1989, Nordlund *et al.,* 1990, Pavel *et al.,* 1994).

As a next step, the solubilized catechol in the water solution was converted into *cis,* cis-muconic acid by the metabolically engineered strain *P. putida* KT2440 BN6. As earlier described, *cis, cis*-muconic acid is among others an intermediate for adipic acid. Although the composition of the selected lignin types is divers, after steam bath distillation catechol of all aqueous phases could be converted metabolically (Fig. 4). As a consequence, this integrated biotechnological process can be applied widely in the lignin industry, which is embodied in the paper and pulp industry as well as the cellulosic biofuel industry. Furthermore, growth of the strain clearly relates to the metabolic conversion of catechol into *cis, cis*-muconic acid. Production yields up to 95% were generated.

### Example 2: Systems metabolic engineering of Corynebacterium glutamicum for the production of cis,cis-muconic acid from phenolic compounds

The soil bacterium *Corynebacterium glutamicum* was engineered into a genetically stable genome-based cell factory for high-level production of MA from aromatics. A basic producer was obtained by disruption of the native β-ketoadipate pathway at the level of muconate cycloisomerase. This enabled stoichiometric (100% yield) accumulation of MA from the aromatics catechol, phenol, and benzoic acid. The production was optimized by decoupling the biosynthetic pathway to MA from its native regulation control, using strong constitutive expression. As a result, the activity of the CatA key enzyme, forming MA from catechol inside the cell, was increased tenfold. Intracellular levels of catechol were more than 30-fold lower than extracellular levels, causing low toxicity, but still saturating the highly affine CatA enzyme. Taken to a fed-batch process, the created strain *C. glutamicum* LIMA-2 accumulated 85 g L⁻¹ MA from catechol in 60 hours, matching a maximum volumetric productivity of 2.4 g L⁻¹ h⁻¹. The excellent performance on catechol as substrate is particularly valuable, due to its role as central intermediate in aromatics degradation. These findings open the door to further valorize lignin, the second most abundant polymer on earth and a rich resource of small aromatics, for industrial MA production. In addition, they provide an important proof of concept for bio-based production from aromatics in *C. glutamicum.*

### 1 Materials and Methods

### 1.1 Microorganisms and plasmids

*C. glutamicum* ATCC 13032 was obtained from the American Type Culture Collection (Manassas, VA, USA). *Escherichia coli* stellar (Clontech Laboratories, Mountain View, CA, USA) and NM522 (Invitrogen, Carlsbad, CA, USA) were used for cloning purposes. *E. coli* NM522 harbors the plasmid pTc, that expresses a *C. glutamicum* specific DNA-methyltransferase (Kind et al., 2010. Metab. Eng. 12, 341-351.). This is needed, because *C. glutamicum* cells identify and degrade non methylated DNA. For genomic modification of *C. glutamicum,* the integrative, non-replicating plasmid pClik int sacB was used (Becker et al., 2005. Appl. Environ. Microbiol. 71, 8587-96.).

### 1.2 Molecular design and genetic engineering

For molecular strain, plasmid and primer design, the Clone Manager Professional 9 (Sci-Ed Software, Denver, USA) was used. The genetic construct for deletion of the *catB* gene (NCgl2318) in the genome of *C. glutamicum* comprised a DNA fragment, lacking 703 bp of the target gene and 500 bp-sized flanking regions as homologous recombination sites. For overexpression of the *catA* gene (NCgl2319) the genetic construct consisted of a 200 bp fragment of the promoter of the structural *tuf* gene (NCgl0480) and 500 bp-sized flanking regions as homologous recombination sites. All DNA fragments were amplified by PCR (2x Phusion Flash PCR Master Mix, Thermo Scientific, Waltham, MA, USA and peQSTAR, PEQLAB Biotechnology GmbH, Erlangen, Germany) from genomic DNA of *C. glutamicum* ATCC13032 with sequence specific primers (**Table 6**). DNA fragment and vector assembly was carried out by the method of Gibson (Gibson et al., 2009. Nat. Methods. 6, 343-5.). The reaction mixture contained per µL: 157.5 mM Tris-HCl (pH 7.5), 15.75 mM MgCl₂, 15.75 mM DTT, 42 mg PEG-800, 0.6 mg NAD, 25 mU Phusion High-Fidelity DNA Polymerase (Thermo Fisher Scientific), 7.5 mU T5 exonuclease (Epicentre, Madison, Wl, USA), 4 U µL⁻¹ Taq Ligase (Thermo Fisher Scientific), and 0.3 mM dNTPs. Prior to the assembly, the vector was linearized via restriction with *Bam*Hl (FastDigest, Thermo Fisher Scientific). Vector amplification in the *E. coli* strains Stellar and NM522, purification of plasmid DNA, and plasmid transformation into *E. coli* and *C. glutamicum* strains were performed as described previously (Becker et al., 2010. Eng. Life Sci. 1, 430-8.). PCR and sequence analysis (GATC Biotech AG, Konstanz, Germany) were used for plasmid and strain validation.

### 1.3 Batch cultivation in shake flasks

*C. glutamicum* was grown in baffled shake flasks with 10% filling volume at 30°C and 230 rpm on an orbital shaker (Multitron, Infors AG, Bottmingen, Switzerland, 5 cm shaking diameter). The cultivation procedure involved one pre-culture in complex medium (37 g L⁻¹ BHI) followed by another pre-cultivation and then by the main cultivation, both in minimal medium (Kind et al., 2013. Metab. Eng. 15, 184-95.), which contained the following salts and vitamins per liter: NaCl, 0.055 g CaCl₂·H₂O, 0.2 g MgSO₄·7H₂O, 15 g (NH₄)₂SO₄, 24.98 g K₂HPO₄, 7.7 g KH₂PO₄, 20 mg FeSO₄·7 H₂O, 0.5 mg biotin, 1 mg thiamin·HCl, 30 mg 3,4-dihydroxybenzoic acid and 10 mL of a 100x trace element solution (Vallino and Stephanopoulos., 1993. Biotechnol. Bioeng. 41, 633-646.). The medium was additionally supplemented with different amounts of benzoic acid, catechol, phenol, and glucose from filter sterilized stocks either alone or in mixtures as described below. The different medium ingredients were combined at room temperature freshly before use. The pH was kept constant at 7.0 ± 0.2 by manual addition of 2 M NaOH. All cultures were conducted as biological triplicate.

### 1.4 Tolerance testing

*C. glutamicum* was grown at 1 mL-scale in a micro bioreactor (BioLector I, m2plabs, Baesweiler, Germany), using 48-well flower plates. The incubation at different levels of catechol, phenol, and benzoic acid was conducted at 1,300 rpm and 30°C. All cultures were conducted as biological triplicate.

### 1.5 Fed-batch production of MA in shake flask

The pre-culture scheme was as described above. The main culture was conducted in minimal glucose medium, which was supplemented pulse-wise with glucose and with catechol. The catechol feed rate of 5 mM per hour was chosen on basis of the estimated specific productivity of the cells in batch culture (**Table 7**) The feed, added every hour, comprised 500 µL of a catechol stock (1 M) and 500 µL of a glucose stock (100 g L⁻¹). Considering the sample amount of 1 mL, taken every hour, the overall culture volume (100 mL) remained constant, so that each pulse increased the catechol concentration by 5 mM. The pH was kept constant at 7.0 ± 0.2 by manual addition of 2 M NaOH. The cultures were conducted as biological triplicate.

### 1.6 Fed-batch production of MA in a stirred tank bioreactor

The production performance of the optimized producer *C. glutamicum* LIMA-2 was evaluated in a fed-batch process in 1000 mL bioreactors (SR0700ODLS, DASGIP AG). The cultivation temperature was kept constant at 30°C via the CWD4 bio-block (DASGIP AG, Jülich, Germany). The pH and the pO₂ level were monitored online with a pH electrode (Mettler Toledo, Giessen, Germany) and a pO₂ electrode (Hamilton, Höchst, Germany). The pH was kept constant at 7.0 ± 0.1 by automated addition of 12M NaOH (MP8 pump system, Eppendorf, Hamburg, Germany). The dissolved oxygen level was maintained at saturation above 30 % by variation of the stirrer speed and the aeration rate. The initial batch of 300 mL minimal medium with 10 g L⁻¹ glucose and 5 mM catechol was inoculated with cells as described above. Catechol was added pulse-wise from a concentrated feed (4 M), using the signal of the dissolved oxygen probe as a trigger. In addition, a glucose feed was given continuously. The feed rate was re-adjusted, when needed, to maintain the level of glucose in a range of about 5 - 15 g L⁻¹. The feed contained per liter: 450 g glucose, 70 g (NH₄)₂SO₄, 1 g NaCl, 0.055 g CaCl₂·H₂O, 0.2 g MgSO₄·7H₂O, 15 g (NH₄)₂SO₄, 24.98 g K₂HPO₄, 7.7 g KH₂PO₄, 20 mg FeSO₄·7 H₂O, 0.5 mg biotin, 1 mg thiamin·HCl, 30 mg 3,4-dihydroxybenzoic acid, and 100 mL of a 100x trace element solution (Vallino and Stephanopoulos., 1993. Biotechnol. Bioeng. 41, 633-646.). The feed rate was adjusted, Data acquisition and process operations were controlled by the DASGIP control software (DASGIP AG). The production process was conducted as duplicate.

### 1.7 Extraction of intracellular metabolites

Two mL of exponentially growing cells were harvested by vacuum filtration (cellulose nitrate membrane filters, 0.2 µm pore size, 47 mm, Sartorius, Göttingen, Germany). The filter was washed with 15 mL of 2.5% NaCl, matching the ionic strength of the medium, and was then transferred into a plastic cup, containing 2 mL of boiling deionized water (Bolten et al., 2007. Anal. Chem. 79, 3843-9.). The sample was incubated in a water bath at 100°C for 15 min and then chilled on ice. The extract was transferred into a fresh vial and clarified from cell debris (5 min, 13,000 xg, and 4 °C).

### 1.8 Substrate and product quantification

Catechol, phenol, benzoic acid, and MA were quantified by HPLC (Agilent 1200 Series, Agilent Technologies, Waldbronn, Germany), including separation at 25 °C on a reversed phase column (Nucleodur E100/3 C18 Isis 3 µm, Macherey-Nagel, Weilmünster, Germany) with a gradient of water (0.0035% H₃PO₄) and acetonitrile as mobile phase at a flow rate of 1 mL min⁻¹. A diode array detector was used for detection of MA (260 nm) and the aromatics (210 nm). The quantification of glucose was carried out by isocratic HPLC (Agilent 1260 Infinity Series, Waldbronn, Germany). The separation was conducted on an Aminex HPX-87H column (300 × 7.8 mm; Bio-Rad, Munich, Germany) at 55 °C. As mobile phase, 3.5 mM H₂SO₄ was used at a flow rate of 0.8 mL min⁻¹. The refraction index was used for detection. The concentration of cell dry mass (CDM) was calculated from the measured optical density (OD₆₆₀) using a correlation factor of CDM (g/L) = 0.32 × OD₆₆₀ (Rohles et al., 2016. Microb. Cell Fact. 15, 154.). The exact monitoring of the cell concentration during the intracellular metabolite sampling and the above correlation allowed to relate the obtained metabolite levels to the CDM (µmol g_{CDM}⁻¹). The correlation between the cytoplasmic volume and the CDM (1.95 mL g_{CDM}⁻¹) was used to express intracellular concentrations in mM (Gutmann et al., 1992. Biochim. Biophys. Acta. 1112, 115-23.).

### 1.9 Determination of enzyme activity

Crude cell extracts were prepared from exponentially growing cells by mechanical cell disruption. Cell harvest was carried out as previously described (Buschke et al., 2011. Biotechnol. J. 6, 306-17.). Aliquots of 1 mL cell suspension were transferred into FastPrep-24 vials (MP Biomedicals, Illkirch-Graffenstaden, France), containing silica beads (Ø 0.1 mm). Cell disruption was carried out in 2 × 30 sec cycles at 5,000 rpm (Precellys-24, Peqlab, Hannover, Germany), including a 5 minute cooling pause on ice. The extract was also chilled on ice after the disruption. Removal of cell debris and protein quantification was performed as previously described (Becker et al., 2009. Appl. Environ. Microbiol. 75, 7866-9.). The activity of catechol-1,2-dioxygenase (CatA) was assayed in Tris-HCl buffer (100 mM, pH 8.2, 0.75 mM DTT). For this purpose, 900 µL buffer was mixed with 50 µL catechol (1 mM, pH 7.0) and 50 µL crude cell extract. The formation of MA (ε = 16.8 mL µM⁻¹ cm⁻¹) was monitored via the change in absorbance at 260 nm (Guzik et al., 2013. Int. J. Gen. Mol. Microbiol. 103, 1297-1307.). Negative controls were conducted without the addition of crude cell extract and catechol, respectively. The substrate affinity of the enzyme was determined by varying the concentration of catechol. The kinetic parameters of the enzyme were obtained by fitting the experimental data to the Michaelis-Menten type kinetic equation (OriginLab, Northhampton, MA, USA). The protein content in the crude cell extract was quantified, using bovine serum albumin as standard (Pierce BCA Protein Assay Kit, Thermo Fisher Scientific).

### 2 Results

### 2.1 Deletion of muconate-cycloisomerase in C. glutamicum enables MA production from small aromatics

To block the metabolization of small aromatics at the level of MA, the *catB* gene, encoding muconate-cycloisomerase was deleted from the genome of the microbe. Clones, obtained after the second homologous recombination, were analyzed for the desired modification. The deletion was found successful on basis of the shortened PCR product of 1,050 bp **(****Fig. 13****).** The wild type control yielded a fragment size of 1,750 bp, representing the native gene. The resulting mutant *C. glutamicum* ATCC130232 Δ*catB* was designated C. *glutamicum* LIMA-1. In contrast to the wild type, the novel strain was no longer able to grow on the aromatic compounds benzoic acid, catechol, and phenol as sole carbon source, respectively (data not shown). Growth, however, was enabled by cultivation of the mutant on the aromatics in the presence of small amounts of glucose. After 24 hours of incubation, elevated levels of MA were found for each of the aromatics tested (**Fig. 7**). Hereby, the conversion was almost stoichiometric: the MA yield on the aromatics was close to 100%. Surprisingly, the mutant completely converted rather high amounts of phenol (5 mM), catechol (10 mM), and benzoic acid (20 mM) within 24 hours. At higher concentrations, the conversion was incomplete within the studied time period, eventually resulting from inhibitory effects of the substrates and slower growth.

### 2.2 C. glutamicum LIMA-1 shows a remarkable tolerance to small aromatics

A set of growth experiments was conducted to study potential inhibitory effects of the compounds involved on growth of the producing strains. The mutant exhibited a remarkable tolerance and grew up to high levels of catechol (30 mM), phenol (30 mM), benzoic acid (80 mM), the highest concentrations tested. The non-charged aromatics catechol and phenol were found more toxic and caused a stronger decrease of the vitality than the acid **(****Fig. 14****).**

### 2.3 The efficiency of MA production differs strongly with the aromatic substrate

Although the conversion of the aromatics into MA was complete in all cases, the cells differed strongly in their substrate preference (Figs. 8A-C). On 20 mM benzoic acid, the LIMA-1 strain consumed the aromatic substrate from early on and reached an MA titer of 20 mM within only 14 h. During this phase, benzoic acid was preferred over glucose: the cells assimilated only small amounts of glucose, showed minor growth and rather formed MA in a growth-decoupled biotransformation. The MA level increased linearly, indicating a stable productivity. At the time point of benzoic acid depletion, most of the glucose was still present. Different from that, catechol was co-consumed with glucose **(****Fig. 8B****).** The MA production was growth associated, which enabled an increasing accumulation rate correlating to the increasing cell concentration. The complete conversion of 10 mM catechol took about 20 hours. The overall yield from catechol to MA was 1.00 ± 0.01 mol mol⁻¹ (**Table 7**). Phenol was metabolized even slower **(****Fig. 8C****).** Here, the cells mainly consumed the glucose. During the initial 10 hours of glucose utilization, less than 1 mM phenol was converted into MA. The cells, however, later managed to convert the entire phenol into the target product. The MA conversion from the aromatic substrate was again 100 % (**Table 7**). Regarding cellular growth, the cultures on benzoic acid and on phenol exhibited similar biomass yields, whereas the catechol-grown cells were somewhat less efficient.

### 2.4 The induction level of catechol-1,2-dioxygenase is responsible for the MA production performance

Catechol-1,2-dioxygenase (CatA), the MA forming enzyme in *C*. *glutamicum,* was now studied. It was interesting to explore the role of this key enzyme for the strong substrate-dependency of MA formation in more detail. A first culture, only on glucose but without any aromatics added, revealed a low CatA activity of 30 mU mg⁻¹ **(****Fig. 9A****).** Interestingly, the presence of either catechol or phenol in the growth medium did not result in any change of the CatA activity. In fact, CatA was still expressed at the constitutive basal level. In contrast, the cells showed 16-fold higher CatA activity, when cultivated on benzoate (490 mU mg⁻¹). The crude extract of benzoate-grown cells was used to assess the kinetics of the CatA enzyme, in particular the affinity of the enzyme for catechol. The reaction rates, obtained at different catechol levels were fitted to a Michaelis-Menten type kinetics **(****Fig. 9B****).** The K_{M}-value for catechol, enabling operation of the enzyme at 50% of its maximum rate, was 2.3 µM. Hence, the enzyme had a high affinity, enabling efficient conversions already at low levels of the substrate. Therefore, the poor production performance on catechol and phenol seemed due to a limited capacity of the key enzyme CatA in the producing strain.

### 2.5 Targeted overexpression of catechol-1,2-dioxygenase decouples the enzyme from native induction and enables much faster MA production from catechol

To overcome the bottleneck at the level of CatA, caused by the native induction mechanism, the catA gene was overexpressed, using the strong constitutive *tuf* promoter. Positive clones were obtained after the two recombination events. They revealed the fragment size of 1,240 bp, expected for the promoter exchange **(****Fig. 15****).** In contrast, the PCR product of the native genomic region as control resulted in a smaller fragment length of 1,000 bp. The correctness of the genetic modification was verified by sequencing. The obtained strain *C*. *glutamicum ΔcatB P_{tuf}catA*, was designated C. *glutamicum* LIMA-2. To investigate the effect of the promoter exchange, the novel mutant was analyzed for its CatA activity **(****Fig. 9C****).** When the cells were grown on either benzoic acid, catechol or phenol, CatA was generally expressed at high activity (330 mU mg⁻¹), independent from the substrate used. In comparison to the native expression control, the *tuf* promoter enabled 10-fold higher CatA levels on catechol and on phenol. For benzoic acid, the enzyme activity did not fully reach that of the native induction system. The novel strain LIMA-2 enabled a much faster MA conversion (**Fig. 10A**). The catechol added (10mM) was completely converted into MA in less than 6 hours (extrapolated from the time profile of the catechol concentration). This corresponded to a specific MA production rate of 5.2 mmol g⁻¹ h⁻¹ (0.7 g g⁻¹ h⁻¹) (**Table 8**), which was 25-fold higher than that of the parent strain (**Table 7**). The MA yield on catechol was 100 %. Again, cells showed minor growth until the entire catechol was converted and co-consumed the aromatic together with only small amounts of glucose. The intracellular levels of substrate and product were quantified during the mid-phase of the production process. The cells contained 330 ± 42 µmol g⁻¹ catechol and 200 ± 18 mol g⁻¹ MA.

### 2.6 Rational expression control of catechol-1,2-dioxygenase by the strong tuf promoter provides higher MA productivity from catechol than the native induction control by benzoic acid

As shown above, benzoic acid acted as natural inducer of CatA (**Fig. 10A**). The addition of small amounts of the inducer appeared as alternative strategy to boost catechol conversion. The MA producer with the native regulation mechanism, LIMA-1, was therefore studied using mixture of catechol (10 mM) and benzoate (2 mM). The strain successfully formed MA at stoichiometric yield, but needed 50% longer time to complete the conversion **(****Fig. 10B****),** indicated by the significantly reduced specific productivity **(Table 8).** Benzoic acid was not degraded, until the entire amount catechol had been consumed. It obviously only functioned as inducer during this time period. Additional tests at higher benzoic acid levels (3, 5, and 10 mM) yielded rather similar results: the specific productivity and the yield were not affected (data not shown). In contrast to catechol, the conversion of phenol into MA remained slow in the superior producer. Similar to its ancestor, *C. glutamicum* LIMA-2 quickly consumed the glucose present and again preferred the sugar strongly over phenol. About 1 mM of MA was formed during 9 h of cultivation, the time needed for complete consumption of glucose. Obviously, the overexpressed *catA* gene did not result in an improvement in the production from phenol.

### 2.7 Pulse-wise feeding of catechol reveals excellent process robustness of C. glutamicum LIMA-2

A fed-batch operated shake flask culture should aim for higher MA titers, using pulse-wise feeding to avoid toxic levels of catechol. *C. glutamicum* LIMA-2 was grown on glucose for the first 2 hours and then fed every hour with catechol **(****Fig. 11****).** It was interesting to note that the specific growth rate immediately dropped to about 0.08 h⁻¹, when the first feed pulse was added. The cells kept growing at this reduced rate. Throughout the entire cultivation, the added aromatic was completely converted into the target product. Catechol did not accumulate at any stage of the process. After 30 h, the cells achieved a final MA titer of 19 g L⁻¹ (133 mM) at a product yield from catechol of 100%. The high production efficiency was kept until the end of the cultivation. After about 24 hours, the glucose addition was intentionally stopped to study the impact of the sugar. Even when glucose was depleted, the cells still maintained MA accumulation.

### 2.8 C. glutamicum LIMA-2 sets a benchmark in high-level MA production

The production performance of *C. glutamicum* LIMA-2 was next investigated in a fed-batch process. For this purpose, a lean medium was used, containing only catechol, sugar, and salts. The MA production started immediately and reached a titer of 85 g L⁻¹ within 60 hours **(****Fig. 12A****).** The cell concentration increased from initially 1 g L⁻¹ to about 14 g L⁻¹ after 34 hours, roughly half of the total process time. During this phase, glucose was co-consumed with catechol and utilized for growth at a reduced rate. At the end of this initial phase, about 30 g L⁻¹ MA was formed. Subsequently, the cells switched to an almost exclusive production mode for the rest of the process. They converted catechol at an even higher rate into MA, and formed additionally more than 50 g L⁻¹ of the product within 25 hours, but did not grow anymore and consumed only limited amounts of glucose. The space-time-yield increased continuously during the process and reached a maximum value of 2.4 g L⁻¹ h⁻¹ **(****Fig. 12B****).** During the entire process, the strain exhibited a constant MA yield from catechol of 100% **(****Fig. 12C****).** Regarding the process control, the on-line signal for dissolved oxygen (DO) served as excellent trigger for the addition of new catechol. Each time, when catechol was depleted and the oxygen-dependent reaction of CatA was accordingly halted, the DO signal sharply increased **(****Fig. 12D****).** Likewise, also the pH value sensitively indicated the physiology of the cells and dropped during each interval, as long as MA was formed. The established feed addition allowed a tight control of catechol in the broth at low level. An exception was a phase in the early process stage, when the feed exceeded the capacity of the cells and the catechol level transiently raised to more than 10 mM in concentration. However, the cells could cope with this process environment and quickly degraded catechol to levels below 5 mM, as long as the feed addition was halted.

### Tables

**Table 6: Description of primers that were used in the present work for genome-based deletion of the catB gene (NCgl2318) and integration of the tuf-promoter for overexpression of the catA gene (NCgl2319) in Corynebacterium glutamicum. AT = annealing temperature.**

| **Name** | **Sequence** | **Use** | **AT [°C]** |
|---|---|---|---|
| *ΔcatB* TS1 FW | 5’- TTAACAATTGGGATCCTCTAGACC CACTCCAGAGCATTGGGGTGTTT- 3’ (SEQ ID No. 110) | Amplification of TS1; TS1 FW + overlap to pClik, TS 1 RV + overlap to TS2 FW | 55 |
| *ΔcatB* TS1 RV | 5'- AGGTCCAATCGGCACCGCAGCAT- 3' (SEQ ID No. 111) | | |
| *ΔcatB* TS2 FW | 5'- GGATGCTGCGGTGCCGATTGGAC CCTCAAGCGCCTTCATGGTCTCAA C-3' (SEQ ID No. 112) | Amplification of TS2; TS2 FW + overlap to TS1 RV, TS 2 RV + overlap to pClik | 57 |
| *ΔcatB* TS2 RV | 5'- GCAGCCCGCTAGCGATTTAAATC CCTGGGCACCCATGGCGCCCAG- 3' (SEQ ID No. 113) | | |
| P*_{ef-tu}-catA -* TS1 FW | 5'- AATTGGGATCCTCTAGACCCACG ATTCCCTCTTCAGGGCG-3'    (SEQ ID No. 114) | Amplification of TS1; TS1 FW + overlap to pClik, TS1 RV + overlap to P*_{ef-tu}* | 61 |
| P*_{ef-tu}-catA -* TS1 RV | 5'- CATTCGCAGGGTAACGGCCAGTC ATACTCCTAGAGACTTTGGGGG-3' (SEQ ID No. 115) | | |
| P_{ef-t*u*}-*catA* - P*_{ef-fu}* FW | 5'- AAAGTCTCTAGGAGTATGACTGG CCGTTACCCTGCGAATG-3'    (SEQ ID No. 116) | Amplification of P*_{ef-fu}*; P*_{ef-tu}* FW + overlap to TS1 RV, P*_{ef-tu}* RV + overlap to TS2 FW | 62 |
| P*_{ef-tu}-catA -* P*_{ef-tu}* RV | | | |
| | 5'- ATCTGTTCAGCTGAAGTCATTGTA TGTCCTCCTGGACTTCGTGG-3' (SEQ ID No. 117) | | |
| P*_{ef-tu}-catA -* TS2 FW | 5'- GAAGTCCAGGAGGACATACAATG ACTTCAGCTGAACAGATCG-3' (SEQ ID No. 118) | Amplification of TS2; TS2 FW + overlap to P*_{ef-tu}*, TS2 RV + overlap to pClik | 57 |
| P*_{ef-tu}-catA -* TS2 RV | 5’- CCGCTAGCGATTTAAATCCCTAGT ATCCGTCCTCATCTGCG-3’ (SEQ ID No. 119) | | |
| PR154 | 5’- ATTGTCTGTTGTGCCCAGTCATAG -3’ (SEQ ID No. 120) | Validation of vector pClik int *sacB* (including respective insert) | 53 |
| PR318 | 5’-AATAATAGTGAACGGCAGGT-3’ (SEQ ID No. 121) | | |

**Table 7. Kinetics and stoichiometry of MA production from aromatic compounds, using Corynebacterium glutamicum LIMA-1. The data shown comprise the specific glucose uptake rate (q_{Glc}), the specific MA production rate (q_{MA}), the biomass yield (Y_{X/}_{Glc}), and the MA yield (Y_{mA/Aro}) and represent mean values and standard deviations from three biological replicates. The initial concentrations were: 20 mM benzoate, 10 mM catechol, and 5 mM phenol, respectively.**

| | **Benzoic acid** | **Catechol** | **Phenol** |
|---|---|---|---|
| *q_{MA}* [mmol g⁻¹ h⁻¹] | 3.3 ± 0.1 | 0.2 ± 0.0 | 0.1 ± 0.0 |
| *q_{Glc}* [mmol g⁻¹ h⁻¹] | 0.5 ± 0.0 | 0.3 ± 0.0 | 0.6 ± 0.0 |
| *Y*_{*X*/*Glc*} [g g⁻¹] | 0.46 ± 0.01 | 0.38 ± 0.01 | 0.48 ± 0.01 |
| *Y*_{*MA*/*Aro*} [mol mol⁻¹] | 1.00 ± 0.02 | 0.99 ± 0.02 | 0.95 ± 0.09 |

**Table 8. Kinetics and stoichiometry of MA production from catechol, using Corynebacterium glutamicum LIMA-1 and LIMA-2. The data shown comprise the specific glucose uptake rate (q_{Glc}), the specific MA production rate (q_{MA}), the biomass yield (Y_{X/}_{Glc}), and the MA yield (Y_{MA/Aro}) and represent mean values and standard deviations from three biological replicates. The culture of the LIMA-1 strain contained 10 mM catechol plus 2 mM benzoate as inducer, whereas the medium for the LIMA-2 strain contained only 10 mM catechol.**

| | **LIMA-1** | **LIMA-2** |
|---|---|---|
| *q_{MA}* [mmol g⁻¹ h⁻¹] | 5.2 ± 0.4 | 3.5 ± 0.1 |
| *q_{Glc}* [mmol g⁻¹ h⁻¹] | 0.7 ± 0.0 | 0.4 ± 0.0 |
| *Y*_{*X*/*Glc*} [g g⁻¹] | 0.40 ± 0.02 | 0.40 ± 0.02 |
| *Y*_{*MA*/*Aro*} [mol mol⁻¹] | 1.00 ± 0.01 | 1.00 ± 0.01 |

### Example 3: Systems metabolic engineering of Amycolatopsis sp. for the production of cis,cis-muconic acid from phenolic compounds

### 1 Materials and Methods

### 1.1 Strains and plasmids

*Amycolatopsis species* (ATCC 39116) was obtained from the American Type Culture Collection (Manassas, VA, USA). The strain has been previously deposited as *Streptomyces setonii* 75iv2. *Escherichia coli* DH5α was used for cloning (Invitrogen, Carlsbad, CA, USA). The methylation-deficient *E. coli* ET12567 (*dam-13::Tn9, dcm-6, hsdM, hsdS*), containing pUZ8002 was used as the donor in intergeneric conjugation (Kieser et al., 2000. Practical Streptomyces genetics. The John Innes Foundation, Norwich, UK.). The plasmid pKC1132 (Kieser et al., 2000, cited above) was used to derive the integrative plasmid pKG1132 with β-glucuronidase reporter activity (Myronovskyi et al., 2011 Microbiol. 77, 5370-83.). All strains and plasmids used in this study are listed in **Table 12.** All primers used in this study are listed in **Table 13.**

### 1.2 Media

For genetic engineering work, *E. coli* strains were grown in liquid Luria-Bertani (LB) medium or on solid LB medium, which additionally contained 20 g L⁻¹ agar (Becton Dickinson, Heidelberg, Germany). For conjugation, *A. species* ATCC 39116 was grown in liquid GYM medium, containing per liter: 4 g glucose, 4 g yeast extract (Becton Dickinson), and 10 g malt extract (Becton Dickinson). For sporulation and for cloning purposes, solid GYM medium, additionally containing 20 g L¹ agar (Becton Dickinson), was used. For the pre-culture and the main culture in growth and production studies, a mineral medium (pH 7.2) was used, which contained per liter: 4.5 g Na₂HPO₄*7H₂O, 3.0 g (NH₄)₂SO₄, 1.0 g KH₂PO₄, 200 mg NaCl, 200 mg MgSO₄·7H₂O, 50 mg CaCl₂·2H₂O, 2 mg ZnSO₄·7H₂O, 1.2 mg MnCl₂·4H₂O, 0.4 mg CoCl₂·6H₂O, 0.2 mg NaMoO₄·2H₂O, 0.2 mg CuSO₄·5H₂O. Furthermore, the minimal medium contained one or more of the following carbon sources at varied concentration: catechol, benzoate, *o*-cresol, guaiacol, phenol, *p*-coumarate, MA, and/or glucose, respectively. For direct MA production from pre-treated lignin, appropriate amounts of the obtained hydrolysate (see below) were added. For plasmid maintenance, kanamycin (50 µg mL⁻¹) or apramycin (50 µg mL⁻¹) were added, respectively, when needed. In addition, 5-Bromo-4-chloro-1*H*-indol-3-yl β-D-gluco-pyranosiduronic acid (X-Gluc) was added to selection agar plates (40 µg mL⁻¹) for blue white screening.

### 1.3 Recombinant DNA techniques

DNA fragments were amplified by PCR (2× Phusion High-Fidelity PCR Master Mix with GC Buffer, Thermo Scientific, Waltham, MA, USA) from genomic DNA of A. *species* ATCC 39116, using sequence specific primers **(Table 12).** Subsequently, fragments of interest were purified (Wizard^{®} SV Gel, PCR Clean-Up System, Promega, Mannheim, Germany) and assembled in vitro (Gibson et al., 2009. Nat Meth. 6, 343-345.). The reaction mixture in the assembly contained 157.5 mM Tris·HCl (pH 7.5), 15.75 mM MgCl₂, 15.75 mM DTT, 42 mg µL⁻¹ PEG-800, 0.6 mg µL⁻¹ NAD, 25 mU µL⁻¹ Phusion High-Fidelity DNA Polymerase (Thermo Fisher Scientific), 7.5 mU µL⁻¹ T5 exonuclease (Epicentre, Madison, USA), 4 U µL⁻¹ Taq Ligase (Thermo Fisher Scientific), and 0.3 mM dNTPs. In order to later clone the DNA fragments into a linearized vector, homologous overlaps were created by fusing the forward and reverse gene priming sequences with 20 nt sequences at their 5' end. For plasmid construction, the vector backbone was linearized using *Eco*RV (FastDigest, Thermo Fisher Scientific, St. Leon-Roth, Germany). The plasmids were transformed into *E*. *coli* DH5α by heat shock, multiplied in the cloning host, isolated (QIAprep Spin MiniPrep Kit, Quiagen, Hilden, Germany), transformed into *E. coli* ET12567/pUZ8002, and subsequently transformed into *Amycolatopsis,* using conjugation. In short, 1.5 mL suspension of the transformed *E. coli* ET12567/pUZ8002, carrying the plasmid to transferred, was mixed with 1.5 mL suspension, containing spores of the recipient *A. species* ATCC 39116 wildtype. The mixture was plated on GYM agar and incubated for 14 hours at 30 °C. Then, 10 µL apramycine solution (25 µg mL⁻¹) and 10 µL of nalidixic acid solution (200 µg mL⁻¹) were spread onto the plate. After about 3 days incubation at 30°C, the newly grown colonies were transferred onto GYM agar plates, containing apramycine, and were incubated for 24 hours. Subsequently, 3 µL of X-Gluc solution (100 mg mL⁻¹) was pipetted onto each colony. After 20 minutes at 30°C, colonies were evaluated for their color. Positive clones were cultivated overnight in liquid GYM medium without selection pressure, plated on GYM agar, containing 40 µg mL⁻¹ X-Gluc, and incubated at 30°C for another 24 hours, followed again by evaluation of the obtained colonies. Positive clones, now white-colored, were tested by PCR to differentiate between the desired mutants and wildtype. The Clone Manager Professional 9 (Sci Ed Software, Denver, CO, USA) was used for molecular strain, plasmid and primer design.

### 1.4 Construction of the integrative plasmid pKG1132 for gene inactivation

In order to enable a blue-white screening in *Amycolatopsis species* ATCC 39116, the *gusA* gene, encoding the β-glucuronidase protein (NP 416134) from *Escherichia coli,* was implemented as selection marker. Accordingly, a DNA fragment, containing the *gusA* gene under the control of the *tipA* promoter, was synthesized and cloned via ligation as Bglll fragment into the Bglll site of pKC1132 (Bierman et al., 1992. Gene. 116, 43-9.). The plasmid was designated pKG1132 **(****Fig. 24A****).**

### 1.5 Construction of the deletion mutants Amycolatopsis species ATCC 39116 MUC-1 and MUC-2

The gene deletion relied on homologous recombination, using the integrative plasmid pKG1132. The constructs for the deletion comprised (i) the upstream sequence (2500 bp) together with the translational start codon of the target gene at the 3' end and (ii) the downstream sequence (2500 bp) together with the translational stop codon of the target gene at the 5' prime end. For the deletion of the AATC3_020100018510 gene (G10GW-3575, 1104 bp), the genomic DNA of *A. species* ATCC 39116 was amplified by PCR using the primer pairs P40/P53 and P54/P45. For the deletion of the AATC3_020100009302 gene (G10GW-1735, 1278 bp), the genomic DNA of *A. species* ATCC 39116 was amplified by PCR using the primer pairs P17/P18 and P19/P20. The fragments were cloned via Gibson assembly into the EcoRV site of pKG1132, which yielded the deletion vectors pKG1132 MUC1 and pKG1132 MUC-2, respectively (**Figs. 24B****,C**). The correctness of the vectors was confirmed by restriction analysis.

### 1.6 Low temperature hydrothermal conversion of lignin

Hydrothermal conversion was conducted in a 500 mL stirred, stainless steel pressure vessel (4575A, Parr Instruments, Moline, IL, USA). A suspension of 5 g lignin from pine (IndulinAT, S3Chemicals, Bad Oeynhausen, Germany) in 250 mL demineralized water was filled into the vessel, followed by sealing of the reactor. Subsequently, the stirrer speed was set to 400 rpm and nitrogen gas was used to purge the reactor five times. Then, the reactor was heated. The time point, when the chosen temperature (330, 350, or 370 °C) was reached, was taken as the start point of the reaction. The hydrothermal conversion was conducted for 15, 20, and 25 minutes. When the selected reaction time was over, the reactor was cooled down, using the internal cooling coil, flushed with cold water (8 °C) in combination with a fan from the outside. Once, a temperature of 80 °C was reached, the reactor was purged 3 times with nitrogen and was then opened. The hydrolysate was clarified from debris by centrifugation (10,000 × *g,* 5 minutes, and room temperature). The collected supernatant (250 mL) was concentrated by steam distillation. For this purpose, it was filled into a warmed distillation flask (500 mL), flushed with water steam and connected to a water-cooled Liebig condenser.
The distillation was continued for 3 hours, while the steam-distilled concentrated aromatics were collected.

### 1.7 Cultivation

*Amycolatopsis* was cultivated in baffled shake flasks, filled to 10% with liquid medium. Single colonies from agar plates, incubated for 24 h at 37 °C, were used as inoculum for the first pre-culture. From there, cells were harvested during the exponential growth by centrifugation (5 min, 8800 xg, and 37°C), and used afterwards as inoculum for the main culture. All shake flask cultivations were conducted in triplicate at 37°C and 230 rpm on an orbital shaker (Multitron, Infors AG, Bottmingen, Switzerland).

### 1.8 Fed-batch production of MA in a stirred tank bioreactor

The production performance of the optimized MA producer was evaluated in a fed-batch process in a 250 mL bioreactor (SR0700ODLS, DASGIP AG). The cultivation temperature was kept constant at 37°C via the CWD4 bio-block (DASGIP AG, Jülich, Germany). The pH and the pO₂ level were monitored online with a pH electrode (Mettler Toledo, Giessen, Germany) and a pO₂ electrode (Hamilton, Höchst, Germany). The pH was kept constant at 7.2 ± 0.1 by automated addition of 6M NaOH (MP8 pump system, Eppendorf, Hamburg, Germany). The dissolved oxygen level was maintained at saturation above 30 % by variation of the stirrer speed and the aeration rate. The initial batch of 100 mL minimal medium, supplemented with 5 mM guaiacol and 6 g L⁻¹ glucose was inoculated with cells as described above. Guaiacol was added pulse-wise from a pure feed (9 M), when it had been depleted by the cells. The exact time points were estimated on basis of at-line monitoring of the guaiacol level, using HPLC (see 2.10). Data acquisition and process operations were controlled by the DASGIP control software (DASGIP AG).

### 1.9 Quantification of cell and biomass concentration

The cell concentration was determined in duplicate as optical density at 600 nm (OD₆₀₀). The cell dry mass (CDM) was determined gravimetrically in triplicate. In short, 20 mL culture broth was filtered (Whatman cellulose filter, Grade 3, GE-Healthcare Life Sciences, Little Chalfont, UK), washed three times, and dried to constant weight (HB43-S, Mettler-Toledo, Columbus, OH, UA). The correlation factor was CDM [g L^{- 1}] = 0.45 × OD₆₀₀.

### 1.10 Quantification of substrates and products

The quantification of glucose was performed by isocratic HPLC (1260 Infinity Series, Agilent). The separation was carried out on an Aminex HPX-87H column (300 × 7.8 mm; Bio-Rad) at 65°C, using 50 mM H₂SO₄ as mobile phase at a flow rate of 0.5 mL min⁻¹. Detection was performed via refraction index. For the quantification, external standards were used. The aromatics (catechol, guaiacol, o-cresol, m-cresol, p-cresol, phenol), and MA were separated by HPLC (1260 Infinity, Agilent, Waldbronn, Germany), using a reversed phase column (Nucleodur C18 Isis, 3 µm, Macherey Nagel, Düren, Germany), and a gradient of 0.025% H₃PO₄ (A) and acetonitrile (B), at 25 °C and a flow rate of 1 mL min⁻¹. The gradient was as follows: 0-13.8 min, 100-31% A, 0-69% B; 13.8-14.3 min, 31-0% A, 69-100% B; 14.3-17.3 min, 100% B; 17.3-17.8, 0-100% A, 100-0% B; 17.8-24.3 min, 100% A. The quantification was done by UV absorption at the individual absorbance maximum for each analyte: 210 nm for catechol, phenol, guaiacol, and the cresols; 220 nm for benzoate; 260 nm for MA, respectively. It should be noted that methyl-MA, formed in some of the experiments from o-cresol, did not interfere with any of the above analyte signals. A quantification of methyl-MA was not done due to a lack of a pure standard.

### 1.11 Enzymatic analysis of catechol 1,2-dioxygenase

The cells were harvested by centrifugation, washed in 60 mM Tris-HCl buffer (pH 8.2), and disrupted in pre-packed lysis tubes (0.1 mm silica spheres, Lysing Matrix B, MP Biomedicals, Heidelberg, Germany), using three intervals of disruption in a ribolyzer (Precellys-24, PeqLab, Hannover, Germany, 3 × 30 s, 6 m s⁻¹) with 1 cooling pauses on ice in between. Removal of cell debris and protein quantification was performed as previously described (Becker et al., 2009. Appl. Environ. Microbiol. 75, 7866-9.). The final reaction mixture contained 30 mM Tris-HCl buffer (pH 8.2), 20 µM catechol and 100 µL mL⁻¹ crude cell extract. The reaction was monitored on-line via the change in absorbance at 260 nm, reflecting the accumulation of MA (ε = 16,800 M⁻¹ cm⁻¹) (Jimenez et al., 2014. Microbiol. 16, 1767-78.). This extinction coefficient was also considered in additional experiments, in which catechol was replaced by equimolar amounts of 3-methyl catechol and 4-methyl catechol and the enzyme formed methylated forms of MA, respectively. Controls without cell extract and without substrate were included.

### 2 Results

### 2.1 Aromatics substrate spectrum of wildtype

The wildtype was cultivated in minimal medium on a range of aromatics, reflecting a spectrum of potential lignin-derivable raw materials. The microbe could use various compounds as a sole source of carbon **(Table 9),** namely p-coumaric acid, benzoic acid, guaiacol, catechol, phenol, and toluene. In addition, also MA was used by the strain.

### 2.2 The wildtype A. species ATCC 39116 accumulates MA from guaiacol in the milligram scale

The capability to use guaiacol was found most relevant, given the huge importance of the aromatic as the dominant building block of softwood lignin, and was therefore studied now in more detail. The wildtype was found robust and tolerated elevated levels of guaiacol. Concentrations up 25 mM were utilized **(****Fig. 25****).** Regarding growth kinetics and stoichiometry, the cells immediately started to consume guaiacol **(****Fig. 18****).** Interestingly, a slight but significant excretion of MA was observed. On a quantitative basis, MA reached a level of 0.3 mM, which represented about 5% of the initially added guaiacol. The aromatic enabled fast exponential growth at a specific growth rate of 0.30 h⁻¹. After about 7 hours, guaiacol was completely depleted, which obviously triggered the re-uptake of MA.

In a second experiment, guaiacol was used in addition to glucose (**Figs. 18B****,C,D).** Again, the cells immediately started to grow, whereby guaiacol was clearly preferred over glucose as a substrate **(****Fig. 18C****).** The slightly increased specific growth rate (µ = 0.32 h⁻¹) during the co-consumption phase seemed to indicate a synergistic use of the two substrates **(****Fig. 18D****).** Again, MA accumulated to a low extent until guaiacol was depleted **(****Fig 18B****).** Subsequently, the microbe switched to a co-consumption of glucose and MA, whereby glucose was the dominant substrate. About 2.5 g CDM was synthetized from about 5 g glucose during the glucose consumption phase, which resulted in a biomass yield of 0.50 g g⁻¹. This indicated that glucose could serve as an efficient growth substrate in cases, where aromatics were not available for growth. Accordingly, glucose appeared as suitable growth nutrient in a later MA production set-up, which should preferably involve a producer with a disrupted β-ketoadipate pathway, unable to grow on aromatics anymore.

### 2.3 Low temperature hydrothermal conversion of softwood lignin

One of the ultimate goals of this work, was the MA production from real lignin. For this purpose, the aim was to depolymerize industrial softwood Kraft lignin, devoid of carbohydrates, into small aromatics. Hydrothermal conversion was chosen for the lignin treatment. The idea was to use the contained aqueous phase for the harvest of water soluble small aromatics, eventually generated during the process, because they promised a good bio-availability to the microbe. The hydrothermal conversion of the lignin was first conducted at 350 °C and 165 bar for 20 minutes. The water phase, collected after the process, contained signicant amounts of the following aromatics: guaiacol, catechol, phenol and o-cresol **(****Fig. 19****).** On a relative basis, guaiacol was the dominant compound formed (44%), followed by catechol (30%), phenol (14%), and o-cresol (12%). Tests at different temperatures revealed that the formation of guaiacol was enhanced, using a lower temperature (330 °C, 130 bar). The aromatics spectrum was slightly shifted to catechol and phenol at higher temperature (370 °C, 210 bar), but guaiacol remained a prominent product. The temperature also had an influence on the amount of aromatics formed. Higher temperature enhanced the aromatics yield as indicated by the relative area given. Up to 12% of the total lignin was converted into the four aromatics. A variation of the reaction time to 15 and to 25 minutes, tested at 350 °C, had only minor influence on the result (data not shown). The enrichment of the aromatics from the hydrothermal broth was straightforward, using steam distillation. Under the conditions chosen, this partly changed the ratios of the aromatics. Altogether, the total process provided an aqueous solution from the Kraft lignin, which contained larger amounts of guaiacol (7 g L⁻¹) and o-cresol (3 g L⁻¹), and smaller amounts of phenol (0.2 g L⁻¹).

### 2.4 The wildtype metabolizes the aromatics derived from depolymerized lignin

It was now interesting to see, if the *A*. *species* ATCC 39116 was able to utilize the mixture from the hydrothermal conversion. In a growth experiment, the mineral salt mixture of the basic medium was amended with the obtained softwood lignin hydrolysate and small amounts of glucose **(****Fig. 20 A)****.** Beautifully, the microbe started to grow on the hydrolysate from early on without any lag-phase. All aromatics were co-consumed. The degradation was fastest for guaiacol, followed by phenol and by o-cresol. Within about 9 hours, the three lignin-derived substrates were completely depleted. During this phase, glucose remained practically untouched, but was only taken up afterwards. As observed for pure guaiacol **(****Fig. 18****),** small levels of MA were formed. This proof-of-concept demonstrated the feasibility to couple a chemical conversion of lignin into aromatics to a biological conversion by the microbe. MA was not formed, requesting next for metabolic engineering.

### 2.5 Genetic engineering by conjugation and β-glucuronidase selection

The accumulation of MA in the broth **(****Fig. 18****)** and the observed growth on the aromatics mixture **(****Fig. 20A****)** was a promising indication that the microbe is capable to accumulate and, in particular, secrete the target product from lignin-based raw materials. In a next step, a strain with enhanced production performance should be constructed. Admittedly, genetic engineering of *A*. *species* ATCC 39116 is not straightforward (Meyer et al., 2017. Environ. Microbiol. 83.). Therefore, it was decided to establish a new cloning strategy for A. *species* ATCC 39116. First, conjugation was chosen for the transformation step involved. This approach turned out to be efficient. For each of the two gene deletions, conducted in this work (see below), several hundred apramycine resistant transformants from the conjugation were obtained on the selection agar plates within 2 days. Usually, about 50 colonies were observed on each of the 20 plates, inoculated. A blue white screening was done for confirmation of a successful first cross-over. In order to enable an unambiguous differentiation, the reporter substrate X-Gluc was not included into the agar plates, but used a second step for the screening. About 50 transformants were streaked in small squares of about 5 × 5 mm on new apramycine selection plates. Within 24 hours, the cells were grown into a clearly visible, dense cell layer **(****Figs. 21A**, D). The dropwise addition of X-Gluc onto each colony then, within only 20 minutes, provided an absolutely clear blue color signal in positive clones. Hereby, 100% of the tested colonies were found positive, indicating efficient selection by the apramycine. One of these was then picked, incubated overnight for the second recombination in liquid culture without selection pressure and then plated with X-Gluc. Within 24 hours, a large number of colonies was grown, i.e. 50 per plate after diluting the liquid culture 10⁶-fold. This provided a pre-screen of positive clones, which appeared white. In order to unambiguously verify the desired double-crossover, selected clones were again streaked out and tested after an additional 24 hour incubation by X-Gluc **(****Figs. 21B****,C,E,F).** The average frequency of the successful second recombination, clearly visible by the white color, was more than 15%. The analysis of white clones by colony PCR finally differentiated between wildtype and mutant. The success rate for the desired deletion, among all tested white clones, was about 50%. Taken together, the new protocol enabled a marker-free genomic deletion in about one week.

### 2.6 Deletion of the putative muconate cycloisomerase does not functionally disrupt the β-ketoadipate pathway

Three genes were identified in the genome of *A. species* ATCC 39116 with demonstrated or predicted muconate cycloisomerase activity, using a BLAST search **(Table 10).** The deletion of the gene AATC3_020100009302 was done first, using the integrative plasmid pKG1132 MUC-1 (**Fig. 24B**). The established cloning workflow allowed for a clear identification of the desired recombination events. A number of single crossover clones, which had integrated the plasmid pKG1132 MUC-1 into their genome was selected via the applied apramycine selection pressure. The desired single crossover was confirmed by blue-white screening (**Fig. 21A**). All clones were found positive as expected. Subsequent incubation without selection pressure allowed for the double crossover, which was visualized by white colony color (**Figs. 21B****,C**). All white clones were then tested by colony PCR for the desired deletion, which was found at 50% frequency. The total deletion took only about one week. The deletion mutant, designated *A*. *species* ATCC 39116 MUC-1, however, did not reveal any significant change in growth and production physiology, but behaved like the wildtype, when grown on guaiacol **(****Fig. 26****).**

### 2.7 Additional deletion of the muconate cycloisomerase gene AATC3_020100018510 enables efficient MA formation from guaiacol

The deletion of the gene AATC3_020100009302 did not lead to enhanced MA production. On a first glance, it seemed that the protein did not play a major role in the β-ketoadipate pathway, but compensating effects by the remaining other two candidates could not be excluded either. Therefore, the second deletion was realized in the MUC-1 background. The AATC3_020100018510 gene was deleted. Again, the genomic modification turned out to be straightforward. Several clones were found positive after the conjugation **(****Fig. 21D****).** The subsequent incubation of the mutants without selection pressure resulted in a number of white clones, which had lost the plasmid backbone (**Figs. 21E****,F**). The double cross over mutants occurred at a frequency of about 15%. About half of the white colonies had lost the target gene, as desired. The double deletion strain, verified by PCR, was designated MUC-2. In contrast to wild type and the MUC-1 mutant, the MUC-2 strain accumulated huge amounts of MA from guaiacol **(****Fig. 22****).** Within about 10 hours, the aromatic (5 mM) was converted at a high yield of 0.8 mol mol⁻¹ into MA. This matched a 16-fold improvement, as compared to the wildtype. Glucose, also present in the medium, was consumed only slightly, and the cells showed weak growth.

### 2.8 The metabolically engineered strain MUC-2 accumulates MA from guaiacol in the gram scale

The MA production performance of the novel mutant from guaiacol was now evaluated in a bioreactor, operated in fed-batch mode (**Figs. 23A****,B,C**). In short, an initial batch phase with 5 mM guaiacol was followed by an extended feeding phase, which added further amounts of the aromatic substrate. Overall, the strain produced 3.1 g L⁻¹ (25 mM) MA from guaiacol within 24 hours. The conversion yield remained stable at 96% (0.96 mol mol⁻¹). Immediately upon process start, the cells converted guaiacol into MA. The initial amount of the substrate was consumed after 5 hours. Guaiacol was then added pulse-wise. Inferred from the linear decrease of the guaiacol level, measured at-line by HPLC, the time point of substrate depletion could be exactly predicted. In addition, the HPLC analysis also covered the pathway intermediate catechol. This allowed a precise control throughout the whole process: higher accumulation of the toxic compounds as well as phases of substrate limitation could be avoided. During the first hours of the feed phase the cells continued to convert guaiacol into MA, which was accompanied by significant glucose consumption and cell growth (**Figs. 23A**,B). However, a certain fraction of guaiacol was only partly converted and accumulated as catechol up to a level of about 2mM (10 hours). In order to avoid further accumulation of the toxic intermediate the subsequent feed-periods were slightly extended. Accordingly, the catechol level quickly dropped and remained in a negligible range towards the end of the process. Remarkably, the cells did not reveal any disturbance in MA production by the transient accumulation.

### 2.9 The metabolically engineered strain MUC-2 converts softwood lignin hydrolysate into MA at 72% yield

Next, the production performance of the engineered double deletion mutant was investigated directly on the lignin hydrolysate **(****Fig. 20 B)****.** The strain efficiently converted the lignin aromatics into MA. Again, all aromatics were co-consumed. Within 10 hours, 1.8 mM MA was formed. Considering the total amount of the three aromatics substrates (2.5 mM), the formation of the product occurred at a yield of 0.72 mol mol⁻¹. The cell growth was weaker, as compared to the wildtype **(****Fig. 20A****),** which was obviously linked to the redirection of the aromatics towards MA. The level of MA remained stable also in later production phases, when the aromatics were depleted. Taken together, the mutant revealed high-yield production of MA from lignin. The production efficiency, inferred from the final MA titer, was enhanced more than six-fold by the genomic modification.

### 2.10 The metabolically engineered producer accumulates methyl-MA from o-cresol

As shown, *A. species* ATCC 39116 was capable to utilize o-cresol. In contrast to the other aromatics tested here, o-cresol possesses a methyl group at the aromatic ring. It appeared unlikely, that this rather inactive chemical group is removed from the molecule during the conversion, deserving further analysis.
*A. species* ATCC 39116 did not accumulate MA from o-cresol. However, the HPLC analysis revealed an unknown substance, which was not present in the initial medium, but accumulated during the process (data not shown). The corresponding peak eluted later than MA, suggesting a slightly more hydrophobic nature. Very similar to MA, but different to all tested aromatics, the UV absorption of the new metabolite was higher at 260 nm than at 220 nm. Obviously, the substance was non-aromatic. Subsequently, GC-MS analysis of pure MA and of lyophilized culture supernatant from a culture on o-cresol was conducted. The t-butyldimethylsilyl-derivatised MA, eluting after 9.5 minutes revealed ion clusters at m/z 370 [M], 355 [M-15], 313 [M-57], and 285 [M-85] (**Fig. 24A**), which matched the typical fragmentation pattern for this type of derivate (Wittmann, C., 2007. Cell Fact. 6, 6.). The fragmentation spectrum of the novel metabolite from o-cresol, eluting after 9.7 minutes, resembled that of MA, however, with a mass shift of 14 in all specific fragment ions (**Fig. 24B**). This suggested that the new metabolite was structurally similar to MA, and additionally contained a CH₂-group. The fact that the molecule was derivatised at both carboxyl groups, indicated that the additional group was located in the inner hydrocarbon chain. It was concluded that the metabolite was methyl-muconate (methyl-MA), likely 2-methyl MA given the neighboring positions of the methyl group and the hydroxyl group in o-cresol. It was now interesting to see, how this product was actually derived. For this purpose, the substrate specificity of the central MA forming enzyme catechol-dioxygenase (CatA) was studied. A control assay with catechol revealed substantial CatA activity of 640 mU mg⁻¹ (**Table 11**). In an additional experiment, catechol was replaced by 3-methyl catechol, which mimicked the methylated pathway intermediate that was expected from o-cresol. The cell extract was able to convert 3-methyl catechol at about 70% of the activity, found for catechol. An additional experiment revealed that the enzyme also accepted 4-catechol as substrate. Taken together, the engineered producer was able to form a novel product, likely 2-methyl MA, from o-cresol.

**Table 9: Utilization of aromatics and their degradation products by Amycolatopsis sp. ATCC 39116 as sole source of carbon. Each set-up contained 2 mM of the corresponding substrate in mineral salt medium. In addition, a control without added substrate was conducted. Growth was monitored as optical density (OD₆₀₀) after 40 hours and is given as follows: OD > 1, +++; 1 > OD > 0.5, ++; 0.5 > OD > 0.25, +; OD < 0.25, -.**

| **Aromatics** | **Growth** |
|---|---|
| *p*-Coumaric acid | + + + |
| *Cis,cis*-muconic acid | + + + |
| Benzoic acid | + + + |
| Guaiacol | + + |
| Catechol | + + |
| Phenol | + + |
| Toluene | + |
| Control | - |

**Table 10: Genes encoding for enzymes with demonstrated and putative muconate cycloisomerase activity in the genome of Amycolatopsis sp. ATCC 39116. The function of AATC3_020100018510 was previously demonstrated (Park, et al., 2003. FEMS Microbiol. Lett. 226, 151-7.), whereas the other two candidates have not been investigated so far. These were inferred by sequence similarity to genes, annotated as muconate cycloisomerases or muconate lactonizing proteins, respectively.**

| **Gene** | **GC content (%)** | **Sequence similarity, Organism** |
|---|---|---|
| AATC3_020100001440 | 71.6 | 86%, *Saccharopolyspora erythraea* NRRL2338 |
| | | 82%, *Nocardiopsis dassonvillei* strain NOCA502F |
| | | 81%, *Geodermatophilus obscurus* DSM 43160 |
| | | 80%, *Streptomyces pactum strain* KLBMP 5084 |
| AATC3_020100009302 | 70.8 | 96%, *Amycolatopsis methanolica* 239 |
| | | 81%, *Saccharopolyspora erythraea* NRRL2338 |
| AATC3_020100018510 | 70.5 | 96%, *Amycolatopsis methanolica* 239 |
| | | 90%, *Saccharopolyspora erythraea* NRRL2338 |

**Table 11: Substrate specificity of catechol dioxygenase in the cell extract of Amycolatopsis species ATCC 39116. The data reflect mean values and deviations from three replicates.**

| **Substrate** | **Specific activity [mU mg⁻¹]** |
|---|---|
| Catechol | 640 ± 19 |
| 3-Methyl catechol | 470 ± 9 |
| 4-Methyl catechol | 380 ± 5 |

**Table 12: Strains and plasmids used in this study.**

| | Description | Reference |
|---|---|---|
| **Strains** | | |
| *E. coli* DH5α | Heat shock competent cells for the amplification of the transformation vector conjugative transfer of DNA | Invitrogen |
| *E. coli ET12567 (pUZ8002)* | | (Kieser et al., 2000. The John Innes Foundation, Norwich, UK.) ATCC |
| *A*. *species* ATCC 39116 | Wild type | |
| *A*. *species* ATCC 39116 MUC1 | Deletion of the putative *catB* gene AATC3_02010009302 gene (G10GW-1735) Deletion of the *catB* gene AATC3_02010018510 gene (G10GW-3575) | This work |
| *A*. *species* ATCC 39116 MUC2 | | This work |

| **Plasmids** | | |
|---|---|---|
| pKC1132 | Integrative vector for genome-based modifications, comprising a MCS for A. *species,* an ORI for E. *coli,* and Am^{R} as selection marker. | (Bierman et al., 1992. Gene. 116, 43-9.) |
| pKG1132 | Integrative vector for genome-based modifications, comprising a MCS for A. *species,* an ORI for *E*. *coli,* and Am^{R} and a gusA gene as selection markers. | This work |
| pKG1132 MUC1 | Vector for the deletion of the putative *catB* gene AATC3_02010009302 gene (G10GW-1735) | This work |
| pKG1132 MUC2 | Vector for the deletion of the *catB* gene AATC3_02010018510 gene (G10GW-3575) | This work |

**Table 13: Primers used in the present work for genomic engineering of Amycolatopsis sp. ATCC 39116.**

| No. | Sequence | T^{A} [C°] |
|---|---|---|
| PR17 | GATCCGCGGCCGCGCGCGATCACGAACCGGCGGATCAG (SEQ ID No. 126) | 65 |
| PR18 | AAGGGAGTGGGTCCGTGTGAGGTTCCGGGCCCGTTCGCG (SEQ ID No. 127) | 68 |
| PR19 | CGAACGGGCCCGGAACCTCACACGGACCCACTCCCTTG (SEQ ID No. 128) | 64 |
| PR20 | GACATGATTACGAATTCGATCGACCACGCTCAGCGCCTTC (SEQ ID No. 129) | 70 |
| PR40 | GATCCGCGGCCGCGCGCGATCGCCCTTGTTCGGGTAGTAG (SEQ ID No. 130) | 64 |
| PR45 | GACATGATTACGAATTCGATGACGCTCCACTCGTTTC (SEQ ID No. 131) | 59 |
| PR53 | CTTGATATCCGGGGGAGTTTCACATCGGCTGACCTCGTGTGGTTG (SEQ ID No. 132) | 66 |
| PR54 | CCCCCAACCACACGAGGTCAGCCGATGTGAAACTCCCCCGGATATCAAGG (SEQ ID No. 133) | 65 |

### References

- Azadi P., Inderwildi O.R., Farnood R., King D.A. Liquid fuels, hydrogen and chemicals from lignin: a critical review. Renew. Sust. Energ. Rev. 21, (2013) 506-523.
- Bagdasarian M., Lurz R., Ruckert B., Franklin F.C., Bagdasarian M.M., Frey J., Timmis K.N. Specific-purpose plasmid cloning vectors. II. Broad host range, high copy number, RSF1010-derived vectors, and a host-vector system for gene cloning in Pseudomonas. Gene 16, (1981) 237-247.
- Beckham, G., Johnson, C. & M Karp, E., Salvachúa, D., Vardon, D. (2016). Opportunities and challenges in biological lignin valorization. Current Opinion in Biotechnology. 42. 40-53.
- Black B.A., Michener W.E., Ramirez K.J., Biddy M.J., Knott B.C., Jarvis M.W., Olstad J., Mante O.D., Dayton D.C., Beckhamm G.T. Aquaous stream characterization from biomass fast pyrolsis and catalytic fast pyrolysis. ACS Sustainable Chem. Eng. In Press.
- Bridgwater A.V. Review of fast pyrolysis of biomass and product upgrading. Biomass Bioenerg. 38, (2012) 1306-1316.
- Butler E., Devlin G., Meier D., McDonnell K. A review of recent laboratory research and commercial developments in fast pyrolysis and upgrading. Renew. Sust. Energ. Rev. 15 (2011) 4171-4186.
- Chavarria M., Nikel P.I., Pérez-Pantoja D., Lorenzo V. The Entner-Doudoroff pathway empowers Pseudomonas putida KT2440 with a high tolerance to oxidative stress. Environ. Microbiol. 15, (2013) 1772-1785.
- S., Barakat A., Robitzer M., Di Renzo F., Dumas C., Quignard F. Composition, texture and methane potential of cellulosic residues from Lewis acids organosolv pulping of wheat straw. Bioresour. Technol. 216, (2016) 737-743.
- Davis et al., J. Bacteriol. May 2012 vol. 194 no. 9 2396-2397
- De Lorenzo V., Timmis K.N. Analysis and construction of stable phenotypes in Gram-negative bacteria with Tn5 and Tn10- derived transposons. Meth. Enzymol. 235, (1994) 386-405.
- De Wild P.J., Huijgen W.J.J., Heeres H.J. Pyrolysis of wheat straw-derived organosolv lignin. J. Anal. Appl. Pyrol. 93 (2012) 95-103.
- De Wild P.J., den Uil H., Reith J.H., Kiel J.H.A., Heeres H.J. Biomass valorization by staged degasification: A new pyrolysis-based thermochemical conversion option to produce value-added chemicals from lignocellulosic biomass. J. Anal. Appl. Pyrol. 85, (2009) 124-133.
- De Wild P.J., Reith H., Heeres H.J. Biomass Pyrolysis for chemicals. Biofuels 2, (2011) 185-208.
- De Wild P.J., Huijgen W.J., Gosselink R.J. Lignin pyrolysis for profitable lignocellulosic biorefineries. Biofuels Bioprod. Bioref. 8, (2014) 645-657.
- Gosselink R.J.A., De Jong E., Guran B., Abächerli A. Co-ordination network for lignin-standardisation, production and applications adapted to market requirements (EUROLIGNIN). Ind. Crops Prod. 20, (2004) 121-129.
- Greenhalf C.E., Nowakowski D.J., Harms A.B., Titiloye J.O., Bridgwater A.V. Sequential pyrolysis of willow SRC at low and high heating rates - Implications for selecetive pyrolysis. Fuel 93 (2012) 692-702.
- Hanahan D., Meselson M. Plasmid screening at high colony density. Methods Enzymol. 100, (1983) 333-342.
- Hartmans S., J.P., M.J., Volkering F., de Bont J.A.M. Metabolism of Styrene Oxide and 2-Phenylethanol in the Styrene-Degrading Xanthobacter Strain 124X. Appl. Environ. Microbiol. 55, (1989) 2850-2855.
- Hellinger M., Carvalho H.W.P., Baier S., Wang D., Kleist W., Grunwaldt J.D. Catalytic hydrodeoxygenation of guaiacol over platinum supported on metal oxides and zeaolites. Appl. Catal. A 490, (2015) 181-192.
- Jiang L., Wu N., Zheng A., Zhao Z., He F., Li H. The integration of dilute acid hydrolysis of xylan and fast pyrolysis of glucan to obtain fermentable sugars. Biotechnol. Biofuels 9 (2016) 196.
- Jiménez J.I., Miñambres B., Garcia E., Diaz J.L. Genomic analysis of the aromatic catabolic pathways from Pseudomonas putida KT2440. Environ. Microbiol. 4, (2002) 824:841.
- Kalil M.S., Asyigin Z., Zaki M. Catechol synthesis via demethylation of guaiacol by anaerobic bacterium Acetobacterium woodii DSM 1030. Pak. J. Biol. Sci. 5, (2002) 1186-1188.
- Kim J.S. Production, separation and applications of phenolic-rich bio-oil-A review. Bioresour. Technol. 178, (2015) 90-98.
- Larsson E.D. Biomass gasification systems for electric power, cogeneration, liquid fuels, and hydrogen. (2004) GCEP Biomass Energy Workshop Stanford University.
- Laurichesse S., Averous L. Chemical modification of lignins: Towards biobased polymers. Prog. Polym. Sci. 39, (2014) 1266-1290.
- Lawson J.R., Klein M.T. Influence of Water on Guaiacol Pyrolysis. Ind. Eng. Chem. Fundam. 24, (1985) 203-208.
- Martinez-Garcia E., de Lorenzo, V. Engineering multiple genomic deletions in Gram-negative bacteria: analysis of the multi-resistant antibiotic profile of Pseudomonas putida KT2440. Environ. Microbiol. 13, (2011) 2702-2716.
- Merchant Research & Consulting Ltd., 2011. Adipic Acid 2011 World Market Outlook and Forecast
- Mohan D., Pittman C.U., Steele P.H. Pyrolysis of Wood/Biomass for Bio-oil: A Critical Review. Energy Fuels 2006, 20 (3), 848-889.
- Nelson K.E., Weinel C., Paulsen I.T., Dodson R.J., Hilbert H., Martins dos Santos V.A.P.ef al,. Complete genome sequence and comparative analysis of the metabolically versatile Pseudomonas putida KT2440. Environ. Microbiol. 4 (2002), 799-808.
- Nikel P.I., Martinez-Garcia E., de Lorenzo V. Biotechnological domestication of pseudomonads using synthetic biology. Nat. Rev. Microbiol. 12, (2014) 368-379.
- Niu W., Draths K.M., Frost J.W. Benzene-free synthesis of adipic acid. Biotechnol. Prog. 18, (2002) 201-211.
- Nordlund I., Powlowski J., Shingler V. Complete nucleotide sequence and polypeptide analysis of multicomponent phenol hydroxylase from Pseudomonas sp. strain CF600. J. Bacteriol. 172 (1990) 6826-6833.
- Pavel H., Forsman M., Shingler V. An aromatic effector specificity mutant of the transcriptional regulator DmpR overcomes the growth constraints of Pseudomonas sp. strain CF600 on para-substituted methylphenols. J. Bacteriol. 176 (1994) 7550-7557.
- Pollard A.S., Rover M.R., Brown R.C. Characterization of bio-oil recovered as stage fractions with unique chemical and physical properties. J. Anal. Appl. Pyrol. 93 (2012) 129-138.
- Rahimi A., Ulbrich A., Coon J.J., Stahl S.S. Formic-acid-induced depolymerization of oxidized lignin to aromatics. Nature 515, (2014) 249-252.
- Remón J., Broust F., Valette J., Chhiti, Y., Alava, I., Fernandez-Akarregi A.R., Arauzo J., Garcia L. Production of a hydrogen-rich gas from fast pyrolysis bio-oils: Comparison between homogeneous and catalytic steam reforming routes. Int. J. Hydrogen Energy 39, (2014) 171-182.
- Sambrook J., Maniatis T., Fritsch E.F. Molecular Cloning: A Laboratory Manual. Cold Spring Harbor, NY, USA: Cold Spring Harbor Laboratory Press (1989).
- Shingler V., Franklin F.C.H., Tsuda M., Holroyd D., Bagdasarian M. Molecular analysis of a plasmid-encoded phenol hydroxylase from Pseudomonas CF600. J. Gen. Microbiol. 135, (1989) 1083-1092.
- Sipilä K., Kuoppala E., Fagernas L., Oasmaa A. Characterization of biomass-based flash pyrolysis oils. Biomass Bioenergy 14, (1998) 103-113.
- Tomani P. The Lignoboost process. Cellulose Chem. Technol. 44, (2010) 53-55.
- Townsend S.H., Abraham M.A., Huppert G.L., Klein M.T., Paspek S.C. Solvent Elffects During Reactions in Supercritical Water. Ind. Eng. Chem. Res. 27, (1988) 143-149.
- Tuck C.O., Pérez E., Horváth I.T., Sheldon R.A., Poliakoff M. Valorization of biomass: deriving more value from waste. Science 337, (2012) 695-699.
- Valle B., Remiro A., Aguayo A.T., Bilbao J., Gayubo A.G. Catalysts of Ni/α-Al2O3 and Ni/La2O3-α Al2O3 for hydrogen production by steam reforming of bio-oil aqueous fraction with pyrolytic lignin retention. Int. J. Hydrogen Energy 38, (2013) 1307-1318.
- van Duuren J.B., Pucha 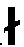 ka J., Mars A.E., Bücker R., Eggink G., Wittmann C., dos Santos V.A.M. Reconciling *in vivo* and *in silico* key biological parameters of *Pseudomonas putida* KT2440 during growth on glucose under carbon-limited condition. BMC biotechnol. 13, (2013) 93.
- Vardon D.R., Franden M.A., Johnson C.W., Karp E.M., Guarnieri M.T., Linger J.G., Salm M.J., Strathmann T.J., Beckhamm G.T. Adipic acid production from lignin. Energy Environ. Sci. 8, (2015), 617-628.
- Vishtal A.G., Kraslawski A. Challenges in industrial applications of technical lignins. BioRes. 6, (2011) 3547-3568.
- Vispute T.P., Huber G.W. Production of hydrogen, alkanes and polyols by aqueous phase processing of wood-derived pyrolysis oils. Green Chem. 11, (2009) 1433 -1445.
- Vispute T.P., Zhang H., Sanna A., Xiao R., Huber G.W. Renewable Chemical Commodity Feedstocks from Integrated Catalytic Processing of Pyrolysis Oils. Science 330, (2010) 1222-1227
- Wildschut J, Smit AT, Reith JH, Huijgen WJJ. Ethanol-based organosolv fractionation of wheat straw for the production of lignin and enzymatically digestible cellulose. Bioresour. Technol. 135, (2013) 58-66.
- Wong S.M., Mekalanos J.J. Genetic footprinting with mariner-based transposition in Pseudomonas aeruginosa. Proc. Natl. Acad. Sci. USA 97, (2000) 10191-10196.
- Yildiz G, Ronsse F, van Duren R, Prins W. Challenges in the design and operation of processes for catalytic fast pyrolysis of woody biomass. Renew. Sust. Energ. Rev. 57, (2016) 1596-1610.
- Beerthuis R, Rothenberg G, Shiju NR (2015) Green Chemistry 17 (3):1341-1361.
- Coudray L, Bui V, Frost JW, Schweitzer D (2012) WO2012141997,
- Burk MJ, Oosterhout RE, Sun J (2011) WO2011017560A1
- Frost JW, Miermont A, Schweitzer D, Bui V (2010) WO2010148049A2

## Claims

1. A method of producing a phenolic compound, comprising the steps of
i) Subjecting lignin to a fast pyrolysis process;
ii) Condensing the resulting pyrolysis vapors by directly introducing the resulting pyrolysis vapors into at least one aqueous phase, resulting in at least one acquatic phase comprising a phenolic compound and at least one organic phase;
iii) Separating the resulting at least one acquatic phase comprising a phenolic compound, and the resulting at least one organic phase.

2. The method of claim 1, wherein the phenolic compound is catechol.

3. A method of producing cis-cis muconic acid, comprising the steps i) to iii) according to claim 1 or 2 and further comprising the steps of
iiia) Optionally purifying catechol of the acquatic phase;
iv) Subjecting at least one acquatic phase comprising catechol of step iii) or purified catechol of step iiia) of said fast pyrolysis process to a biocatalytic conversion by contacting said acquatic phase with a biocatalyst, wherein said biocatalyst is a host cell which expresses an enzyme showing catechol-1,2-dioxygenase activity (EC 1.13.11.1) (catA) to prepare cis-cis muconic acid;
iva) Optionally purifying cis-cis muconic acid from the biocatalyst.

4. A method of producing adipic acid, comprising the steps i) to iii) according to claim 1 or 2 and further comprising the steps of
iiia) Optionally purifying catechol of the acquatic phase;
iv) Subjecting at least one acquatic phase comprising catechol of step iii) or purified catechol of step iiia) of said fast pyrolysis process to a biocatalytic conversion by contacting said acquatic phase with a biocatalyst, wherein said biocatalyst is a host cell which expresses an enzyme showing catechol-1,2-dioxygenase activity (EC 1.13.11.1) (catA) to prepare cis-cis muconic acid;
iva) Optionally purifying cis-cis muconic acid from the biocatalyst;
v) Preparing adipic acid by hydrogenation of cis-cis muconic acid.

5. A method of producing Nylon, comprising the steps i) to iii) according to claim 1 or 2 and further comprising the steps of
iiia) Optionally purifying catechol of the acquatic phase;
iv) Subjecting at least one acquatic phase comprising catechol of step iii) or purified catechol of step iiia) of said fast pyrolysis process to a biocatalytic conversion by contacting said acquatic phase with a biocatalyst, wherein said biocatalyst is a host cell which expresses an enzyme showing catechol-1,2-dioxygenase activity (EC 1.13.11.1) (catA) to prepare cis-cis muconic acid;
iva) Optionally purifying cis-cis muconic acid from the biocatalyst;
v) Preparing adipic acid by hydrogenation of cis-cis muconic acid;
vi) Reacting hexamethylenediamine and adipic acid, preferably equivalent amounts of hexamethylenediamine and adipic acid, with water, preferably in a reactor;
vii) Crystallizing the resulting product to produce nylon salt;
viii) Polymerize the nylon salt to nylon 6,6 in a reaction vessel, preferably either in batches or continuously.

6. A method of producing caprolactam comprising the steps i) to iii) according to claim 1 or 2 and further comprising the steps of
iiia) Optionally purifying catechol of the acquatic phase;
iv) Subjecting at least one acquatic phase comprising catechol of step iii) or purified catechol of step iiia) of said fast pyrolysis process to a biocatalytic conversion by contacting said acquatic phase with a biocatalyst, wherein said biocatalyst is a host cell which expresses an enzyme showing catechol-1,2-dioxygenase activity (EC 1.13.11.1) (catA) to prepare cis-cis muconic acid;
iva) Optionally purifying cis-cis muconic acid from the biocatalyst;
ix) Chemically converting cis-cis muconic acid to caprolactam by using a catalyst, preferably an alumina-catalyst in a solvent at a reaction temperature between 200 °C and 350 °C.

7. A method of producing terephthalic acid comprising the steps i) to iii) according to claim 1 or 2 and further comprising the steps of
iiia) Optionally purifying catechol of the acquatic phase;
iv) Subjecting at least one acquatic phase comprising catechol of step iii) or purified catechol of step iiia) of said fast pyrolysis process to a biocatalytic conversion by contacting said acquatic phase with a biocatalyst, wherein said biocatalyst is a host cell which expresses an enzyme showing catechol-1,2-dioxygenase activity (EC 1.13.11.1) (catA) to prepare cis-cis muconic acid;
iva) Optionally purifying cis-cis muconic acid from the biocatalyst;
x) Conversion of cis-cis muconic acid to *trans, trans*-muconic acid in the presence of a catalyst, preferably I₂;
xi) Reacting *trans, trans*-muconic acid and acetylene via a Diels-Alder reaction to cyclohexa-2,5-diene-1,4-dicarboxylate (PI);
xii) Oxygenating of PI to terephthalic acid.

8. The method according to any one of the preceding claims, wherein the biocatalyst is selected from the group consisting of bacteria, yeast, filamentous fungi, cyanobacteria, algae, and plant cells.

9. The method of any of claims 3 to 8, wherein said host cell comprises at least one, optionally heterologous, gene encoding a polypeptide having catechol 1,2-dioxygenase activity.

10. The method of any of claims 3 to 9, wherein said host cell comprises at least one, optionally heterologous, catA gene, wherein said at least one, optionally heterologous, catA gene encodes a polypeptide comprising a sequence corresponding to SEQ ID No. 1, 108 or 122.

11. The method of any of claims 3 to 10, wherein said host cell comprises, operably linked to the at least one, optionally heterologous, gene, a promoter sequence corresponding to
vi) SEQ ID No. 5 [Pem7]; or
vii) SEQ ID No. 6 [Pem7*]; or
viii) SEQ ID No. 7 [PtufJ; or
ix) SEQ ID No. 8 [PrpoD]; or
x) SEQ ID No. 9 [Plac]; or
xi) SEQ ID No. 10 [PgyrB];
xii) SEQ ID No. 11; or
xiii) SEQ ID No. 12; or
xiv) SEQ ID No. 13; or
xv) SEQ ID No. 14; or
xvi) SEQ ID No. 15; or
xvii) SEQ ID No. 16; or
xviii) SEQ ID No. 88 [Ptuf_1]; or
xix) SEQ ID No. 89 [Ptuf_short]; or
xx) SEQ ID No. 90 [Ptuf_s_2]; or
xxi) SEQ ID No. 91 [Ptuf_s_3]; or
xxii) SEQ ID No. 92 [Ptuf_s_4]; or
xxiii) SEQ ID No. 93 [Ptuf_s_5]; or
xxiv) SEQ ID No. 94 [Ptuf_s_6]; or
xxv) SEQ ID No. 95 [Ptuf_s_7]; or
xxvi) SEQ ID No. 96 [Ptuf_s_8]; or
xxvii) SEQ ID No. 97 [Ptuf_s_9]; or
xxviii)SEQ ID No. 98 [Ptuf_s_10]; or
xxix) SEQ ID No. 99 [Ptuf_s_11]; or
xxx) SEQ ID No. 100 [Ptuf_s_12]; or
xxxi) SEQ ID No. 101 [Pgro]; or
xxxii) SEQ ID No. 102 [Pgro_1]; or
xxxiii)SEQ ID No. 103 [Pgro_2]; or
xxxiv)SEQ ID No. 104 [Pgro_4]; or
xxxv) SEQ ID No. 105 [Pgro_5];
preferably SEQ ID No. 5 [Pem7].

12. The method of any of claims 3 to 11, wherein said host cell is selected from *Pseudomonas,* preferably *Pseudomonas putida,* more preferably *Pseudomonas putida* strain KT2440, strain KT2440 JD2S or strain KT2440 BN6, even more preferably *Pseudomonas putida* strain KT2440 BN6.

13. The method of any of claims 3 to 12, wherein the host cell expresses a functional catA polypeptide and does not express a functional catB polypeptide.

14. The method of claim 13, wherein the host cell which expresses a functional catA polypeptide, said catA polypeptide being **characterized in that** it has
(a) the amino acid sequence shown in SEQ ID No. 108; or
(b) an amino acid sequence which has at least 40% identity to the amino acid sequence shown in SEQ ID No. 108 and having catechol-1,2-dioxygenase activity;
and does not express a functional catB polypeptide, said catB polypeptide being **characterized in that** it has
(c) the amino acid sequence shown in SEQ ID No. 109; or
(d) an amino acid sequence which has at least 25% identity to the amino acid sequence shown in SEQ ID No. 109 and having muconate cycloisomerase activity.

15. The method of claim 14, wherein the bacterial host cell is Corynebacterium glutamicum ATCC13032; or wherein the bacterial host cell is Amycolatopsis sp. ATCC39116.

## Patentansprüche

1. Verfahren zum Herstellen einer Phenolverbindung, umfassend die folgenden Schritte
i) Unterziehen von Lignin einem Schnellpyrolyseprozess;
ii) Kondensieren der entstandenen Pyrolysedämpfe durch direktes Einleiten der entstandenen Pyrolysedämpfe in mindestens eine wässrige Phase, was zu mindestens einer aquatischen Phase, die eine Phenolverbindung umfasst, und mindestens einer organischen Phase führt;
iii) Trennen der resultierenden mindestens einen aquatischen Phase, die eine Phenolverbindung umfasst, und der resultierenden mindestens einen organischen Phase.

2. Verfahren nach Anspruch 1, wobei die Phenolverbindung Catechol ist.

3. Verfahren zum Herstellen von cis-cis-Muconsäure, umfassend die Schritte i) bis iii) nach Anspruch 1 oder 2 und ferner umfassend die folgenden Schritte
iiia) Gegebenenfalls Reinigen des Catechols der aquatischen Phase;
iv) Unterziehen mindestens einer aquatischen, Catechol umfassenden Phase aus Schritt iii) oder von gereinigtem Catechol aus Schritt iiia) aus dem Schnellpyrolyseprozess einer biokatalytischen Umwandlung durch Inkontaktbringen der aquatischen Phase mit einem Biokatalysator, wobei der Biokatalysator eine Wirtszelle ist, die ein Enzym exprimiert, das Catechol-1,2-dioxygenase-Aktivität (EC 1.13.11.1) (catA) zeigt, um cis-cis-Muconsäure herzustellen;
iva) Gegebenenfalls Reinigen der cis-cis-Muconsäure von dem Biokatalysator.

4. Verfahren zum Herstellen von Adipinsäure, umfassend die Schritte i) bis iii) nach Anspruch 1 oder 2 und ferner umfassend die folgenden Schritte
iiia) Gegebenenfalls Reinigen des Catechols der aquatischen Phase;
iv) Unterziehen mindestens einer aquatischen, Catechol umfassenden Phase aus Schritt iii) oder von gereinigtem Catechol aus Schritt iiia) aus dem Schnellpyrolyseprozess einer biokatalytischen Umwandlung durch Inkontaktbringen der aquatischen Phase mit einem Biokatalysator, wobei der Biokatalysator eine Wirtszelle ist, die ein Enzym exprimiert, das Catechol-1,2-dioxygenase-Aktivität (EC 1.13.11.1) (catA) zeigt, um cis-cis-Muconsäure herzustellen;
iva) Gegebenenfalls Reinigen der cis-cis-Muconsäure von dem Biokatalysator;
v) Herstellen von Adipinsäure durch Hydrierung von cis-cis-Muconsäure.

5. Verfahren zum Herstellen von Nylon, umfassend die Schritte i) bis iii) nach Anspruch 1 oder 2 und ferner umfassend die folgenden Schritte
iiia) Gegebenenfalls Reinigen des Catechols der aquatischen Phase;
iv) Unterziehen mindestens einer aquatischen, Catechol umfassenden Phase aus Schritt iii) oder von gereinigtem Catechol aus Schritt iiia) aus dem Schnellpyrolyseprozess einer biokatalytischen Umwandlung durch Inkontaktbringen der aquatischen Phase mit einem Biokatalysator, wobei der Biokatalysator eine Wirtszelle ist, die ein Enzym exprimiert, das Catechol-1,2-dioxygenase-Aktivität (EC 1.13.11.1) (catA) zeigt, um cis-cis-Muconsäure herzustellen;
iva) Gegebenenfalls Reinigen der cis-cis-Muconsäure von dem Biokatalysator;
v) Herstellen von Adipinsäure durch Hydrierung von cis-cis-Muconsäure;
vi) Umsetzen von Hexamethylendiamin und Adipinsäure, vorzugsweise äquivalenter Mengen von Hexamethylendiamin und Adipinsäure, mit Wasser, vorzugsweise in einem Reaktor;
vii) Auskristallisieren des entstandenen Produkts, um Nylonsalz herzustellen;
viii) Polymerisieren des Nylonsalzes zu Nylon-6,6 in einem Reaktionsgefäß, vorzugsweise entweder chargenweise oder kontinuierlich.

6. Verfahren zum Herstellen von Caprolactam, umfassend die Schritte i) bis iii) nach Anspruch 1 oder 2 und ferner umfassend die folgenden Schritte
iiia) Gegebenenfalls Reinigen des Catechols der aquatischen Phase;
iv) Unterziehen mindestens einer aquatischen, Catechol umfassenden Phase aus Schritt iii) oder von gereinigtem Catechol aus Schritt iiia) aus dem Schnellpyrolyseprozess einer biokatalytischen Umwandlung durch Inkontaktbringen der aquatischen Phase mit einem Biokatalysator, wobei der Biokatalysator eine Wirtszelle ist, die ein Enzym exprimiert, das Catechol-1,2-dioxygenase-Aktivität (EC 1.13.11.1) (catA) zeigt, um cis-cis-Muconsäure herzustellen;
iva) Gegebenenfalls Reinigen der cis-cis-Muconsäure von dem Biokatalysator;
ix) Chemisches Umwandeln von cis-cis-Muconsäure in Caprolactam unter Verwendung eines Katalysators, vorzugsweise eines Aluminiumoxidkatalysators, in einem Lösungsmittel bei einer Reaktionstemperatur zwischen 200°C und 350°C.

7. Verfahren zum Herstellen von Terephthalsäure, umfassend die Schritte i) bis iii) nach Anspruch 1 oder 2 und ferner umfassend die folgenden Schritte
iiia) Gegebenenfalls Reinigen des Catechols der aquatischen Phase;
iv) Unterziehen mindestens einer aquatischen, Catechol umfassenden Phase aus Schritt iii) oder von gereinigtem Catechol aus Schritt iiia) aus dem Schnellpyrolyseprozess einer biokatalytischen Umwandlung durch Inkontaktbringen der aquatischen Phase mit einem Biokatalysator, wobei der Biokatalysator eine Wirtszelle ist, die ein Enzym exprimiert, das Catechol-1,2-dioxygenase-Aktivität (EC 1.13.11.1) (catA) zeigt, um cis-cis-Muconsäure herzustellen;
iva) Gegebenenfalls Reinigen der cis-cis-Muconsäure von dem Biokatalysator;
x) Umwandlung von cis-cis-Muconsäure in *trans,trans-Muconsäure* in Gegenwart eines Katalysators, vorzugsweise I₂;
xi) Umsetzen von *trans,trans*-Muconsäure und Acetylen über eine Diels-Alder-Reaktion zu Cyclohexa-2,5-dien-1,4-dicarboxylat (PI);
xii) Oxygenieren von PI zu Terephthalsäure.

8. Verfahren nach einem der vorhergehenden Ansprüche, wobei der Biokatalysator aus der Gruppe, bestehend aus Bakterien, Hefen, filamentösen Pilzen, Cyanobakterien, Algen und Pflanzenzellen ausgewählt ist.

9. Verfahren nach einem der Ansprüche 3 bis 8, wobei die Wirtszelle mindestens ein gegebenenfalls heterologes Gen umfasst, das für ein Polypeptid mit Catechol-1,2-dioxygenase-Aktivität kodiert.

10. Verfahren nach einem der Ansprüche 3 bis 9, wobei die Wirtszelle mindestens ein gegebenenfalls heterologes catA-Gen umfasst, wobei das mindestens eine gegebenenfalls heterologe catA-Gen für ein Polypeptid kodiert, das eine Sequenz umfasst, die SEQ ID No. 1, 108 oder 122 entspricht.

11. Verfahren nach einem der Ansprüche 3 bis 10, wobei die Wirtszelle in funktionsfähiger Verbindung mit dem mindestens einen gegebenenfalls heterologen Gen eine Promotorsequenz umfasst, die
vi) SEQ ID No. 5 [Pem7]; oder
vii) SEQ ID No. 6 [Pem7*]; oder
viii) SEQ ID No. 7 [PtufJ; oder
ix) SEQ ID No. 8 [PrpoD]; oder
x) SEQ ID No. 9 [Plac]; oder
xi) SEQ ID No. 10 [PgyrB];
xii) SEQ ID No. 11; oder
xiii) SEQ ID No. 12; oder
xiv) SEQ ID No. 13; oder
xv) SEQ ID No. 14; oder
xvi) SEQ ID No. 15; oder
xvii) SEQ ID No. 16; oder
xviii) SEQ ID No. 88 [Ptuf_1]; oder
xix) SEQ ID No. 89 [Ptuf_short]; oder
xx) SEQ ID No. 90 [Ptuf_s_2]; oder
xxi) SEQ ID No. 91 [Ptuf_s_3]; oder
xxii) SEQ ID No. 92 [Ptuf_s_4]; oder
xxiii) SEQ ID No. 93 [Ptuf_s_5]; oder
xxiv) SEQ ID No. 94 [Ptuf_s_6]; oder
xxv) SEQ ID No. 95 [Ptuf_s_7]; oder
xxvi) SEQ ID No. 96 [Ptuf_s_8]; oder
xxvii) SEQ ID No. 97 [Ptuf_s_9]; oder
xxviii)SEQ ID No. 98 [Ptuf_s_10]; oder
xxix) SEQ ID No. 99 [Ptuf_s_11]; oder
xxx) SEQ ID No. 100 [Ptuf_s_12]; oder
xxxi) SEQ ID No. 101 [Pgro]; oder
xxxii) SEQ ID No. 102 [Pgro_1]; oder
xxxiii)SEQ ID No. 103 [Pgro_2]; oder
xxxiv)SEQ ID No. 104 [Pgro_4]; oder
xxxv) SEQ ID No. 105 [Pgro_5];
vorzugsweise SEQ ID No. 5 [Pem7] entspricht.

12. Verfahren nach einem der Ansprüche 3 bis 11, wobei die Wirtszelle ausgewählt ist aus *Pseudomonas*, vorzugsweise *Pseudomonas putida*, mehr bevorzugt *Pseudomonas putida* Stamm KT2440, Stamm KT2440 JD2S oder Stamm KT2440 BN6, noch mehr bevorzugt *Pseudomonas putida* Stamm KT2440 BN6.

13. Verfahren nach einem der Ansprüche 3 bis 12, wobei die Wirtszelle ein funktionelles catA-Polypeptid exprimiert und kein funktionelles catB-Polypeptid exprimiert.

14. Verfahren nach Anspruch 13, wobei die Wirtszelle, die ein funktionelles catA-Polypeptid exprimiert, wobei das catA-Polypeptid **dadurch gekennzeichnet ist, dass** es Folgendes aufweist
(a) die in SEQ ID No. 108 gezeigte Aminosäuresequenz; oder
(b) eine Aminosäuresequenz, die zu mindestens 40 % Identität mit der in SEQ ID No. 108 gezeigten Aminosäuresequenz aufweist und Catechol-1,2-dioxygenase-Aktivität besitzt;
und kein funktionelles catB-Polypeptid exprimiert, wobei das catB-Polypeptid **dadurch gekennzeichnet ist, dass** es Folgendes aufweist
(c) die in SEQ ID No. 109 gezeigte Aminosäuresequenz; oder
(d) eine Aminosäuresequenz, die zu mindestens 25 % Identität mit der in SEQ ID No. 109 gezeigten Aminosäuresequenz aufweist und Muconat-Cycloisomerase-Aktivität besitzt.

15. Verfahren nach Anspruch 14, wobei die bakterielle Wirtszelle Corynebacterium glutamicum ATCC13032 ist; oder wobei die bakterielle Wirtszelle Amycolatopsis sp. ATCC39116 ist.

## Revendications

1. Procédé de production d'un composé phénolique, comprenant les étapes suivantes
i) la soumission de lignine à un processus de pyrolyse rapide ;
ii) la condensation des vapeurs de pyrolyse résultantes en introduisant directement les vapeurs de pyrolyse résultantes dans au moins une phase aqueuse, résultant en au moins une phase aqueuse comprenant un composé phénolique et au moins une phase organique ;
iii) la séparation de l'au moins une phase aqueuse résultante comprenant un composé phénolique résultante, et de l'au moins une phase organique résultante.

2. Procédé selon la revendication 1, dans lequel le composé phénolique est le catéchol.

3. Procédé de production d'acide cis-cis muconique, comprenant les étapes i) à iii) selon la revendication 1 ou 2 et comprenant en outre les étapes suivantes
iiia) éventuellement la purification du catéchol de la phase aqueuse ;
iv) la soumission d'au moins une phase aqueuse comprenant le catéchol de l'étape iii) ou le catéchol purifié de l'étape iiia) dudit processus de pyrolyse rapide à une conversion biocatalytique en mettant en contact ladite phase aqueuse avec un biocatalyseur, ledit biocatalyseur étant une cellule hôte qui exprime une enzyme présentant une activité catéchol-1,2-dioxygénase (EC 1.13.11.1) (catA) pour préparer de l'acide cis-cis muconique ;
iva) éventuellement la purification de l'acide cis-cis muconique à partir du biocatalyseur.

4. Procédé de production d'acide adipique, comprenant les étapes i) à iii) selon la revendication 1 ou 2 et comprenant en outre les étapes suivantes
iiia) éventuellement la purification du catéchol de la phase aqueuse ;
iv) la soumission d'au moins une phase aqueuse comprenant le catéchol de l'étape iii) ou le catéchol purifié de l'étape iiia) dudit processus de pyrolyse rapide à une conversion biocatalytique en mettant en contact ladite phase aqueuse avec un biocatalyseur, ledit biocatalyseur étant une cellule hôte qui exprime une enzyme présentant une activité catéchol-1,2-dioxygénase (EC 1.13.11.1) (catA) pour préparer de l'acide cis-cis muconique ;
iva) éventuellement la purification de l'acide cis-cis muconique à partir du biocatalyseur ;
v) la préparation d'acide adipique par hydrogénation d'acide cis-cis muconique.

5. Procédé de production de Nylon, comprenant les étapes i) à iii) selon la revendication 1 ou 2 et comprenant en outre les étapes suivantes
iiia) éventuellement la purification du catéchol de la phase aqueuse ;
iv) la soumission d'au moins une phase aqueuse comprenant le catéchol de l'étape iii) ou le catéchol purifié de l'étape iiia) dudit processus de pyrolyse rapide à une conversion biocatalytique en mettant en contact ladite phase aqueuse avec un biocatalyseur, ledit biocatalyseur étant une cellule hôte qui exprime une enzyme présentant une activité catéchol-1,2-dioxygénase (EC 1.13.11.1) (catA) pour préparer de l'acide cis-cis muconique ;
iva) éventuellement la purification de l'acide cis-cis muconique à partir du biocatalyseur ;
v) la préparation d'acide adipique par hydrogénation d'acide cis-cis muconique ;
vi) la mise en réaction d'hexaméthylènediamine et d'acide adipique, de préférence de quantités équivalentes d'hexaméthylènediamine et d'acide adipique, avec de l'eau, de préférence dans un réacteur ;
vii) la cristallisation du produit résultant pour produire un sel de nylon ;
viii) la polymérisation du sel de nylon en nylon 6,6 dans une cuve de réaction, de préférence par lots ou en continu.

6. Procédé de production de caprolactame, comprenant les étapes i) à iii) selon la revendication 1 ou 2 et comprenant en outre les étapes suivantes
iiia) éventuellement la purification du catéchol de la phase aqueuse ;
iv) la soumission d'au moins une phase aqueuse comprenant le catéchol de l'étape iii) ou le catéchol purifié de l'étape iiia) dudit processus de pyrolyse rapide à une conversion biocatalytique en mettant en contact ladite phase aqueuse avec un biocatalyseur, ledit biocatalyseur étant une cellule hôte qui exprime une enzyme présentant une activité catéchol-1,2-dioxygénase (EC 1.13.11.1) (catA) pour préparer de l'acide cis-cis muconique ;
iva) éventuellement la purification de l'acide cis-cis muconique à partir du biocatalyseur ;
ix) la conversion chimique de l'acide cis-cis muconique en caprolactame à l'aide d'un catalyseur, de préférence un catalyseur à base d'alumine, dans un solvant à une température de réaction comprise entre 200 °C et 350 °C.

7. Procédé de production d'acide téréphtalique comprenant les étapes i) à iii) selon la revendication 1 ou 2 et comprenant en outre les étapes suivantes
iiia) éventuellement la purification du catéchol de la phase aqueuse ;
iv) la soumission d'au moins une phase aqueuse comprenant le catéchol de l'étape iii) ou le catéchol purifié de l'étape iiia) dudit processus de pyrolyse rapide à une conversion biocatalytique en mettant en contact ladite phase aqueuse avec un biocatalyseur, ledit biocatalyseur étant une cellule hôte qui exprime une enzyme présentant une activité catéchol-1,2-dioxygénase (EC 1.13.11.1) (catA) pour préparer de l'acide cis-cis muconique ;
iva) éventuellement la purification de l'acide cis-cis muconique à partir du biocatalyseur ;
x) la conversion d'acide cis-cis muconique en acide trans,trans-muconique en présence d'un catalyseur, de préférence I₂ ;
xi) la mise en réaction de l'acide trans,trans-muconique et d'acétylène via une réaction de Diels-Alder en 1,4-dicarboxylate de cyclohexa-2,5-diène (PI) ;
xii) l'oxygénation du PI en acide téréphtalique.

8. Procédé selon l'une quelconque des revendications précédentes, dans lequel le biocatalyseur est choisi dans le groupe constitué par les bactéries, les levures, les champignons filamenteux, les cyanobactéries, les algues et les cellules végétales.

9. Procédé selon l'une quelconque des revendications 3 à 8, dans lequel ladite cellule hôte comprend au moins un gène, éventuellement hétérologue, codant pour un polypeptide ayant une activité catéchol 1,2-dioxygénase.

10. Procédé selon l'une quelconque des revendications 3 à 9, dans lequel ladite cellule hôte comprend au moins un gène catA, éventuellement hétérologue, ledit gène catA au moins, éventuellement hétérologue, codant pour un polypeptide comprenant une séquence correspondant à SEQ ID No. 1, 108 ou 122.

11. Procédé selon l'une quelconque des revendications 3 à 10, dans lequel ladite cellule hôte comprend, liée de manière opérationnelle à l'au moins un gène, éventuellement hétérologue, une séquence promotrice correspondant à
vi) SEQ ID No. 5 [Pem7] ; ou
vii) SEQ ID No. 6 [Pem7*] ; ou
viii) SEQ ID No. 7 [Ptuf] ; ou
ix) SEQ ID No. 8 [PrpoD] ; ou
x) SEQ ID No. 9 [Plac] ; ou
xi) SEQ ID No. 10 [PgyrB] ;
xii) SEQ ID No. 11 ; ou
xiii) SEQ ID No. 12 ; ou
xiv) SEQ ID No. 13 ; ou
xv) SEQ ID No. 14 ; ou
xvi) SEQ ID No. 15 ; ou
xvii) SEQ ID No. 16 ; ou
xviii) SEQ ID No. 88 [Ptuf_1] ; ou
xix) SEQ ID No. 89 [Ptuf_short] ; ou
xx) SEQ ID No. 90 [Ptuf_s_2] ; ou
xviii) SEQ ID No. 91 [Ptuf_s_3] ; ou
xxii) SEQ ID No. 92 [Ptuf_s_4] ; ou
xxiii) SEQ ID No. 93 [Ptuf_s_5] ; ou
xxiv) SEQ ID No. 94 [Ptuf_s_6] ; ou
xxv) SEQ ID No. 95 [Ptuf_s_7] ; ou
xxvi) SEQ ID No. 96 [Ptuf_s_8] ; ou
xxvii) SEQ ID No. 97 [Ptuf_s_9] ; ou
xxviii)SEQ ID No. 98 [Ptuf_s_10] ; ou
xxix) SEQ ID No. 99 [Ptuf_s_11] ; ou
xxx) SEQ ID No. 100 [Ptuf_s_12] ; ou
xxxi) SEQ ID No. 101 [Pgro] ; ou
xxxii) SEQ ID No. 102 [Pgro_1] ; ou
xxxiii)SEQ ID No. 103 [Pgro_2] ; ou
xxxiv)SEQ ID No. 104 [Pgro_4] ; ou
xxxv) SEQ ID No. 105 [Pgro_5] ;
de préférence SEQ ID No. 5 [Pem7].

12. Procédé selon l'une quelconque des revendications 3 à 11, dans lequel ladite cellule hôte est choisie parmi les *Pseudomonas,* de préférence *Pseudomonas putida,* plus préférentiellement la souche KT2440, la souche KT2440 JD2S ou la souche KT2440 BN6 de *Pseudomonas putida* , encore plus préférentiellement la souche KT2440 BN6 de *Pseudomonas putida.*

13. Procédé selon l'une quelconque des revendications 3 à 12, dans lequel la cellule hôte exprime un polypeptide catA fonctionnel et n'exprime pas un polypeptide catB fonctionnel.

14. Procédé selon la revendication 13, dans lequel la cellule hôte qui exprime un polypeptide catA fonctionnel, ledit polypeptide catA étant caractérisé que ce qu'il a
(a) la séquence d'acides aminés indiquée dans le document SEQ ID n° 108 ; ou
(b) une séquence d'acides aminés ayant au moins 40 % d'identité avec la séquence d'acides aminés indiquée dans SEQ ID n° 108 et ayant une activité catéchol-1,2-dioxygénase ;
et n'exprime pas un polypeptide catB fonctionnel, ledit polypeptide catB étant **caractérisé en ce qu'**il a
(c) la séquence d'acides aminés indiquée dans le document SEQ ID n° 109 ; ou
(d) une séquence d'acides aminés ayant au moins 25 % d'identité avec la séquence d'acides aminés indiquée dans SEQ ID n° 109 et ayant une activité muconate cycloisomérase.

15. Procédé selon la revendication 14, dans lequel la cellule hôte bactérienne est Corynebacterium glutamicum ATCC13032 ; ou dans lequel la cellule hôte bactérienne est Amycolatopsis sp. ATCC39116.
